# EUROPEAN PATENT SPECIFICATION

(11) **EP 4 121 532 B1**
(45) Date of publication and mention of the grant of the patent: **30.07.2025**
(21) Application number: 21771038.3
(22) Date of filing: 17.03.2021
(51) Int. Cl.: C12N 15/10, C12N 15/11, C12N 15/113, C12N 15/115, C12Q 1/6837

(54) **CRISPR SYSTEM HIGH THROUGHPUT DIAGNOSTIC SYSTEMS AND METHODS**
CRISPR-SYSTEM, DIAGNOSTISCHE SYSTEME UND VERFAHREN MIT HOHEM DURCHSATZ
SYSTÈMES ET PROCÉDÉS DE DIAGNOSTIC À HAUT RENDEMENT DE SYSTÈME CRISPR

(30) Priority: 17.03.2020 US 202062991004 P
(43) Date of publication of application: 25.01.2023
(73) Proprietor: The Broad Institute, Inc., Cambridge, MA 02142 (US); President And Fellows Of Harvard College, Cambridge, Massachusetts 02138 (US); MASSACHUSETTS INSTITUTE OF TECHNOLOGY, Cambridge, MA 02139 (US); The General Hospital Corporation, Boston, Massachusetts 02114 (US)
(72) Inventor: BLAINEY, Paul, Cambridge, Massachusetts 02142 (US); SABETI, Pardis, Cambridge, Massachusetts 02138 (US); MYHRVOLD, Cameron, Cambridge, Massachusetts 02142 (US); HUNG, Deborah, Cambridge, Massachusetts 02142 (US); ACKERMAN, Cheri, Cambridge, Massachusetts 02139 (US); THAKKU, Gowtham, Cambridge, Massachusetts 02139 (US)
(74) Representative: Impact Intellectual Property LLP
(86) International application number: PCT/US2021/022845
(87) International publication number: WO 2021/188734

(56) References cited:
- WO-A1-2018/170340
- WO-A1-2020/102610
- US-A1- 2020 063 126
- HULTQUIST JUDD F ET AL: "CRISPR-Cas9 genome engineering of primary CD4+T cells for the interrogation of HIV-host factor interactions", NATURE PROTOCOLS, NATURE PUBLISHING GROUP, GB, vol. 14, no. 1, 17 December 2018 (2018-12-17), pages 1 - 27, XP036660419, ISSN: 1754-2189, [retrieved on 20181217], DOI: 10.1038/S41596-018-0069-7

## Description

### CROSS-REFERENCE TO RELATED APPLICATIONS

This application claims the benefit of U.S. Provisional Application No. 62/991,004, filed March 17, 2020.

### SEQUENCE LISTING

This application contains a sequence listing filed in electronic form as an ASCII.txt file entitled BROD-5095WP_ST25.txt, created on March 17, 2021 and having a size of 7,046 bytes (8 KB on disk).

### TECHNICAL FIELD

The subject matter disclosed herein is generally directed to spatially segregated high-throughput diagnostics related to the use of CRISPR systems.

### BACKGROUND

The ability to rapidly detect nucleic acids with high sensitivity and single-base specificity for a large number of samples in a rapid timeframe has the potential to revolutionize diagnosis and monitoring for many diseases, provide valuable epidemiological information, and serve as a generalizable scientific tool. With a platform capable of testing a large number of samples at one time utilizing a small amount of sample would provide distinct advantage over the current state of the art.

### SUMMARY

In certain example embodiments, high throughput method for detecting target molecules comprising the steps of: generating sets of guide molecules, each set of guide molecules comprising guide molecules capable of binding one or more target sequences of a target molecule and designed to form a complex with a Cas protein; distributing a plurality of sets of guide molecules thereby spatially segregating each set of guide molecules; distributing to each set of guide molecules a sample solution, detection reagents, and a reporter construct comprising a non-target sequence, and a Cas protein; initiating a detection reaction, wherein the Cas protein cleaves the non-target sequence of the reporter constructs once activated by the target sequences; and measuring the signal generated from the reporter construct associated with each set of guide molecules from cleavage of the non-target sequence of the reporter constructs.

In an aspect, the plurality of sets of guide molecules are distributed to individual discrete volumes. Each individual discrete volume can comprise a set of one or more detection beads, wherein each detection bead comprises the set of guide molecules capable of binding one of more target sequences of a target molecule.

Each individual discrete volume comprises a set of one or more detection beads, wherein each detection bead comprises the set of guide molecules capable of binding the one or more target sequences of the target molecule. In an embodiment, the method can further comprise the step of mixing the detection bead with Cas protein, thereby coupling the Cas protein to the set of guide molecules disposed on the bead. The guide molecule can comprise a nucleic acid that is coupled to the detection bead at the 5' end or the 3' end of the guide molecule.

In an aspect, the methods and systems further comprise multiple sets of detection beads, each bead in a given set comprising guide molecules configured to detect a particular target molecule, and each different set of beads configured to detect a different target molecule such that detection of multiple target molecules is screened at once. In an aspect, the reporter construct is attached to a reporter bead. In an aspect, the detection bead is encapsulated in a droplet, the reporter bead is encapsulated in the same droplet as the detection bead, or in a separate droplet that can be fused with the droplet comprising the detection bead. In an aspect, the detection bead and the reporter bead are sized differently and such that each individual discrete volume can hold only one of each bead.

In an aspect, the reagents are encoded prior to being delivered to the individual discrete volumes.

The step of the distributing may comprise distributing to wells arrayed on a microfluidic device.

In an aspect, the beads are sized between 2 µm to 100 µm.

The guide molecule comprises, on a 5' or 3' end, a first binding partner of a binding-partner pair, and the bead comprises a second binding partner of the binding-partner pair, in certain embodiments. In an aspect, the first binding partner is biotin and the second binding partner is streptavidin.

The guide molecule can further comprise optical barcodes associated with the guide molecules, the sample, or both.

The detection bead, reporter bead, or both can be magnetic.

In an aspect, the detection reagents further comprise amplification reagents. The amplification reagents may comprise isothermal amplification reagents.

The methods disclosed herein may comprise a Cas protein that is an RNA-targeting protein, a DNA-targeting protein, or a combination thereof. In an aspect, the Cas protein is a Class 1 or Class 2 Cas protein, in an aspect, the Cas protein is a Class 2, Type II, Type V, or Type VI protein.

The methods and systems may comprise a detectable signal that is a level of absorbance, fluorescence, luminescence, polarization, lifetime, or other linear or nonlinear optical property.

The methods and systems can further comprise optical barcodes or unique molecular identifiers associated with the guide molecule, the sample, the individual discrete volume, or a combination thereof.

In certain embodiments, methods and systems further comprise lyophilizing the guide molecules, and optionally the Cas protein, detection reagents, reporter molecule, or a combination thereof.

Methods may further comprise the step of amplifying target molecules in the sample prior to distributing the sample.

The methods and systems may comprise a guide molecule comprising RNA, the RNA comprising a nucleic acid sequence hybridized to a DNA linker sequence disposed on the detection bead.

In certain embodiments, kits are provided comprising a device comprising a set of arrayed capture wells, the capture wells comprising lyophilized guide molecules, and optionally a Cas protein, detection reagents, reporter molecule, or a combination thereof. In an aspect, the arrayed capture wells are pre-decoded.

In certain embodiments, kits are provided comprising: a device comprising a set of arrayed capture wells; a Cas protein; one or more sets of detection beads, wherein each bead in a set comprises a plurality of guide sequences configured to detect a particular target sequence and each set of beads is configured to detect a different target sequence; and a reporter construct comprising a non-target sequence. In an aspect, the kit further comprises a means for generating droplets comprising at least a sample, the Cas protein, and the detection bead. In an aspect, the droplets further comprise the reporter construct. Kits may further comprise the means for generating a second droplet set comprising the reporter construct. In an aspect, the arrayed wells on the device are sized to capture droplets generated using the droplet generating means. In an aspect, each well is sized to capture one droplet or two droplets. Kits may comprise a device with wells having a diameter of 20 µm to 200 µm, optionally between 80 µm to 120 µm, preferably about 100 µm. The device can further comprise one or more spacer lanes between the arrayed capture wells. In an aspect, the device further comprises a mechanism for inducing fusion of droplets in the capture wells. In an aspect, the kit comprises reporter construct is attached to a reporter bead. The kit may comprise a device for the detection bead and reporter bead of different sizes and wherein the capture wells are sized to hold one of each.

In an aspect, the kit includes one or more guide molecules that comprise, on a 5' or 3' end, a first binding partner of a binding-partner pair, and the bead comprises a second binding partner of the binding-partner pair, which may comprise a first binding partner that is biotin and the second binding partner is streptavidin. In an aspect, Cas proteins provided with the kit may be a RNA-targeting Cas or a DNA targeting Cas. In an aspect, the Cas protein is a Class 1 or Class 2 Cas protein, in an aspect, the Cas protein is a Class 2, Type II, Type V, or Type VI protein. Detection reagents of the kit may further comprise amplification reagents, which may be isothermal amplification reagents in certain embodiments. In an aspect, one or more of the detection reagents, detection bead, and reporter construct are lyophilized inside the capture wells of the device.

These and other aspects, objects, features, and advantages of the example embodiments will become apparent to those having ordinary skill in the art upon consideration of the following detailed description of illustrated example embodiments.

### BRIEF DESCRIPTION OF THE DRAWINGS

An understanding of the features and advantages of the present invention will be obtained by reference to the following detailed description that sets forth illustrative embodiments, in which the principles of the invention may be utilized, and the accompanying drawings of which:
**FIG. 1** - Exemplary design using beads to spatially segregate CRISPR guide RNAs.

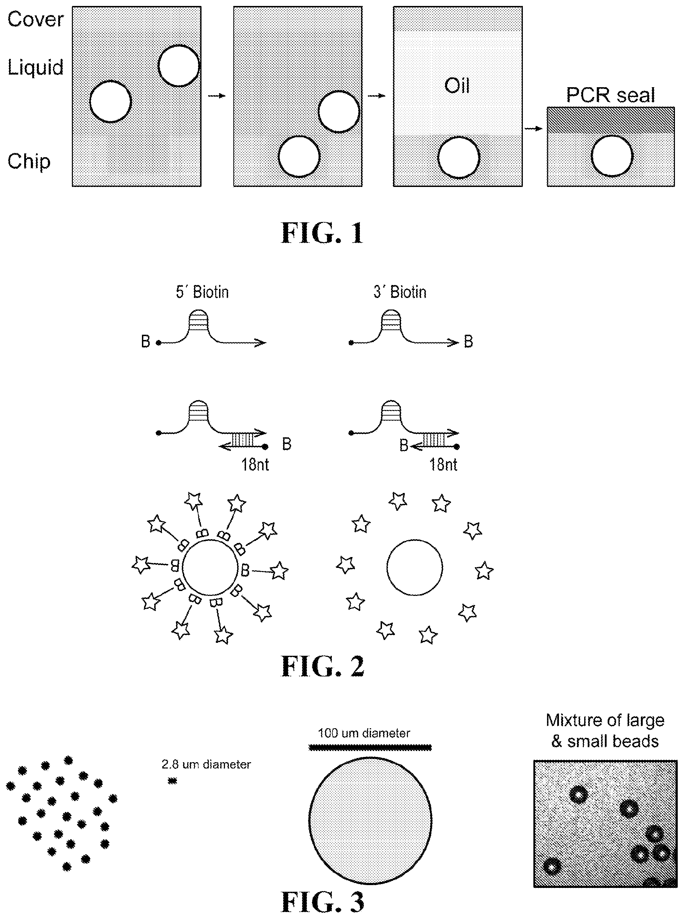

**FIG. 2** - Exemplary attachment strategies for guide RNA. In upper panel, direct modification of biotin can be performed at the 5' or the 3' end of the crRNA; Middle Panel, hybridization of a short complementary DNA to part of the spacer sequence, or an extended sequence of the guide RNA. Can be utilized. An 18 nucleotide DNA sequence is used, with biotin at the 5' or the 3' end of the complementary DNA; Lower panel shows appending of FAM-biotin reporter on the detection bead, with the FAM-Biotin reporter leaving the bead when cleaved. Other designs can contain split luciferase or split lacZ reporters.
**FIG. 3** - Depiction of use of two types of streptavidin beads: large and small, with image on right panel of a mixture of large and small beads.
**FIG. 4** - Exemplary chip design, showing a chip size of 6cmx1cmx3mm optimized for technological development.
**FIG. 5A-5B** - Biotinylated crRNAs can be coupled to streptavidin beads. FIG. 5A. 5' Biotinylated ZIKA crRNA coupled at different concentrations; FIG. 5B 3' Biotinylated ZIKA crRNA coupled at different concentrations. Lowest crRNA density is 6,000 molecules/µm², 2-fold steps in concentration.
**FIG. 6A-6B** FAM-Biotin Reporter is quenched when coupled to streptavidin beads. FIG. 6A charts FAM-Biotin Reporter Fluorescence in A.U. coupled on beads or in solution, with density of 12,000 molecules/µm² at 3 hr; FIG. 6B FAM-Biotin Reporter Fluorescence Kinetics measured for 3 hours with FAM-Biotin reporter in solution and on bead, with and without target.
**FIG. 7** **-** FAM-Biotin reporter coupled to 100 µm beads and used in microwell format produces low signal, at 16 nM FAM-Biotin (upper panel); 16nM FAM-Biotin measured at 0 hr with target (lower left and middle panel) and 5 hr with target (lower right panel).
**FIG. 8** - Lyophilization workflow in an example embodiment including step preparing library of detection droplets, loading of droplets and deposition of droplet components through lyophilization; lower panel shows assay step for sample loading
**FIG. 9A-9B** Exemplary lyophilization workflow. **FIG. 9A** Loading with dropletization; **FIG. 9B** loading without dropletization; and **FIG 9C** loading without dropletization results show ability to use workflow loading without dropletization.
**FIG. 10** shows differences of microfluidic device loading without exemplary plasma treatment and with an exemplary plasma treatment; with no plasma treatment signal:background =3.4, with treatment, signal:background = 5.7.
**FIG. 11** - user workflow according to an exemplary embodiment.
**FIG. 12A-12B** - Images of exemplary embodiments utilizing beads; FIG. 12A fluorescence images of HMPV beads in blue channel distributed onto flow cell at Time 0, Time 30 minutes and Time 60 minutes; FIG. 12B transmitted light images of beads in UV channel of beads at Time 0, Time 30 minutes and Time 60 minutes.
**FIG. 13** - heat map of fluorescence image of bead specific for HMPV, T60 at 37C incubation.

The figures herein are for illustrative purposes only and are not necessarily drawn to scale.

### DETAILED DESCRIPTION OF THE EXAMPLE EMBODIMENTS

WO 2018/170340A1 relates to a high-throughput method for detecting target molecules. WO 2020/102610A1 relates to methods providing high throughput multiplexed detection by cleavage of a reporter construct.

### General Definitions

Unless defined otherwise, technical and scientific terms used herein have the same meaning as commonly understood by one of ordinary skill in the art to which this disclosure pertains. Definitions of common terms and techniques in molecular biology may be found in Molecular Cloning: A Laboratory Manual, 2nd edition (1989) (Sambrook, Fritsch, and Maniatis); Molecular Cloning: A Laboratory Manual, 4th edition (2012) (Green and Sambrook); Current Protocols in Molecular Biology (1987) (F.M. Ausubel et al. eds.); the series Methods in Enzymology (Academic Press, Inc.): PCR 2: A Practical Approach (1995) (M.J. MacPherson, B.D. Hames, and G.R. Taylor eds.): Antibodies, A Laboratory Manual (1988) (Harlow and Lane, eds.): Antibodies A Laboraotry Manual, 2nd edition 2013 (E.A. Greenfield ed.); Animal Cell Culture (1987) (R.I. Freshney, ed.); Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); Robert A. Meyers (ed.), Molecular Biology and Biotechnology: a Comprehensive Desk Reference, published by VCH Publishers, Inc., 1995 (ISBN 9780471185710); Singleton et al., Dictionary of Microbiology and Molecular Biology 2nd ed., J. Wiley & Sons (New York, N.Y. 1994), March, Advanced Organic Chemistry Reactions, Mechanisms and Structure 4th ed., John Wiley & Sons (New York, N.Y. 1992); and Marten H. Hofker and Jan van Deursen, Transgenic Mouse Methods and Protocols, 2nd edition (2011)

As used herein, the singular forms "a", "an", and "the" include both singular and plural referents unless the context clearly dictates otherwise.

The term "optional" or "optionally" means that the subsequent described event, circumstance or substituent may or may not occur, and that the description includes instances where the event or circumstance occurs and instances where it does not.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

As used herein, a "biological sample" may contain whole cells and/or live cells and/or cell debris. The biological sample may contain (or be derived from) a "bodily fluid". The present invention encompasses embodiments wherein the bodily fluid is selected from amniotic fluid, aqueous humour, vitreous humour, bile, blood serum, breast milk, cerebrospinal fluid, cerumen (earwax), chyle, chyme, endolymph, perilymph, exudates, feces, female ejaculate, gastric acid, gastric juice, lymph, mucus (including nasal drainage and phlegm), pericardial fluid, peritoneal fluid, pleural fluid, pus, rheum, saliva, sebum (skin oil), semen, sputum, synovial fluid, sweat, tears, urine, vaginal secretion, vomit and mixtures of one or more thereof. Biological samples include cell cultures, bodily fluids, cell cultures from bodily fluids. Bodily fluids may be obtained from a mammal organism, for example by puncture, or other collecting or sampling procedures.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s). Reference throughout this specification to "one embodiment", "an embodiment," "an example embodiment," means that a particular feature, structure or characteristic described in connection with the embodiment is included in at least one embodiment of the present invention. Thus, appearances of the phrases "in one embodiment," "in an embodiment," or "an example embodiment" in various places throughout this specification are not necessarily all referring to the same embodiment, but may be. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention. For example, in the appended claims, any of the claimed embodiments can be used in any combination.

Reference is made to International Application PCT/US2019/061577 entitled CRSIPR System Based Droplet Diagnostic Systems and Methods, filed November 14, 2019 and U.S. Provisional Application No. 62/767,070, filed November 14, 2018, U.S. Provisional Application No. 62/841,812, filed May 1, 2019, and U.S. Provisional Application No. 62/871,056, filed July 5, 2019.

### OVERVIEW

Embodiments disclosed herein provide high throughput methods of CRISPR-based diagnostics utilizing spatial segregation of CRISPR guide molecules to achieve high throughput methods of detection. Embodiments disclosed herein can detect both DNA and RNA with comparable levels of sensitivity and can differentiate targets from non-targets based on single base pair differences at nanoliter volumes. The presently disclosed subject matter utilizes programmable endonucleases, including certain Class 2 Type V and Type VI Cas proteins, to provide a platform for specific target nucleic acid sensing. The endonucleases from CRISPR systems can be reprogrammed using guide sequences to cleave target RNAs. Utilizing endonucleases that remain active after cleaving its nucleic acid target, like Cas13, 'collateral cleavage' of non-targeted RNAs in proximity is achieved, allowing utilization of the RNA-guided endonucleases to detect the presence of a specific target molecule by *in vitro* non-specific RNA degradation as a readout for detection. (Abudayyeh et al., 2016; East-Seletsky et al., 2016). Such embodiments are useful in multiple scenarios in human health including, for example, viral detection, bacterial strain typing, sensitive genotyping, multiplexed SNP detection, multiplexed strain discrimination and detection of disease-associated cell free DNA In certain embodiments, spatial segregation is achieved through use of detection beads comprising guide molecules. Spatial segregation can also be achieved through placement of guide molecules in an individual discrete volume, for example a well, and lyophilizing the guide molecules until needed for detection. The presently disclosed subject matter utilizes the cleavage activity with spatially segregated or isolated guide molecules to enable high throughput sample diagnostics.

Kits for carrying out the methods disclosed herein are also provided, including device comprising a set of arrayed capture wells. The capture wells can (described but not specifically claimed herein) comprise lyophilized guide molecules, and (described but not specifically claimed herein) optionally one or more of a Cas protein, detection reagents, and a reporter molecule. Kits comprise a device comprising a set of arrayed capture wells; a Cas protein; one or more sets of detection beads, wherein each bead in a set comprises a plurality of guide sequences configured to detect a particular target sequence and each set of beads is configured to detect a different target sequence; and a reporter construct comprising a non-target sequence. Kits can further comprise means for generating droplets which can comprise at least a sample, a Cas protein, and a detection bead.

### Systems and Kits for Methods of Detection

The systems described herein can be utilized in high throughput methods for detecting target molecules. The systems comprise sets of guide molecules. The sets of guide molecules can comprise multiple guide molecules, e.g. a panel of guide molecules, designed to bind to a target molecule. In an aspect, each set of guide molecules comprise guide molecules capable of binding the same target molecule and designed to form a complex with a Cas protein. The guide molecules are distributed in a manner spatially segregating each set of guide molecules. In an aspect, the guide molecules are distributed to individual discrete volumes. The individual discrete volumes are as defined elsewhere herein, and can comprise beads or wells in exemplary embodiments. A sample solution, detection reagents and a reporter construct comprising a non-target sequence, and a Cas protein are also distributed to each individual discrete volume.

### Guide molecules

The systems and kits used in the methods described herein comprise one or more sets of guide molecules. The sets of guide molecules can comprise multiple guide molecules, e.g. a panel of guide molecules, designed to bind to a target molecule. In an aspect, each set of guide molecules comprise guide molecules capable of binding one or more target sequences of a target molecule and designed to form a complex with a Cas protein. The guide molecules are distributed to individual discrete volumes, thereby spatially segregating each set of guide molecules. The individual discrete volumes are as defined elsewhere herein, and can comprise beads or wells in exemplary embodiments. Accordingly, the guide molecules can be provided on a bead, or in a well. The guide molecules may further comprise a first binding partner of a binding partner pair on the 5' or the 3' end. The binding partner may also be attached by hybridization of a complementary DNA sequence comprising a first binding partner of a partner pair to part of the spacer sequence of the guide molecule. The complementary DNA may comprise about 10 to about 20 nucleotides, preferably about 17 to 19 or about 18 nucleotides. The DNA sequence can comprise the first binding partner on the 3' or the 5' end of the DNA sequence. In an aspect, the guide is biotinylated, e.g. the first binding partner pair is biotin. The second binding partner of the binding partner pair is streptavidin and may be functionalized on a surface of the individual discrete volume, e.g. well or bead. In an aspect the binding partner pairs can contain a split luciferase (See, e.g. Yoshimura et al., Chem Rec. 14, 492-501 (2014) doi: 10.1002/tcr.201402001) or split lacZ reporter (See, e.g., Broome et al., Mol. Pharm. 2010 Feb 1: 7(1):60-74; doi: 10.1021/mp900188e), for example, with a first portion of the partner on the guide molecule and the second binding partner portion on the individual discrete volume.

### Guides

As used herein, the term "crRNA" or "guide RNA" or "single guide RNA" or "sgRNA" or "one or more nucleic acid components" of a Type V or Type VI CRISPR-Cas locus effector protein comprises any polynucleotide sequence having sufficient complementarity with a target nucleic acid sequence to hybridize with the target nucleic acid sequence and direct sequence-specific binding of a nucleic acid-targeting complex to the target nucleic acid sequence. In some embodiments, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a nucleic acid-targeting complex to a target nucleic acid sequence may be assessed by any suitable assay. For example, the components of a nucleic acid-targeting CRISPR system sufficient to form a nucleic acid-targeting complex, including the guide sequence to be tested, may be provided to a host cell having the corresponding target nucleic acid sequence, such as by transfection with vectors encoding the components of the nucleic acid-targeting complex, followed by an assessment of preferential targeting (e.g., cleavage) within the target nucleic acid sequence, such as by Surveyor assay as described herein. Similarly, cleavage of a target nucleic acid sequence may be evaluated in a test tube by providing the target nucleic acid sequence, components of a nucleic acid-targeting complex, including the guide sequence to be tested and a control guide sequence different from the test guide sequence, and comparing binding or rate of cleavage at the target sequence between the test and control guide sequence reactions. Other assays are possible, and will occur to those skilled in the art. A guide sequence, and hence a nucleic acid-targeting guide may be selected to target any target nucleic acid sequence. The target sequence may be DNA. The target sequence may be any RNA sequence. In some embodiments, the target sequence may be a sequence within a RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (lncRNA), and small cytoplasmatic RNA (scRNA). In some preferred embodiments, the target sequence may be a sequence within a RNA molecule selected from the group consisting of mRNA, pre-mRNA, and rRNA. In some preferred embodiments, the target sequence may be a sequence within a RNA molecule selected from the group consisting of ncRNA, and lncRNA. In some more preferred embodiments, the target sequence may be a sequence within an mRNA molecule or a pre-mRNA molecule.

In some embodiments, a nucleic acid-targeting guide is selected to reduce the degree secondary structure within the nucleic acid-targeting guide. In some embodiments, about or less than about 75%, 50%, 40%, 30%, 25%, 20%, 15%, 10%, 5%, 1%, or fewer of the nucleotides of the nucleic acid-targeting guide participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g., A.R. Gruber et al., 2008, Cell 106(1): 23-24; and PA Carr and GM Church, 2009, Nature Biotechnology 27(12): 1151-62).

In certain embodiments, a guide RNA or crRNA may comprise, consist essentially of, or consist of a direct repeat (DR) sequence and a guide sequence or spacer sequence. In certain embodiments, the guide RNA or crRNA may comprise, consist essentially of, or consist of a direct repeat sequence fused or linked to a guide sequence or spacer sequence. In certain embodiments, the direct repeat sequence may be located upstream (i.e., 5') from the guide sequence or spacer sequence. In other embodiments, the direct repeat sequence may be located downstream (i.e., 3') from the guide sequence or spacer sequence.

In certain embodiments, the crRNA comprises a stem loop, preferably a single stem loop. In certain embodiments, the direct repeat sequence forms a stem loop, preferably a single stem loop.

In certain embodiments, the spacer length of the guide RNA is from 15 to 35 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27-30 nt, e.g., 27, 28, 29, or 30 nt, from 30-35 nt, e.g., 30, 31, 32, 33, 34, or 35 nt, or 35 nt or longer.

The "tracrRNA" sequence or analogous terms includes any polynucleotide sequence that has sufficient complementarity with a crRNA sequence to hybridize. In some embodiments, the degree of complementarity between the tracrRNA sequence and crRNA sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and crRNA sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence.

In general, degree of complementarity is with reference to the optimal alignment of the sca sequence and tracr sequence, along the length of the shorter of the two sequences. Optimal alignment may be determined by any suitable alignment algorithm, and may further account for secondary structures, such as self-complementarity within either the sca sequence or tracr sequence. In some embodiments, the degree of complementarity between the tracr sequence and sca sequence along the length of the shorter of the two when optimally aligned is about or more than about 25%, 30%, 40%, 50%, 60%, 70%, 80%, 90%, 95%, 97.5%, 99%, or higher.

In general, the CRISPR-Cas, CRISPR-Cas9 or CRISPR system may be as used in the foregoing documents, such as WO 2014/093622 (PCT/US2013/074667) and refers collectively to transcripts and other elements involved in the expression of or directing the activity of CRISPR-associated ("Cas") genes, including sequences encoding a Cas gene, in particular a Cas9 gene in the case of CRISPR-Cas9, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. The section of the guide sequence through which complementarity to the target sequence is important for cleavage activity is referred to herein as the seed sequence. A target sequence may comprise any polynucleotide, such as DNA or RNA polynucleotides. In some embodiments, a target sequence is located in the nucleus or cytoplasm of a cell, and may include nucleic acids in or from mitochondrial, organelles, vesicles, liposomes or particles present within the cell. In some embodiments, especially for non-nuclear uses, NLSs are not preferred. In some embodiments, a CRISPR system comprises one or more nuclear exports signals (NESs). In some embodiments, a CRISPR system comprises one or more NLSs and one or more NESs. In some embodiments, direct repeats may be identified *in silico* by searching for repetitive motifs that fulfill any or all of the following criteria: 1. found in a 2Kb window of genomic sequence flanking the type II CRISPR locus; 2. span from 20 to 50 bp; and 3. interspaced by 20 to 50 bp. In some embodiments, 2 of these criteria may be used, for instance 1 and 2, 2 and 3, or 1 and 3. In some embodiments, all 3 criteria may be used.

In embodiments of the invention the terms guide sequence and guide RNA, i.e. RNA capable of guiding Cas to a target genomic locus, are used interchangeably as in foregoing cited documents such as WO 2014/093622 (PCT/US2013/074667). In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. Preferably the guide sequence is 10 30 nucleotides long. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

In some embodiments of CRISPR-Cas systems, the degree of complementarity between a guide sequence and its corresponding target sequence can be about or more than about 50%, 60%, 75%, 80%, 85%, 90%, 95%, 97.5%, 99%, or 100%; a guide or RNA or sgRNA can be about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length; or guide or RNA or sgRNA can be less than about 75, 50, 45, 40, 35, 30, 25, 20, 15, 12, or fewer nucleotides in length; and advantageously tracr RNA is 30 or 50 nucleotides in length. However, an aspect of the invention is to reduce off-target interactions, e.g., reduce the guide interacting with a target sequence having low complementarity. Indeed, in the examples, it is shown that the invention involves mutations that result in the CRISPR-Cas system being able to distinguish between target and off-target sequences that have greater than 80% to about 95% complementarity, e.g., 83%-84% or 88-89% or 94-95% complementarity (for instance, distinguishing between a target having 18 nucleotides from an off-target of 18 nucleotides having 1, 2 or 3 mismatches). Accordingly, in the context of the present invention the degree of complementarity between a guide sequence and its corresponding target sequence is greater than 94.5% or 95% or 95.5% or 96% or 96.5% or 97% or 97.5% or 98% or 98.5% or 99% or 99.5% or 99.9%, or 100%. Off target is less than 100% or 99.9% or 99.5% or 99% or 99% or 98.5% or 98% or 97.5% or 97% or 96.5% or 96% or 95.5% or 95% or 94.5% or 94% or 93% or 92% or 91% or 90% or 89% or 88% or 87% or 86% or 85% or 84% or 83% or 82% or 81% or 80% complementarity between the sequence and the guide, with it advantageous that off target is 100% or 99.9% or 99.5% or 99% or 99% or 98.5% or 98% or 97.5% or 97% or 96.5% or 96% or 95.5% or 95% or 94.5% complementarity between the sequence and the guide.

In particularly preferred embodiments according to the invention, the guide RNA (capable of guiding Cas to a target locus) may comprise (1) a guide sequence capable of hybridizing to a genomic target locus in the eukaryotic cell; (2) a tracr sequence; and (3) a tracr mate sequence. All (1) to (3) may reside in a single RNA, i.e. an sgRNA (arranged in a 5' to 3' orientation), or the tracr RNA may be a different RNA than the RNA containing the guide and tracr sequence. The tracr hybridizes to the tracr mate sequence and directs the CRISPR/Cas complex to the target sequence. Where the tracr RNA is on a different RNA than the RNA containing the guide and tracr sequence, the length of each RNA may be optimized to be shortened from their respective native lengths, and each may be independently chemically modified to protect from degradation by cellular RNase or otherwise increase stability.

The methods according to the invention as described herein comprehend inducing one or more mutations in a eukaryotic cell (in vitro, i.e. in an isolated eukaryotic cell) as herein discussed comprising delivering to cell a vector as herein discussed. The mutation(s) can include the introduction, deletion, or substitution of one or more nucleotides at each target sequence of cell(s) via the guide(s) RNA(s) or sgRNA(s). The mutations can include the introduction, deletion, or substitution of 1-75 nucleotides at each target sequence of said cell(s) via the guide(s) RNA(s) or sgRNA(s). The mutations can include the introduction, deletion, or substitution of 1, 5, 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s) or sgRNA(s). The mutations can include the introduction, deletion, or substitution of 5, 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s) or sgRNA(s). The mutations include the introduction, deletion, or substitution of 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s) or sgRNA(s). The mutations can include the introduction, deletion, or substitution of 20, 21, 22, 23, 24, 25, 26, 27, 28, 29, 30, 35, 40, 45, 50, or 75 nucleotides at each target sequence of said cell(s) via the guide(s) RNA(s) or sgRNA(s). The mutations can include the introduction, deletion, or substitution of 40, 45, 50, 75, 100, 200, 300, 400 or 500 nucleotides at each target sequence of said cell(s) via the guide(s) RNA(s) or sgRNA(s).

For minimization of toxicity and off-target effect, it may be important to control the concentration of Cas mRNA and guide RNA delivered. Optimal concentrations of Cas mRNA and guide RNA can be determined by testing different concentrations in a cellular or non-human eukaryote animal model and using deep sequencing the analyze the extent of modification at potential off-target genomic loci. Alternatively, to minimize the level of toxicity and off-target effect, Cas nickase mRNA (for example S. pyogenes Cas9 with the D10A mutation) can be delivered with a pair of guide RNAs targeting a site of interest. Guide sequences and strategies to minimize toxicity and off-target effects can be as in WO 2014/093622 (PCT/US2013/074667); or, via mutation as herein.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence.

### Guide Modifications

In certain embodiments, guides of the invention comprise non-naturally occurring nucleic acids and/or non-naturally occurring nucleotides and/or nucleotide analogs, and/or chemically modifications. Non-naturally occurring nucleic acids can include, for example, mixtures of naturally and non-naturally occurring nucleotides. Non-naturally occurring nucleotides and/or nucleotide analogs may be modified at the ribose, phosphate, and/or base moiety. In an embodiment of the invention, a guide nucleic acid comprises ribonucleotides and non-ribonucleotides. In one such embodiment, a guide comprises one or more ribonucleotides and one or more deoxyribonucleotides. In an embodiment of the invention, the guide comprises one or more non-naturally occurring nucleotide or nucleotide analog such as a nucleotide with phosphorothioate linkage, boranophosphate linkage, a locked nucleic acid (LNA) nucleotides comprising a methylene bridge between the 2' and 4' carbons of the ribose ring, peptide nucleic acids (PNA), or bridged nucleic acids (BNA). Other examples of modified nucleotides include 2'-O-methyl analogs, 2'-deoxy analogs, 2-thiouridine analogs, N6-methyladenosine analogs, or 2'-fluoro analogs. Further examples of modified nucleotides include linkage of chemical moieties at the 2' position, including but not limited to peptides, nuclear localization sequence (NLS), peptide nucleic acid (PNA), polyethylene glycol (PEG), triethylene glycol, or tetraethyleneglycol (TEG). Further examples of modified bases include, but are not limited to, 2-aminopurine, 5-bromo-uridine, pseudouridine (Ψ), N¹-methylpseudouridine (me¹Ψ), 5-methoxyuridine(5moU), inosine, 7-methylguanosine. Examples of guide RNA chemical modifications include, without limitation, incorporation of 2'-O-methyl (M), 2'-O-methyl-3'-phosphorothioate (MS), phosphorothioate (PS), S-constrained ethyl(cEt), 2'-O-methyl-3'-thioPACE (MSP), or 2'-O-methyl-3'-phosphonoacetate (MP) at one or more terminal nucleotides. Such chemically modified guides can comprise increased stability and increased activity as compared to unmodified guides, though on-target vs. off-target specificity is not predictable. (See, Hendel, 2015, Nat Biotechnol. 33(9):985-9, doi: 10.1038/nbt.3290, published online 29 June 2015; Ragdarm et al., 0215, PNAS, E7110-E7111; Allerson et al., J. Med. Chem. 2005, 48:901-904; Bramsen et al., Front. Genet., 2012, 3:154; Deng et al., PNAS, 2015, 112:11870-11875; Sharma et al., MedChemComm., 2014, 5:1454-1471; Hendel et al., Nat. Biotechnol. (2015) 33(9): 985-989; Li et al., Nature Biomedical Engineering, 2017, 1, 0066 DOI:10.1038/s41551-017-0066; Ryan et al., Nucleic Acids Res. (2018) 46(2): 792-803). In some embodiments, the 5' and/or 3' end of a guide RNA is modified by a variety of functional moieties including fluorescent dyes, polyethylene glycol, cholesterol, proteins, or detection tags. (See Kelly et al., 2016, J. Biotech. 233:74-83). In certain embodiments, a guide comprises ribonucleotides in a region that binds to a target DNA and one or more deoxyribonucleotides and/or nucleotide analogs in a region that binds to Cas9, Cpfl, or C2c1. In an embodiment of the invention, deoxyribonucleotides and/or nucleotide analogs are incorporated in engineered guide structures, such as, without limitation, 5' and/or 3' end, stem-loop regions, and the seed region. In certain embodiments, the modification is not in the 5'-handle of the stem-loop regions. Chemical modification in the 5'-handle of the stem-loop region of a guide may abolish its function (see Li, et al., Nature Biomedical Engineering, 2017, 1:0066). In certain embodiments, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or 75 nucleotides of a guide is chemically modified. In some embodiments, 3-5 nucleotides at either the 3' or the 5' end of a guide is chemically modified. In some embodiments, only minor modifications are introduced in the seed region, such as 2'-F modifications. In some embodiments, 2'-F modification is introduced at the 3' end of a guide. In certain embodiments, three to five nucleotides at the 5' and/or the 3' end of the guide are chemically modified with 2'-O-methyl (M), 2'-O-methyl-3'-phosphorothioate (MS), S-constrained ethyl(cEt), 2'-O-methyl-3'-thioPACE (MSP), or 2'-O-methyl-3'-phosphonoacetate (MP). Such modification can enhance genome editing efficiency (see Hendel et al., Nat. Biotechnol. (2015) 33(9): 985-989; Ryan et al., Nucleic Acids Res. (2018) 46(2): 792-803). In certain embodiments, all of the phosphodiester bonds of a guide are substituted with phosphorothioates (PS) for enhancing levels of gene disruption. In certain embodiments, more than five nucleotides at the 5' and/or the 3' end of the guide are chemically modified with 2'-O-Me, 2'-F or S-constrained ethyl(cEt). Such chemically modified guide can mediate enhanced levels of gene disruption (see Ragdarm et al., 0215, PNAS, E7110-E7111). In an embodiment of the invention, a guide is modified to comprise a chemical moiety at its 3' and/or 5' end. Such moieties include, but are not limited to amine, azide, alkyne, thio, dibenzocyclooctyne (DBCO), Rhodamine, peptides, nuclear localization sequence (NLS), peptide nucleic acid (PNA), polyethylene glycol (PEG), triethylene glycol, or tetraethyleneglycol (TEG). In certain embodiment, the chemical moiety is conjugated to the guide by a linker, such as an alkyl chain. In certain embodiments, the chemical moiety of the modified guide can be used to attach the guide to another molecule, such as DNA, RNA, protein, or nanoparticles. Such chemically modified guide can be used to identify or enrich cells generically edited by a CRISPR system (see Lee et al., eLife, 2017, 6:e25312, DOI:10.7554). In some embodiments, 3 nucleotides at each of the 3' and 5' ends are chemically modified. In a specific embodiment, the modifications comprise 2'-O-methyl or phosphorothioate analogs. In a specific embodiment, 12 nucleotides in the tetraloop and 16 nucleotides in the stem-loop region are replaced with 2'-O-methyl analogs. Such chemical modifications improve *in vivo* editing and stability (see Finn et al., Cell Reports (2018), 22: 2227-2235). In some embodiments, more than 60 or 70 nucleotides of the guide are chemically modified. In some embodiments, this modification comprises replacement of nucleotides with 2'-O-methyl or 2'-fluoro nucleotide analogs or phosphorothioate (PS) modification of phosphodiester bonds. In some embodiments, the chemical modification comprises 2'-O-methyl or 2'-fluoro modification of guide nucleotides extending outside of the nuclease protein when the CRISPR complex is formed or PS modification of 20 to 30 or more nucleotides of the 3'-terminus of the guide. In a particular embodiment, the chemical modification further comprises 2'-O-methyl analogs at the 5' end of the guide or 2'-fluoro analogs in the seed and tail regions. Such chemical modifications improve stability to nuclease degradation and maintain or enhance genome-editing activity or efficiency, but modification of all nucleotides may abolish the function of the guide (see Yin et al., Nat. Biotech. (2018), 35(12): 1179-1187). Such chemical modifications may be guided by knowledge of the structure of the CRISPR complex, including knowledge of the limited number of nuclease and RNA 2'-OH interactions (see Yin et al., Nat. Biotech. (2018), 35(12): 1179-1187). In some embodiments, one or more guide RNA nucleotides may be replaced with DNA nucleotides. In some embodiments, up to 2, 4, 6, 8, 10, or 12 RNA nucleotides of the 5'-end tail/seed guide region are replaced with DNA nucleotides. In certain embodiments, the majority of guide RNA nucleotides at the 3' end are replaced with DNA nucleotides. In particular embodiments, 16 guide RNA nucleotides at the 3' end are replaced with DNA nucleotides. In particular embodiments, 8 guide RNA nucleotides of the 5'-end tail/seed region and 16 RNA nucleotides at the 3' end are replaced with DNA nucleotides. In particular embodiments, guide RNA nucleotides that extend outside of the nuclease protein when the CRISPR complex is formed are replaced with DNA nucleotides. Such replacement of multiple RNA nucleotides with DNA nucleotides leads to decreased off-target activity but similar on-target activity compared to an unmodified guide; however, replacement of all RNA nucleotides at the 3' end may abolish the function of the guide (see Yin et al., Nat. Chem. Biol. (2018) 14, 311-316). Such modifications may be guided by knowledge of the structure of the CRISPR complex, including knowledge of the limited number of nuclease and RNA 2'-OH interactions (see Yin et al., Nat. Chem. Biol. (2018) 14, 311-316).

In one aspect of the invention, the guide comprises a modified crRNA for Cpfl, having a 5'-handle and a guide segment further comprising a seed region and a 3'-terminus. In some embodiments, the modified guide can be used with a Cpfl of any one of *Acidaminococcus* sp. BV3L6 Cpfl (AsCpf1); *Francisella tularensis* subsp. Novicida U112 Cpfl (FnCpf1); *L. bacterium* MC2017 Cpfl (Lb3Cpf1); *Butyrivibrio proteoclasticus* Cpfl (BpCpf1); *Parcubacteria bacterium* GWC2011_GWC2_44_17 Cpfl (PbCpf1); *Peregrinibacteria bacterium* GW2011_GWA_33_10 Cpfl (PeCpf1); *Leptospira inadai* Cpfl (LiCpf1); *Smithella* sp. SC_K08D17 Cpfl (SsCpf1); *L. bacterium* MA2020 Cpfl (Lb2Cpf1); *Porphyromonas crevioricanis* Cpfl (PcCpf1); *Porphyromonas macacae* Cpfl (PmCpf1); *Candidatus Methanoplasma termitum* Cpfl (CMtCpf1); *Eubacterium eligens* Cpfl (EeCpf1); *Moraxella bovoculi* 237 Cpfl (MbCpf1); *Prevotella disiens* Cpf1 (PdCpf1); or *L. bacterium* ND2006 Cpfl (LbCpf1).

In some embodiments, the modification to the guide is a chemical modification, an insertion, a deletion or a split. In some embodiments, the chemical modification includes, but is not limited to, incorporation of 2'-O-methyl (M) analogs, 2'-deoxy analogs, 2-thiouridine analogs, N6-methyladenosine analogs, 2'-fluoro analogs, 2-aminopurine, 5-bromo-uridine, pseudouridine (Ψ), N¹-methylpseudouridine (me¹Ψ), 5-methoxyuridine(5moU), inosine, 7-methylguanosine, 2'-O-methyl-3'-phosphorothioate (MS), S-constrained ethyl(cEt), phosphorothioate (PS), 2'-O-methyl-3'-thioPACE (MSP), or 2'-O-methyl-3'-phosphonoacetate (MP). In some embodiments, the guide comprises one or more of phosphorothioate modifications. In certain embodiments, at least 1, 2, 3, 4, 5, 6, 7, 8, 9, 10, 11, 12, 13, 14, 15, 16, 17, 18, 19, 20, or 25 nucleotides of the guide are chemically modified. In some embodiments, all nucleotides are chemically modified. In certain embodiments, one or more nucleotides in the seed region are chemically modified. In certain embodiments, one or more nucleotides in the 3'-terminus are chemically modified. In certain embodiments, none of the nucleotides in the 5'-handle is chemically modified. In some embodiments, the chemical modification in the seed region is a minor modification, such as incorporation of a 2'-fluoro analog. In a specific embodiment, one nucleotide of the seed region is replaced with a 2'-fluoro analog. In some embodiments, 5 or 10 nucleotides in the 3'-terminus are chemically modified. Such chemical modifications at the 3'-terminus of the Cpfl CrRNA improve gene cutting efficiency (see Li, et al., Nature Biomedical Engineering, 2017, 1:0066). In a specific embodiment, 5 nucleotides in the 3'-terminus are replaced with 2'-fluoro analogues. In a specific embodiment, 10 nucleotides in the 3'-terminus are replaced with 2'-fluoro analogues. In a specific embodiment, 5 nucleotides in the 3'-terminus are replaced with 2'- O-methyl (M) analogs. In some embodiments, 3 nucleotides at each of the 3' and 5' ends are chemically modified. In a specific embodiment, the modifications comprise 2'-O-methyl or phosphorothioate analogs. In a specific embodiment, 12 nucleotides in the tetraloop and 16 nucleotides in the stem-loop region are replaced with 2'-O-methyl analogs. Such chemical modifications improve *in vivo* editing and stability (see Finn et al., Cell Reports (2018), 22: 2227-2235).

In some embodiments, the loop of the 5'-handle of the guide is modified. In some embodiments, the loop of the 5'-handle of the guide is modified to have a deletion, an insertion, a split, or chemical modifications. In certain embodiments, the loop comprises 3, 4, or 5 nucleotides. In certain embodiments, the loop comprises the sequence of UCUU, UUUU, UAUU, or UGUU. In some embodiments, the guide molecule forms a stemloop with a separate non-covalently linked sequence, which can be DNA or RNA.

### Synthetically linked guide

In one aspect, the guide comprises a tracr sequence and a tracr mate sequence that are chemically linked or conjugated via a non-phosphodiester bond. In one aspect, the guide comprises a tracr sequence and a tracr mate sequence that are chemically linked or conjugated via a non-nucleotide loop. In some embodiments, the tracr and tracr mate sequences are j oined via a non-phosphodiester covalent linker. Examples of the covalent linker include but are not limited to a chemical moiety selected from the group consisting of carbamates, ethers, esters, amides, imines, amidines, aminotrizines, hydrozone, disulfides, thioethers, thioesters, phosphorothioates, phosphorodithioates, sulfonamides, sulfonates, fulfones, sulfoxides, ureas, thioureas, hydrazide, oxime, triazole, photolabile linkages, C-C bond forming groups such as Diels-Alder cyclo-addition pairs or ring-closing metathesis pairs, and Michael reaction pairs.

In some embodiments, the tracr and tracr mate sequences are first synthesized using the standard phosphoramidite synthetic protocol (Herdewijn, P., ed., Methods in Molecular Biology Col 288, Oligonucleotide Synthesis: Methods and Applications, Humana Press, New Jersey (2012)). In some embodiments, the tracr or tracr mate sequences can be functionalized to contain an appropriate functional group for ligation using the standard protocol known in the art (Hermanson, G. T., Bioconjugate Techniques, Academic Press (2013)). Examples of functional groups include, but are not limited to, hydroxyl, amine, carboxylic acid, carboxylic acid halide, carboxylic acid active ester, aldehyde, carbonyl, chlorocarbonyl, imidazolylcarbonyl, hydrozide, semicarbazide, thio semicarbazide, thiol, maleimide, haloalkyl, sufonyl, ally, propargyl, diene, alkyne, and azide. Once the tracr and the tracr mate sequences are functionalized, a covalent chemical bond or linkage can be formed between the two oligonucleotides. Examples of chemical bonds include, but are not limited to, those based on carbamates, ethers, esters, amides, imines, amidines, aminotrizines, hydrozone, disulfides, thioethers, thioesters, phosphorothioates, phosphorodithioates, sulfonamides, sulfonates, fulfones, sulfoxides, ureas, thioureas, hydrazide, oxime, triazole, photolabile linkages, C-C bond forming groups such as Diels-Alder cyclo-addition pairs or ring-closing metathesis pairs, and Michael reaction pairs.

In some embodiments, the tracr and tracr mate sequences can be chemically synthesized. In some embodiments, the chemical synthesis uses automated, solid-phase oligonucleotide synthesis machines with 2'-acetoxyethyl orthoester (2'-ACE) (Scaringe et al., J. Am. Chem. Soc. (1998) 120: 11820-11821; Scaringe, Methods Enzymol. (2000) 317: 3-18) or 2'-thionocarbamate (2'-TC) chemistry (Dellinger et al., J. Am. Chem. Soc. (2011) 133: 11540-11546; Hendel et al., Nat. Biotechnol. (2015) 33:985-989).

In some embodiments, the tracr and tracr mate sequences can be covalently linked using various bioconjugation reactions, loops, bridges, and non-nucleotide links via modifications of sugar, internucleotide phosphodiester bonds, purine and pyrimidine residues. Sletten et al., Angew. Chem. Int. Ed. (2009) 48:6974-6998; Manoharan, M. Curr. Opin. Chem. Biol. (2004) 8: 570-9; Behlke et al., Oligonucleotides (2008) 18: 305-19; Watts, et al., Drug. Discov. Today (2008) 13: 842-55; Shukla, et al., ChemMedChem (2010) 5: 328-49.

In some embodiments, the tracr and tracr mate sequences can be covalently linked using click chemistry. In some embodiments, the tracr and tracr mate sequences can be covalently linked using a triazole linker. In some embodiments, the tracr and tracr mate sequences can be covalently linked using Huisgen 1,3-dipolar cycloaddition reaction involving an alkyne and azide to yield a highly stable triazole linker (He et al., ChemBioChem (2015) 17: 1809-1812; WO 2016/186745). In some embodiments, the tracr and tracr mate sequences are covalently linked by ligating a 5'-hexyne tracrRNA and a 3'-azide crRNA. In some embodiments, either or both of the 5'-hexyne tracrRNA and a 3'-azide crRNA can be protected with 2'-acetoxyethl orthoester (2'-ACE) group, which can be subsequently removed using Dharmacon protocol (Scaringe et al., J. Am. Chem. Soc. (1998) 120: 11820-11821; Scaringe, Methods Enzymol. (2000) 317: 3-18).

In some embodiments, the tracr and tracr mate sequences can be covalently linked via a linker (e.g., a non-nucleotide loop) that comprises a moiety such as spacers, attachments, bioconjugates, chromophores, reporter groups, dye labeled RNAs, and non-naturally occurring nucleotide analogues. More specifically, suitable spacers for purposes of this invention include, but are not limited to, polyethers (e.g., polyethylene glycols, polyalcohols, polypropylene glycol or mixtures of ethylene and propylene glycols), polyamines group (e.g., spennine, spermidine and polymeric derivatives thereof), polyesters (e.g., poly(ethyl acrylate)), polyphosphodiesters, alkylenes, and combinations thereof. Suitable attachments include any moiety that can be added to the linker to add additional properties to the linker, such as but not limited to, fluorescent labels. Suitable bioconjugates include, but are not limited to, peptides, glycosides, lipids, cholesterol, phospholipids, diacyl glycerols and dialkyl glycerols, fatty acids, hydrocarbons, enzyme substrates, steroids, biotin, digoxigenin, carbohydrates, polysaccharides. Suitable chromophores, reporter groups, and dye-labeled RNAs include, but are not limited to, fluorescent dyes such as fluorescein and rhodamine, chemiluminescent, electrochemiluminescent, and bioluminescent marker compounds. The design of example linkers conjugating two RNA components are also described in WO 2004/015075.

The linker (e.g., a non-nucleotide loop) can be of any length. In some embodiments, the linker has a length equivalent to about 0-16 nucleotides. In some embodiments, the linker has a length equivalent to about 0-8 nucleotides. In some embodiments, the linker has a length equivalent to about 0-4 nucleotides. In some embodiments, the linker has a length equivalent to about 2 nucleotides. Example linker design is also described in WO2011/008730.

A typical Type II Cas9 sgRNA comprises (in 5' to 3' direction): a guide sequence, a poly U tract, a first complimentary stretch (the "repeat"), a loop (tetraloop), a second complimentary stretch (the "anti-repeat" being complimentary to the repeat), a stem, and further stem loops and stems and a poly A (often poly U in RNA) tail (terminator). In preferred embodiments, certain aspects of guide architecture are retained, certain aspect of guide architecture cam be modified, for example by addition, subtraction, or substitution of features, whereas certain other aspects of guide architecture are maintained. Preferred locations for engineered sgRNA modifications, including but not limited to insertions, deletions, and substitutions include guide termini and regions of the sgRNA that are exposed when complexed with CRISPR protein and/or target, for example the tetraloop and/or loop2.

In certain embodiments, guides of the invention comprise specific binding sites (e.g. aptamers) for adapter proteins, which may comprise one or more functional domains (e.g. via fusion protein). When such a guide forms a CRISPR complex (i.e. CRISPR enzyme binding to guide and target) the adapter proteins bind and, the functional domain associated with the adapter protein is positioned in a spatial orientation which is advantageous for the attributed function to be effective. For example, if the functional domain is a transcription activator (e.g. VP64 or p65), the transcription activator is placed in a spatial orientation which allows it to affect the transcription of the target. Likewise, a transcription repressor will be advantageously positioned to affect the transcription of the target and a nuclease (e.g. Fok1) will be advantageously positioned to cleave or partially cleave the target.

The skilled person will understand that modifications to the guide which allow for binding of the adapter + functional domain but not proper positioning of the adapter + functional domain (e.g. due to steric hindrance within the three-dimensional structure of the CRISPR complex) are modifications which are not intended. The one or more modified guide may be modified at the tetra loop, the stem loop 1, stem loop 2, or stem loop 3, as described herein, preferably at either the tetra loop or stem loop 2, and most preferably at both the tetra loop and stem loop 2.

The repeat:anti repeat duplex will be apparent from the secondary structure of the sgRNA. It may be typically a first complimentary stretch after (in 5' to 3' direction) the poly U tract and before the tetraloop; and a second complimentary stretch after (in 5' to 3' direction) the tetraloop and before the poly A tract. The first complimentary stretch (the "repeat") is complimentary to the second complimentary stretch (the "anti-repeat"). As such, they Watson-Crick base pair to form a duplex of dsRNA when folded back on one another. As such, the anti-repeat sequence is the complimentary sequence of the repeat and in terms to A-U or C-G base pairing, but also in terms of the fact that the anti-repeat is in the reverse orientation due to the tetraloop.

In an embodiment of the invention, modification of guide architecture comprises replacing bases in stemloop 2. For example, in some embodiments, "actt" ("acuu" in RNA) and "aagt" ("aagu" in RNA) bases in stemloop2 are replaced with "cgcc" and "gcgg". In some embodiments, "actt" and "aagt" bases in stemloop2 are replaced with complimentary GC-rich regions of 4 nucleotides. In some embodiments, the complimentary GC-rich regions of 4 nucleotides are "cgcc" and "gcgg" (both in 5' to 3' direction). In some embodiments, the complimentary GC-rich regions of 4 nucleotides are "gcgg" and "cgcc" (both in 5' to 3' direction). Other combination of C and G in the complimentary GC-rich regions of 4 nucleotides will be apparent including CCCC and GGGG.

In one aspect, the stemloop 2, e.g., "ACTTgtttAAGT" (SEQ ID NO: 1) can be replaced by any "XXXXgtttYYYY" (SEQ ID NO: 2), e.g., where XXXX and YYYY represent any complementary sets of nucleotides that together will base pair to each other to create a stem.

In one aspect, the stem comprises at least about 4bp comprising complementary X and Y sequences, although stems of more, e.g., 5, 6, 7, 8, 9, 10, 11 or 12 or fewer, e.g., 3, 2, base pairs are also contemplated. Thus, for example X2-12 and Y2-12 (wherein X and Y represent any complementary set of nucleotides) may be contemplated. In one aspect, the stem made of the X and Y nucleotides, together with the "gttt," will form a complete hairpin in the overall secondary structure; and, this may be advantageous and the amount of base pairs can be any amount that forms a complete hairpin. In one aspect, any complementary X:Y basepairing sequence (e.g., as to length) is tolerated, so long as the secondary structure of the entire sgRNA is preserved. In one aspect, the stem can be a form of X:Y basepairing that does not disrupt the secondary structure of the whole sgRNA in that it has a DR:tracr duplex, and 3 stemloops. In one aspect, the "gttt" tetraloop that connects ACTT and AAGT (or any alternative stem made of X:Y basepairs) can be any sequence of the same length (e.g., 4 basepair) or longer that does not interrupt the overall secondary structure of the sgRNA. In one aspect, the stemloop can be something that further lengthens stemloop2, e.g. can be MS2 aptamer. In one aspect, the stemloop3 "GGCACCGagtCGGTGC" (SEQ ID NO: 3) can likewise take on a "XXXXXXXagtYYYYYYY" (SEQ ID NO: 4) form, e.g., wherein X7 and Y7 represent any complementary sets of nucleotides that together will base pair to each other to create a stem. In one aspect, the stem comprises about 7bp comprising complementary X and Y sequences, although stems of more or fewer basepairs are also contemplated. In one aspect, the stem made of the X and Y nucleotides, together with the "agt", will form a complete hairpin in the overall secondary structure. In one aspect, any complementary X:Y basepairing sequence is tolerated, so long as the secondary structure of the entire sgRNA is preserved. In one aspect, the stem can be a form of X:Y basepairing that doesn't disrupt the secondary structure of the whole sgRNA in that it has a DR:tracr duplex, and 3 stemloops. In one aspect, the "agt" sequence of the stemloop 3 can be extended or be replaced by an aptamer, e.g., a MS2 aptamer or sequence that otherwise generally preserves the architecture of stemloop3. In one aspect for alternative Stemloops 2 and/or 3, each X and Y pair can refer to any basepair. In one aspect, non-Watson Crick basepairing is contemplated, where such pairing otherwise generally preserves the architecture of the stemloop at that position.

In one aspect, the DR:tracrRNA duplex can be replaced with the form: gYYYYag(N)NNNNxxxxNNNN(AAN)uuRRRRu (SEQ ID NO: 5) (using standard IUPAC nomenclature for nucleotides), wherein (N) and (AAN) represent part of the bulge in the duplex, and "xxxx" represents a linker sequence. NNNN on the direct repeat can be anything so long as it basepairs with the corresponding NNNN portion of the tracrRNA. In one aspect, the DR:tracrRNA duplex can be connected by a linker of any length (xxxx...), any base composition, as long as it doesn't alter the overall structure.

In one aspect, the sgRNA structural requirement is to have a duplex and 3 stemloops. In most aspects, the actual sequence requirement for many of the particular base requirements are lax, in that the architecture of the DR:tracrRNA duplex should be preserved, but the sequence that creates the architecture, i.e., the stems, loops, bulges, etc., may be alterred.

### Aptamers

One guide with a first aptamer/RNA-binding protein pair can be linked or fused to an activator, whilst a second guide with a second aptamer/RNA-binding protein pair can be linked or fused to a repressor. The guides are for different targets (loci), so this allows one gene to be activated and one repressed. For example, the following schematic shows such an approach:
Guide 1- MS2 aptamer-------MS2 RNA-binding protein------- VP64 activator; and
Guide 2 - PP7 aptamer-------PP7 RNA-binding protein-------SID4x repressor.

The present invention also relates to orthogonal PP7/MS2 gene targeting. In this example, sgRNA targeting different loci are modified with distinct RNA loops in order to recruit MS2-VP64 or PP7-SID4X, which activate and repress their target loci, respectively. PP7 is the RNA-binding coat protein of the bacteriophage Pseudomonas. Like MS2, it binds a specific RNA sequence and secondary structure. The PP7 RNA-recognition motif is distinct from that of MS2. Consequently, PP7 and MS2 can be multiplexed to mediate distinct effects at different genomic loci simultaneously. For example, an sgRNA targeting locus A can be modified with MS2 loops, recruiting MS2-VP64 activators, while another sgRNA targeting locus B can be modified with PP7 loops, recruiting PP7-SID4X repressor domains. In the same cell, dCas9 can thus mediate orthogonal, locus-specific modifications. This principle can be extended to incorporate other orthogonal RNA-binding proteins such as Q-beta.

An alternative option for orthogonal repression includes incorporating non-coding RNA loops with transactive repressive function into the guide (either at similar positions to the MS2/PP7 loops integrated into the guide or at the 3' terminus of the guide). For instance, guides were designed with non-coding (but known to be repressive) RNA loops (e.g. using the Alu repressor (in RNA) that interferes with RNA polymerase II in mammalian cells). The Alu RNA sequence was located: in place of the MS2 RNA sequences as used herein (e.g. at tetraloop and/or stem loop 2); and/or at 3' terminus of the guide. This gives possible combinations of MS2, PP7 or Alu at the tetraloop and/or stemloop 2 positions, as well as, optionally, addition of Alu at the 3' end of the guide (with or without a linker).

The use of two different aptamers (distinct RNA) allows an activator-adaptor protein fusion and a repressor-adaptor protein fusion to be used, with different guides, to activate expression of one gene, whilst repressing another. They, along with their different guides can be administered together, or substantially together, in a multiplexed approach. A large number of such modified guides can be used all at the same time, for example 10 or 20 or 30 and so forth, whilst only one (or at least a minimal number) of Cas9s to be delivered, as a comparatively small number of Cas9s can be used with a large number modified guides. The adaptor protein may be associated (preferably linked or fused to) one or more activators or one or more repressors. For example, the adaptor protein may be associated with a first activator and a second activator. The first and second activators may be the same, but they are preferably different activators. For example, one might be VP64, whilst the other might be p65, although these are just examples and other transcriptional activators are envisaged. Three or more or even four or more activators (or repressors) may be used, but package size may limit the number being higher than 5 different functional domains. Linkers are preferably used, over a direct fusion to the adaptor protein, where two or more functional domains are associated with the adaptor protein. Suitable linkers might include the GlySer linker.

It is also envisaged that the enzyme-guide complex as a whole may be associated with two or more functional domains. For example, there may be two or more functional domains associated with the enzyme, or there may be two or more functional domains associated with the guide (via one or more adaptor proteins), or there may be one or more functional domains associated with the enzyme and one or more functional domains associated with the guide (via one or more adaptor proteins).

The fusion between the adaptor protein and the activator or repressor may include a linker. For example, GlySer linkers GGGS can be used. They can be used in repeats of 3 ((GGGGS)₃) (SEQ ID NO: 6) or 6, 9 or even 12 or more, to provide suitable lengths, as required. Linkers can be used between the RNA-binding protein and the functional domain (activator or repressor), or between the CRISPR Enzyme (Cas9) and the functional domain (activator or repressor). The linkers the user to engineer appropriate amounts of "mechanical flexibility".

### Tandem guides and uses in a multiplex (tandem) targeting approach

The inventors have shown that CRISPR enzymes as defined herein can employ more than one RNA guide without losing activity. This enables the use of the CRISPR enzymes, systems or complexes as defined herein for targeting multiple DNA targets, genes or gene loci, with a single enzyme, system or complex as defined herein. The guide RNAs may be tandemly arranged, optionally separated by a nucleotide sequence such as a direct repeat as defined herein. The position of the different guide RNAs is the tandem does not influence the activity. It is noted that the terms "CRISPR-Cas system", "CRISP-Cas complex" "CRISPR complex" and "CRISPR system" are used interchangeably. Also the terms "CRISPR enzyme", "Cas enzyme", or "CRISPR-Cas enzyme", can be used interchangeably. In preferred embodiments, said CRISPR enzyme, CRISP-Cas enzyme or Cas enzyme is Cas9, or any one of the modified or mutated variants thereof described herein elsewhere.

In one aspect, the invention provides a non-naturally occurring or engineered CRISPR enzyme, preferably a class 2 CRISPR enzyme, preferably a Type V or VI CRISPR enzyme as described herein, such as without limitation Cas9 as described herein elsewhere, used for tandem or multiplex targeting. It is to be understood that any of the CRISPR (or CRISPR-Cas or Cas) enzymes, complexes, or systems according to the invention as described herein elsewhere may be used in such an approach. Any of the methods, products, compositions and uses as described herein elsewhere are equally applicable with the multiplex or tandem targeting approach further detailed below. By means of further guidance, the following particular aspects and embodiments are provided.

In one aspect, the invention provides for the use of a Cas9 enzyme, complex or system as defined herein for targeting multiple gene loci. In one embodiment, this can be established by using multiple (tandem or multiplex) guide RNA (gRNA) sequences.

In one aspect, the invention provides methods for using one or more elements of a Cas enzyme, complex or system as defined herein for tandem or multiplex targeting, wherein said CRISPR system comprises multiple guide RNA sequences. Preferably, said gRNA sequences are separated by a nucleotide sequence, such as a direct repeat as defined herein elsewhere.

The Cas enzyme, system or complex as defined herein provides an effective means for modifying multiple target polynucleotides. The Ca enzyme, system or complex as defined herein has a wide variety of utility including modifying (e.g., deleting, inserting, translocating, inactivating, activating) one or more target polynucleotides in a multiplicity of cell types. As such the Cas enzyme, system or complex as defined herein of the invention has a broad spectrum of applications in, e.g., gene therapy, drug screening, disease diagnosis, and prognosis, including targeting multiple gene loci within a single CRISPR system.

In one aspect, the invention provides a Cas9 enzyme, system or complex as defined herein, i.e. a Cas9 CRISPR-Cas complex having a Cas9 protein having at least one destabilization domain associated therewith, and multiple guide RNAs that target multiple nucleic acid molecules such as DNA molecules, whereby each of said multiple guide RNAs specifically targets its corresponding nucleic acid molecule, e.g., DNA molecule. Each nucleic acid molecule target, e.g., DNA molecule can encode a gene product or encompass a gene locus. Using multiple guide RNAs hence enables the targeting of multiple gene loci or multiple genes. In some embodiments the Cas9 enzyme may cleave the DNA molecule encoding the gene product. In some embodiments expression of the gene product is altered. The Cas9 protein and the guide RNAs do not naturally occur together. The invention comprehends the guide RNAs comprising tandemly arranged guide sequences. The invention further comprehends coding sequences for the Cas9 protein being codon optimized for expression in a eukaryotic cell. In a preferred embodiment the eukaryotic cell is a mammalian cell, a plant cell or a yeast cell and in a more preferred embodiment the mammalian cell is a human cell. Expression of the gene product may be decreased. The Cas9 enzyme may form part of a CRISPR system or complex, which further comprises tandemly arranged guide RNAs (gRNAs) comprising a series of 2, 3, 4, 5, 6, 7, 8, 9, 10, 15, 25, 25, 30, or more than 30 guide sequences, each capable of specifically hybridizing to a target sequence in a genomic locus of interest in a cell. In some embodiments, the functional Cas9 CRISPR system or complex binds to the multiple target sequences. In some embodiments, the functional CRISPR system or complex may edit the multiple target sequences, e.g., the target sequences may comprise a genomic locus, and in some embodiments there may be an alteration of gene expression. In some embodiments, the functional CRISPR system or complex may comprise further functional domains. In some embodiments, the invention provides a method for altering or modifying expression of multiple gene products. The method may comprise introducing into a cell containing said target nucleic acids, e.g., DNA molecules, or containing and expressing target nucleic acid, e.g., DNA molecules; for instance, the target nucleic acids may encode gene products or provide for expression of gene products (e.g., regulatory sequences).

In preferred embodiments the CRISPR enzyme used for multiplex targeting is Cas9, or the CRISPR system or complex comprises Cas9. In some embodiments, the CRISPR enzyme used for multiplex targeting is AsCas9, or the CRISPR system or complex used for multiplex targeting comprises an AsCas9. In some embodiments, the CRISPR enzyme is an LbCas9, or the CRISPR system or complex comprises LbCas9. In some embodiments, the Cas9 enzyme used for multiplex targeting cleaves both strands of DNA to produce a double strand break (DSB). In some embodiments, the CRISPR enzyme used for multiplex targeting is a nickase. In some embodiments, the Cas9 enzyme used for multiplex targeting is a dual nickase. In some embodiments, the Cas9 enzyme used for multiplex targeting is a Cas9 enzyme such as a DD Cas9 enzyme as defined herein elsewhere.

In some general embodiments, the Cas9 enzyme used for multiplex targeting is associated with one or more functional domains. In some more specific embodiments, the CRISPR enzyme used for multiplex targeting is a deadCas9 as defined herein elsewhere.

Also provided is a model that constitutively expresses the Cas enzyme, complex or system as used herein for use in multiplex targeting. The organism may be transgenic and may have been transfected with the present vectors or may be the offspring of an organism so transfected. In a further aspect, the present invention provides compositions comprising the CRISPR enzyme, system and complex as defined herein or the polynucleotides or vectors described herein. Also provides are Cas9 CRISPR systems or complexes comprising multiple guide RNAs, preferably in a tandemly arranged format. Said different guide RNAs may be separated by nucleotide sequences such as direct repeats.

In one aspect, the invention provides an engineered, non-naturally occurring CRISPR system comprising a Cas protein and multiple guide RNAs that each specifically target a DNA molecule encoding a gene product in a cell, whereby the multiple guide RNAs each target their specific DNA molecule encoding the gene product and the Cas protein cleaves the target DNA molecule encoding the gene product, whereby expression of the gene product is altered; and, wherein the CRISPR protein and the guide RNAs do not naturally occur together. The invention comprehends the multiple guide RNAs comprising multiple guide sequences, preferably separated by a nucleotide sequence such as a direct repeat and optionally fused to a tracr sequence. In an embodiment of the invention the CRISPR protein is a type V or VI CRISPR-Cas protein and in a more preferred embodiment the CRISPR protein is a Cas13 protein. The invention further comprehends a Cas13 protein being codon optimized for expression in a eukaryotic cell. In a preferred embodiment the eukaryotic cell is a mammalian cell and in a more preferred embodiment the mammalian cell is a human cell. In a further embodiment of the invention, the expression of the gene product is decreased.

As used herein, the term "guide RNA" or "gRNA" has the meaning as used herein elsewhere and comprises any polynucleotide sequence having sufficient complementarity with a target nucleic acid sequence to hybridize with the target nucleic acid sequence and direct sequence-specific binding of a nucleic acid-targeting complex to the target nucleic acid sequence. Each gRNA may be designed to include multiple binding recognition sites (e.g., aptamers) specific to the same or different adapter protein. Each gRNA may be designed to bind to the promoter region -1000 - +1 nucleic acids upstream of the transcription start site (i.e. TSS), preferably -200 nucleic acids. This positioning improves functional domains which affect gene activation (e.g., transcription activators) or gene inhibition (e.g., transcription repressors). The modified gRNA may be one or more modified gRNAs targeted to one or more target loci (e.g., at least 1 gRNA, at least 2 gRNA, at least 5 gRNA, at least 10 gRNA, at least 20 gRNA, at least 30 g RNA, at least 50 gRNA) comprised in a composition. Said multiple gRNA sequences can be tandemly arranged and are preferably separated by a direct repeat.

### Lyophilized Guides and Reagents

The guide molecules of the embodiments disclosed herein can be prepared in freeze-dried format for convenient distribution and point-of-care (POC) applications. Optionally one or more of Cas protein, reporter molecules, and detection reagents can also be freeze dried, or lyophilized, according to certain embodiments.

Each discrete volume may comprise a set of guide RNAs for a different target molecule. These guide RNAs may be specific for a target molecule but each guide RNA in the set may vary, creating a panel of guide RNAs for a target molecule. In certain embodiments, the CRISPR effector protein is bound or associated to each discrete volume. The Cas protein, the detection reagents, reporter molecules or a combination thereof can also be lyophilized. Exemplary methods of freeze-drying are described in International Patent Publication WO 2018/107129 at [0470], [0492]. In an aspect, the lyophilized reagents are provided in a pre-decoded microwell array. In this manner, a sample can be provided, optionally pre-amplified, to the individual discrete volume. In particular instances, the sample is provided with one or more of the detection reagents, reporter molecules, or other components of the system, if not previously lyophilized and provided as part of the individual discrete volume. In a preferred embodiment, arrayed and encoded reagent sets are stored in the individual discrete volume. The encoded reagent sets may be provided in solution, on a bead, and/or lyophilized.

In certain embodiments, a sample is exposed to a solid substrate comprising more than one discrete volume each comprising a guide RNA set specific for a target molecule.

### Individual Discrete Volumes

The individual discrete volumes may be a bead, well, or other solid support. In another aspect, an individual discrete volume may be a droplet. The individual discrete volume can comprise a set of one or more detection beads, wherein each detection bead comprises a set of guide molecules capable of binding one or more target sequences of a target molecule. may comprise a bead or a population of beads, or a micro-bead or a population of micro-beads, micro-arrays, micro-wells, or micro-lids.

The guide molecules are distributed to individual discrete volumes to spatially separate the guide molecules. The individual discrete volume can be a solid or a liquid. The solid may be a solid support, such as a bead or well, which can be porous. The liquid may be, for example, a solution contained in a well, or a droplet. Solid supports such as beads may be further contained in a droplet or well. Separation can be by physical, chemical, optical or other means.

An "individual discrete volume" is a discrete volume or discrete space, such as a container, receptacle, or other defined volume or space that can be defined by properties that prevent and/or inhibit migration of nucleic acids, CRISPR detection systems, and reagents necessary to carry out the methods disclosed herein, for example a volume or space defined by physical properties such as walls, for example the walls of a well, tube, or a surface of a droplet, which may be impermeable or semipermeable, or as defined by other means such as chemical, diffusion rate limited, electro-magnetic, or light illumination, or any combination thereof. **In** particularly preferred embodiments, the individual discrete volumes are beads, wells or droplets. By "diffusion rate limited" (for example diffusion defined volumes) is meant spaces that are only accessible to certain molecules or reactions because diffusion constraints effectively defining a space or volume as would be the case for two parallel laminar streams where diffusion will limit the migration of a target molecule from one stream to the other. By "chemical" defined volume or space is meant spaces where only certain target molecules can exist because of their chemical or molecular properties, such as size, where for example gel beads may exclude certain species from entering the beads but not others, such as by surface charge, matrix size or other physical property of the bead that can allow selection of species that may enter the interior of the bead. By "electro-magnetically" defined volume or space is meant spaces where the electro-magnetic properties of the target molecules or their supports such as charge or magnetic properties can be used to define certain regions in a space such as capturing magnetic particles within a magnetic field or directly on magnets. By "optically" defined volume is meant any region of space that may be defined by illuminating it with visible, ultraviolet, infrared, or other wavelengths of light such that only target molecules within the defined space or volume may be labeled. One advantage to the use of non-walled, or semipermeable is that some reagents, such as buffers, chemical activators, or other agents maybe passed in or through the discrete volume, while other material, such as target molecules, maybe maintained in the discrete volume or space. As explained herein, a droplet system allows for the separation of compounds until initiation of a reaction is desired. A discrete volume can include a fluid medium, (for example, an aqueous solution, an oil, a buffer, and/or a media capable of supporting cell growth) suitable for labeling of the target molecule with the indexable nucleic acid identifier under conditions that permit labeling. Exemplary discrete volumes or spaces useful in the disclosed methods include droplets (for example, microfluidic droplets and/or emulsion droplets), hydrogel beads or other polymer structures (for example poly-ethylene glycol di-acrylate beads or agarose beads), tissue slides (for example, fixed formalin paraffin embedded tissue slides with particular regions, volumes, or spaces defined by chemical, optical, or physical means), microscope slides with regions defined by depositing reagents in ordered arrays or random patterns, tubes (such as, centrifuge tubes, microcentrifuge tubes, test tubes, cuvettes, conical tubes, and the like), bottles (such as glass bottles, plastic bottles, ceramic bottles, Erlenmeyer flasks, scintillation vials and the like), wells (such as wells in a plate), plates, pipettes, or pipette tips among others. In certain example embodiments, the individual discrete volumes are droplets. Droplet formation, size and library creation can be as described in International Patent Application PCT/US2019/061577 at [0474] - [0486].

### Beads

In an embodiment, the individual discrete volume is a bead. Sets of guide molecules can be comprised on a bead, and are referred to herein as a detection bead. Beads used in the present invention may comprise a hydrogel bead or a magnetic bead or a magnetic core. In an aspect of the embodiment, the bead is a polymer, for example, hydroxylated methacrylic polymer, a hydroxylated poly(methyl methacrylate), a polystyrene polymer, a polypropylene polymer, a polyethylene polymer agarose, or cellulose. The bead may have a porosity, throughout the bead, or in a portion of the bead. In another aspect of the embodiment, the bead has a shape that is circular, square, star, or generally rounded.

The detection bead, which comprises a set of guide molecules, and optionally a reporter molecule, can be sized between 0.5 µm and 100 µm. The detection bead may comprise guide molecules on any surface of the bead, including disposed within a porosity of the bead. In an embodiment, multiple sets of detection beads are provided, where each bead in a given set comprising guide molecules configured to detect a particular target molecule, and each different set of beads configured to detect a different target molecule such that detection of multiple target molecules is screened at once.

Beads may be used for reporter constructs. The reporter bead comprising a reporter construct can be a separate bead from the detection bead. The reporter bead may comprise a reporter construct utilizing any of the reporter molecules described herein. In an aspect, the detection bead and the reporter bead are of different sizes. In an embodiment, the reporter bead is smaller in size than the detection bead, and may be utilized with an individual discrete volume sized to hold one reporter bead and one detection bead.

In particular embodiments, the bead has an average particle size or diameter between about 0.5 microns to 100 microns, about 1 micron to 100 microns, about 2 micron to 100 microns, about 3 microns to about 100 microns, about 5 micron to 90 microns, about 10 microns to about 90 microns, about 10 microns to about 80 microns, about 10 microns to about 70 microns, about 10 microns to about 60 microns, about 10 microns to about 50 microns, about 10 microns to about 40 microns, about 10 microns to 30 microns, about 10 microns to about 20 microns, about 20 microns to about 30 microns, about 20 microns to about 40 microns, about 20 microns to about 50 microns, about 20 microns to about 60 microns, about 20 microns to about 70 microns, about 20 microns to about 80 microns, about 20 microns to about 100 microns.

The detection bead, and optionally the reporter bead, are encapsulated in a droplet. In an embodiment, the detection bead is encapsulated in a first droplet and the reporter bead is encapsulated in a second droplet that can be subsequently merged into the same droplet.

The bead may be functionalized to permit covalent attachment of the guide and/or reporter construct. Two beads of varying sizes and materials can be used; for example, a larger detection bead comprising guide molecules, and a smaller reporter bead comprising the reporter construct. Functionalization on the bead may comprise reactive groups that permit covalent attachment to the desired construct or guide molecule, and/or a label.

In one embodiment, the detection bead, reporter bead, or both may be comprised in a droplet. In one example embodiment, a detection bead is provided and emulsified into droplets. The bead may be color-coded (I.e. optically encoded) according to embodiments and may further be coupled to a guide RNA. In one embodiment, the optically encoded bead coupled to a guide RNA is provided as a set of beads that can be pooled. The pooled beads can be emulsified into droplets via microfluidics or by shaking.

The reporter bead and the detection bead are sized differently and are utilized with an individual discrete volume to hold only one of each bead type, e.g. one reporter bead and one detection bead. In an aspect, the beads can be sequentially flowed onto the microfluidic device. In an aspect, the beads are distributed to wells arrayed on a microfluidic device.

### CRISPR-Cas Systems

Embodiments disclosed herein utilize Cas proteins possessing non-specific nuclease collateral activity to cleave detectable reporters upon target recognition, providing sensitive and specific diagnostics, including single nucleotide variants, detection based on rRNA sequences, screening for drug resistance, monitoring microbe outbreaks, genetic perturbations, and screening of environmental samples, as described, for example, in PCT/US18/054472 filed October 22, 2018 at [0183] - [0327]. Reference is made to WO 2017/219027, WO2018/107129, US20180298445, US 2018-0274017, US 2018-0305773, WO 2018/170340, U.S. Application 15/922,837, filed March 15, 2018 entitled "Devices for CRISPR Effector System Based Diagnostics", PCT/US18/50091, filed September 7, 2018 "Multi-Effector CRISPR Based Diagnostic Systems", PCT/US18/66940 filed December 20, 2018 entitled "CRISPR Effector System Based Multiplex Diagnostics", PCT/US18/054472 filed October 4, 2018 entitled "CRISPR Effector System Based Diagnostic", U.S. Provisional 62/740,728 filed October 3, 2018 entitled "CRISPR Effector System Based Diagnostics for Hemorrhagic Fever Detection", U.S. Provisional 62/690,278 filed June 26, 2018 and U.S. Provisional 62/767,059 filed November 14, 2018 both entitled "CRISPR Double Nickase Based Amplification, Compositions, Systems and Methods", U.S. Provisional 62/690,160 filed June 26, 2018 and 62,767,077 filed November 14, 2018, both entitled "CRISPR/CAS and Transposase Based Amplification Compositions, Systems, And Methods", U.S. Provisional 62/690,257 filed June 26, 2018 and 62/767,052 filed November 14, 2018 both entitled "CRISPR Effector System Based Amplification Methods, Systems, And Diagnostics", US Provisional 62/767,076 filed November 14, 2018 entitled "Multiplexing Highly Evolving Viral Variants With SHERLOCK" and 62/767,070 filed November 14, 2018 entitled "Droplet SHERLOCK." Reference is further made to WO2017/127807, WO2017/184786, WO 2017/184768, WO 2017/189308, WO 2018/035388, WO 2018/170333, WO 2018/191388, WO 2018/213708, WO 2019/005866, PCT/US18/67328 filed December 21, 2018 entitled "Novel CRISPR Enzymes and Systems", PCT/US18/67225 filed December 21, 2018 entitled "Novel CRISPR Enzymes and Systems" and PCT/US18/67307 filed December 21, 2018 entitled "Novel CRISPR Enzymes and Systems", US 62/712,809 filed July 31, 2018 entitled "Novel CRISPR Enzymes and Systems", U.S. 62/744,080 filed October 10, 2018 entitled "Novel Cas12b Enzymes and Systems" and U.S. 62/751,196 filed October 26 2018 entitled "Novel Cas12b Enzymes and Systems", U.S. 715,640 filed August 7, 2-18 entitled "Novel CRISPR Enzymes and Systems", WO 2016/205711, U.S. 9,790,490, WO 2016/205749, WO 2016/205764, WO 2017/070605, WO 2017/106657, and WO 2016/149661, WO2018/035387, WO2018/194963, Cox DBT, et al., RNA editing with CRISPR-Cas13, Science. 2017 Nov 24;358(6366):1019-1027; Gootenberg JS, et al., Multiplexed and portable nucleic acid detection platform with Cas13, Cas12a, and Csm6., Science. 2018 Apr 27;360(6387):439-444; Gootenberg JS, et al., Nucleic acid detection with CRISPR-Cas13a/C2c2., Science. 2017 Apr 28;356(6336):438-442; Abudayyeh OO, et al., RNA targeting with CRISPR-Cas13, Nature. 2017 Oct 12;550(7675):280-284; Smargon AA, et al., Cas13b Is a Type VI-B CRISPR-Associated RNA-Guided RNase Differentially Regulated by Accessory Proteins Csx27 and Csx28. Mol Cell. 2017 Feb 16;65(4):618-630.e7; Abudayyeh OO, et al., C2c2 is a single-component programmable RNA-guided RNA-targeting CRISPR effector, Science. 2016 Aug 5;353(6299):aaf5573; Yang L, et al., Engineering and optimising deaminase fusions for genome editing. Nat Commun. 2016 Nov 2;7:13330, Myhrvold et al., Field deployable viral diagnostics using CRISPR-Cas13, Science 2018 360, 444-448, Shmakov et al. "Diversity and evolution of class 2 CRISPR-Cas systems," Nat Rev Microbiol. 2017 15(3):169-182.

When using two or more CRISPR effector systems, the CRISPR effector systems may be RNA-targeting effector proteins, DNA-targeting effector proteins, or a combination thereof. The RNA-targeting effector proteins may be a Type VI Cas protein, such as Cas13 protein, including Cas13b, Cas13c, or Cas13d. The DNA-targeting effector protein may be a Type V Cas protein, such as Cas12a (Cpfl), Cas12b (C2c2), or Cas12c (C2c3).

In general, a CRISPR-Cas or CRISPR system as used in herein and in documents, such as WO 2014/093622 (PCT/US2013/074667), refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). See, e.g, Shmakov et al. (2015) "Discovery and Functional Characterization of Diverse Class 2 CRISPR-Cas Systems", Molecular Cell, DOI: dx.doi.org/10.1016/j.molcel.2015.10.008.

### RNA targeting Cas protein

In an aspect, the invention utilizes an RNA targeting Cas protein. In certain embodiments, protospacer flanking site, or protospacer flanking sequence (PFS) directs binding of the effector proteins (.e.g Type VI) as disclosed herein to the target locus of interest. A PFS is a region that can affect the efficacy of Cas13a mediated targeting, and may be adjacent to the protospacer target in certain Cas13a proteins, while other orthologs do not require a specific PFS.In a preferred embodiment, the CRISPR effector protein may recognize a 3' PFS. In certain embodiments, the CRISPR effector protein may recognize a 3' PFS which is 5'H, wherein H is A, C or U. See, e.g. Abudayyeh, 2016. In certain embodiments, the effector protein may be Leptotrichia shahii Cas13p, more preferably Leptotrichia shahii DSM 19757 Cas13, and the 3' PFS is a 5' H.

In the context of formation of a CRISPR complex, "target molecule" or "target sequence" or "target nucleic acid" refers to a molecule harboring a sequence, or a sequence to which a guide sequence is designed to have complementarity, where hybridization between a target sequence and a guide sequence promotes the formation of a CRISPR complex. A target sequence may comprise RNA polynucleotides. The term "target RNA" refers to a RNA polynucleotide being or comprising the target sequence. In other words, the target RNA may be a RNA polynucleotide or a part of a RNA polynucleotide to which a part of the gRNA, i.e. the guide sequence, is designed to have complementarity and to which the effector function mediated by the complex comprising CRISPR effector protein and a gRNA is to be directed. In some embodiments, a target sequence is located in the nucleus or cytoplasm of a cell. A target sequence may comprise DNA polynucleotides.

As such, a CRISPR system may comprise RNA-targeting effector proteins. A CRISPR system may comprise DNA-targeting effector proteins. In some embodiments, a CRISPR system may comprise a combination of RNA- and DNA-targeting effector proteins, or effector proteins that target both RNA and DNA.

In some embodiments, one or more elements of a nucleic acid-targeting system is derived from a particular organism comprising an endogenous CRISPR RNA-targeting system. In certain example embodiments, the effector protein CRISPR RNA-targeting system comprises at least one HEPN domain, including but not limited to the HEPN domains described herein, HEPN domains known in the art, and domains recognized to be HEPN domains by comparison to consensus sequence motifs. Several such domains are provided herein. In one non-limiting example, a consensus sequence can be derived from the sequences of Cas13a or Cas13b orthologs provided herein. In certain example embodiments, the effector protein comprises a single HEPN domain. In certain other example embodiments, the effector protein comprises two HEPN domains.

In one example embodiment, the effector protein comprises one or more HEPN domains comprising a RxxxxH motif sequence. The RxxxxH motif sequence can be, without limitation, from a HEPN domain described herein or a HEPN domain known in the art. RxxxxH motif sequences further include motif sequences created by combining portions of two or more HEPN domains. As noted, consensus sequences can be derived from the sequences of the orthologs disclosed in U.S. Provisional Patent Application 62/432,240 entitled "Novel CRISPR Enzymes and Systems," U.S. Provisional Patent Application 62/471,710 entitled "Novel Type VI CRISPR Orthologs and Systems" filed on March 15, 2017, and U.S. Provisional Patent Application entitled "Novel Type VI CRISPR Orthologs and Systems," labeled as attorney docket number 47627-05-2133 and filed on April 12, 2017.

In an embodiment of the invention, a HEPN domain comprises at least one RxxxxH motif comprising the sequence of R(N/H/K)X1X2X3H (SEQ ID NO:7-9). In an embodiment of the invention, a HEPN domain comprises a RxxxxH motif comprising the sequence of R(N/H)X1X2X3H (SEQ ID NO: 10-11). In an embodiment of the invention, a HEPN domain comprises the sequence of R(N/K)X1X2X3H (SEQ ID NO: 12-13). In certain embodiments, X1 is R, S, D, E, Q, N, G, Y, or H. In certain embodiments, X2 is I, S, T, V, or L. In certain embodiments, X3 is L, F, N, Y, V, I, S, D, E, or A.

In particular embodiments, the Type VI RNA-targeting Cas enzyme is Cas13a. In other example embodiments, the Type VI RNA-targeting Cas enzyme is Cas13b. In certain embodiments, the Cas13b protein is from an organism of a genus selected from the group consisting of: Bergeyella, Prevotella, Porphyromonas, Bacterioides, Alistipes, Riemerella, Myroides, Capnocytophaga, Porphyromonas, Flavobacterium, Porphyromonas, Chryseobacterium, Paludibacter, Psychroflexus, Riemerella, Phaeodactylibacter, Sinomicrobium, Reichenbachiella.

In particular embodiments, the homologue or orthologue of a Type VI protein such as Cas13a as referred to herein has a sequence homology or identity of at least 30%, or at least 40%, or at least 50%, or at least 60%, or at least 70%, or at least 80%, more preferably at least 85%, even more preferably at least 90%, such as for instance at least 95% with a Type VI protein such as Cas13a (e.g., based on the wild-type sequence of any of Leptotrichia shahii Cas13a, Lachnospiraceae bacterium MA2020 Cas13a, Lachnospiraceae bacterium NK4A179 Cas13a, Clostridium aminophilum (DSM 10710) Cas13a, Carnobacterium gallinarum (DSM 4847) Cas13, Paludibacter propionicigenes (WB4) Cas13, Listeria weihenstephanensis (FSL R9-0317) Cas13, Listeriaceae bacterium (FSL M6-0635) Cas13, Listeria newyorkensis (FSL M6-0635) Cas13, Leptotrichia wadei (F0279) Cas13, Rhodobacter capsulatus (SB 1003) Cas13, Rhodobacter capsulatus (R121) Cas13, Rhodobacter capsulatus (DE442) Cas13, Leptotrichia wadei (Lw2) Cas13, or Listeria seeligeri Cas13). In further embodiments, the homologue or orthologue of a Type VI protein such as Cas13 as referred to herein has a sequence identity of at least 30%, or at least 40%, or at least 50%, or at least 60%, or at least 70%, or at least 80%, more preferably at least 85%, even more preferably at least 90%, such as for instance at least 95% with the wild type Cas13 (e.g., based on the wild-type sequence of any of Leptotrichia shahii Cas13, Lachnospiraceae bacterium MA2020 Cas13, Lachnospiraceae bacterium NK4A179 Cas13, Clostridium aminophilum (DSM 10710) Cas13, Carnobacterium gallinarum (DSM 4847) Cas13, Paludibacter propionicigenes (WB4) Cas13, Listeria weihenstephanensis (FSL R9-0317) Cas13, Listeriaceae bacterium (FSL M6-0635) Cas13, Listeria newyorkensis (FSL M6-0635) Cas13, Leptotrichia wadei (F0279) Cas13, Rhodobacter capsulatus (SB 1003) Cas13, Rhodobacter capsulatus (R121) Cas13, Rhodobacter capsulatus (DE442) Cas13, Leptotrichia wadei (Lw2) Cas13, or Listeria seeligeri Cas13).

In certain other example embodiments, the CRISPR system the effector protein is a Cas13 nuclease. The activity of Cas13 may depend on the presence of two HEPN domains. These have been shown to be RNase domains, i.e. nuclease (in particular an endonuclease) cutting RNA. Cas13a HEPN may also target DNA, or potentially DNA and/or RNA. On the basis that the HEPN domains of Cas13a are at least capable of binding to and, in their wild-type form, cutting RNA, then it is preferred that the Cas13a effector protein has RNase function. Regarding Cas13a CRISPR systems, reference is made to U.S. Provisional 62/351,662 filed on June 17, 2016 and U.S. Provisional 62/376,377 filed on August 17, 2016. Reference is also made to U.S. Provisional 62/351,803 filed on June 17, 2016. Reference is also made to U.S. Provisional entitled "Novel Crispr Enzymes and Systems" filed December 8, 2016 bearing Broad Institute No. 10035.PA4 and Attorney Docket No. 47627.03.2133. Reference is further made to East-Seletsky et al. "Two distinct RNase activities of CRISPR-C2c2 enable guide-RNA processing and RNA detection" Nature doi:10/1038/nature19802 and Abudayyeh et al. "C2c2 is a single-component programmable RNA-guided RNA targeting CRISPR effector" bioRxiv doi:10.1101/054742.

RNase function in CRISPR systems is known, for example mRNA targeting has been reported for certain type III CRISPR-Cas systems (Hale et al., 2014, Genes Dev, vol. 28, 2432-2443; Hale et al., 2009, Cell, vol. 139, 945-956; Peng et al., 2015, Nucleic acids research, vol. 43, 406-417) and provides significant advantages. In the Staphylococcus epidermis type III-A system, transcription across targets results in cleavage of the target DNA and its transcripts, mediated by independent active sites within the Cas10-Csm ribonucleoprotein effector protein complex (see, Samai et al., 2015, Cell, vol. 151, 1164-1174). A CRISPR-Cas system, composition or method targeting RNA via the present effector proteins is thus provided.

In an embodiment, the Cas protein may be a Cas13a ortholog of an organism of a genus which includes but is not limited to Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma and Campylobacter. Species of organism of such a genus can be as otherwise herein discussed.

It will be appreciated that any of the functionalities described herein may be engineered into CRISPR enzymes from other orthologs, including chimeric enzymes comprising fragments from multiple orthologs. Examples of such orthologs are described elsewhere herein. Thus, chimeric enzymes may comprise fragments of CRISPR enzyme orthologs of an organism which includes but is not limited to Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma and Campylobacter. A chimeric enzyme can comprise a first fragment and a second fragment, and the fragments can be of CRISPR enzyme orthologs of organisms of genera herein mentioned or of species herein mentioned; advantageously the fragments are from CRISPR enzyme orthologs of different species.

In embodiments, the Cas13a protein as referred to herein also encompasses a functional variant of Cas13a or a homologue or an orthologue thereof. A "functional variant" of a protein as used herein refers to a variant of such protein which retains at least partially the activity of that protein. Functional variants may include mutants (which may be insertion, deletion, or replacement mutants), including polymorphs, etc. Also included within functional variants are fusion products of such protein with another, usually unrelated, nucleic acid, protein, polypeptide or peptide. Functional variants may be naturally occurring or may be man-made. Advantageous embodiments can involve engineered or non-naturally occurring Type VI RNA-targeting effector protein.

In an embodiment, nucleic acid molecule(s) encoding the Cas13 or an ortholog or homolog thereof, may be codon-optimized for expression in a eukaryotic cell. A eukaryote can be as herein discussed. Nucleic acid molecule(s) can be engineered or non-naturally occurring.

In an embodiment, the Cas13a or an ortholog or homolog thereof, may comprise one or more mutations (and hence nucleic acid molecule(s) coding for same may have mutation(s). The mutations may be artificially introduced mutations and may include but are not limited to one or more mutations in a catalytic domain.

Examples of catalytic domains with reference to a Cas13 enzyme may include but are not limited to HEPN domains, with reference to a Cas12a, the Ruv C, e.g. RuvC I, RuvC II, RuvC III and Nuc domains.

In an embodiment, the Cas13a or an ortholog or homolog thereof, may comprise one or more mutations. The mutations may be artificially introduced mutations and may include but are not limited to one or more mutations in a catalytic domain. Examples of catalytic domains with reference to a Cas enzyme may include but are not limited to HEPN domains.

In an embodiment, the Cas13a or an ortholog or homolog thereof, may be used as a generic nucleic acid binding protein with fusion to or being operably linked to a functional domain. Exemplary functional domains may include but are not limited to translational initiator, translational activator, translational repressor, nucleases, in particular ribonucleases, a spliceosome, beads, a light inducible/controllable domain or a chemically inducible/controllable domain.

In certain example embodiments, the Cas13a effector protein may be from an organism selected from the group consisting of; Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, and Campylobacter.

In certain embodiments, the effector protein may be a Listeria sp. Cas13p, preferably Listeria seeligeria Cas13p, more preferably Listeria seeligeria serovar 1/2b str. SLCC3954 Cas13p and the crRNA sequence may be 44 to 47 nucleotides in length, with a 5' 29-nt direct repeat (DR) and a 15-nt to 18-nt spacer.

In certain embodiments, the effector protein may be a Leptotrichia sp. Cas13p, preferably Leptotrichia shahii Cas13p, more preferably Leptotrichia shahii DSM 19757 Cas13p and the crRNA sequence may be 42 to 58 nucleotides in length, with a 5' direct repeat of at least 24 nt, such as a 5' 24-28-nt direct repeat (DR) and a spacer of at least 14 nt, such as a 14-nt to 28-nt spacer, or a spacer of at least 18 nt, such as 19, 20, 21, 22, or more nt, such as 18-28, 19-28, 20-28, 21-28, or 22-28 nt.

In certain example embodiments, the effector protein may be a Leptotrichia sp., Leptotrichia wadei F0279, or a Listeria sp., preferably Listeria newyorkensis FSL M6-0635.

In certain example embodiments, the Cas13 effector proteins of the invention include, without limitation, the following 21 ortholog species (including multiple CRISPR loci: Leptotrichia shahii; Leptotrichia wadei (Lw2); Listeria seeligeri; Lachnospiraceae bacterium MA2020; Lachnospiraceae bacterium NK4A179; [Clostridium] aminophilum DSM 10710; Carnobacterium gallinarum DSM 4847; Carnobacterium gallinarum DSM 4847 (second CRISPR Loci); Paludibacter propionicigenes WB4; Listeria weihenstephanensis FSL R9-0317; Listeriaceae bacterium FSL M6-0635; Leptotrichia wadei F0279; Rhodobacter capsulatus SB 1003; Rhodobacter capsulatus R121; Rhodobacter capsulatus DE442; Leptotrichia buccalis C-1013-b; Herbinix hemicellulosilytica; [Eubacterium] rectale; Eubacteriaceae bacterium CHKCI004; Blautia sp. Marseille-P2398; and Leptotrichia sp. oral taxon 879 str. F0557. Twelve (12) further non-limiting examples are: Lachnospiraceae bacterium NK4A144; Chloroflexus aggregans; Demequina aurantiaca; Thalassospira sp. TSL5-1; Pseudobutyrivibrio sp. OR37; Butyrivibrio sp. YAB3001; Blautia sp. Marseille-P2398; Leptotrichia sp. Marseille-P3007; Bacteroides ihuae; Porphyromonadaceae bacterium KH3CP3RA; Listeria riparia; and Insolitispirillum peregrinum.

In certain embodiments, the Cas13 protein according to the invention is or is derived from one of the orthologues as described in the table below, or is a chimeric protein of two or more of the orthologues as described in the table below, or is a mutant or variant of one of the orthologues as described in the table below (or a chimeric mutant or variant), including dead Cas13, split Cas13, destabilized Cas13, etc. as defined herein elsewhere, with or without fusion with a heterologous/functional domain.

In certain example embodiments, the Cas13a effector protein is from an organism of a genus selected from the group consisting of: Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Campylobacter, and Lachnospira.

In an embodiment of the invention, there is provided an effector protein which comprises an amino acid sequence having at least 80% sequence homology to the wild-type sequence of any of Leptotrichia shahii Cas13, Lachnospiraceae bacterium MA2020 Cas13, Lachnospiraceae bacterium NK4A179 Cas13, Clostridium aminophilum (DSM 10710) Cas13, Carnobacterium gallinarum (DSM 4847) Cas13, Paludibacter propionicigenes (WB4) Cas13, Listeria weihenstephanensis (FSL R9-0317) Cas13, Listeriaceae bacterium (FSL M6-0635) Cas13, Listeria newyorkensis (FSL M6-0635) Cas13, Leptotrichia wadei (F0279) Cas13, Rhodobacter capsulatus (SB 1003) Cas13, Rhodobacter capsulatus (R121) Cas13, Rhodobacter capsulatus (DE442) Cas13, Leptotrichia wadei (Lw2) Cas13, or Listeria seeligeri Cas13. According to the invention, a consensus sequence can be generated from multiple Cas13 orthologs, which can assist in locating conserved amino acid residues, and motifs, including but not limited to catalytic residues and HEPN motifs in Cas13 orthologs that mediate Cas13 function. One such consensus sequence, generated from selected orthologs.

In an embodiment of the invention, the effector protein comprises an amino acid sequence having at least 80% sequence homology to a Type VI effector protein consensus sequence including but not limited to a consensus sequence described herein.

In another non-limiting example, a sequence alignment tool to assist generation of a consensus sequence and identification of conserved residues is the MUSCLE alignment tool (www.ebi.ac.uk/Tools/msa/muscle/). For example, using MUSCLE, the following amino acid locations conserved among Cas13a orthologs can be identified in Leptotrichia wadei Cas13a:K2; K5; V6; E301; L331; I335; N341; G351; K352; E375; L392; L396; D403; F446; I466; I470; R474 (HEPN); H475; H479 (HEPN), E508; P556; L561; I595; Y596; F600; Y669; I673; F681; L685; Y761; L676; L779; Y782; L836; D847; Y863; L869; I872; K879; I933; L954; I958; R961; Y965; E970; R971; D972; R1046 (HEPN), H1051 (HEPN), Y1075; D1076; K1078; K1080; I1083; I1090.

In certain example embodiments, the RNA-targeting effector protein is a Type VI-B effector protein, such as Cas13b and Group 29 or Group 30 proteins. In certain example embodiments, the RNA-targeting effector protein comprises one or more HEPN domains. In certain example embodiments, the RNA-targeting effector protein comprises a C-terminal HEPN domain, a N-terminal HEPN domain, or both. Regarding example Type VI-B effector proteins that may be used in the context of this invention, reference is made to US Application No. 15/331,792 entitled "Novel CRISPR Enzymes and Systems" and filed October 21, 2016, International Patent Application No. PCT/US2016/058302 entitled "Novel CRISPR Enzymes and Systems", and filed October 21, 2016, and Smargon et al. "Cas13b is a Type VI-B CRISPR-associated RNA-Guided RNase differentially regulated by accessory proteins Csx27 and Csx28" Molecular Cell, 65, 1-13 (2017); dx.doi.org/10.1016/j.molcel.2016.12.023. In certain example embodiments, the Cas13b effector protein is, or comprises an amino acid sequence having at least 80% sequence homology to any of the sequences of Table 1 of International Patent Application No. PCT/US2016/058302. Further reference is made to example Type VI-B effector proteins of U.S. Provisional Application Nos. 62/471,710, 62/566,829 and International Patent Publication No. WO2018/1703333, entitled "Novel Cas13b Orthologues CRISPR Enzymes and System". In particular embodiments, the Cas13b enzyme is derived from Bergeyella zoohelcum. In certain other example embodiments, the effector protein is, or comprises an amino acid sequence having at least 80% sequence homology to any of the sequences listed in Tables 1A or 1B of International Patent Publication No. WO2018/1703333. In certain embodiments, the Cas 13b effector protein is, or comprises an amino acid sequence having at least 80% sequence homology to any of the polypeptides in U.S. Provisional Applications 62/484,791, 62/561,662, 62/568,129 or International Patent Publication WO2018/191388, all entitled "Novel Type VI CRISPR Orthologs and Systems,". In certain embodiments, the Cas13b effector protein is, or comprises an amin acid sequence having at least 80% sequence homology to a polypeptide as set forth in FIG. 1 of International Patent Publication WO2018/191388. In an aspect, the Cas13b protein is selected from the group consisting of Porphyromonas gulae Casl3b (accession number WP 039434803), Prevotella sp. P5-125 Cas 13b (accession number WP 044065294), Porphyromonas gingivalis Cas 13b (accession number WP 053444417), Porphyromonas sp. COT-052 OH4946 Cas 13b (accession number WP 039428968), Bacteroides pyogenes Cas 13b (accession number WP 034542281), Riemerella anatipestifer Casl3b (accession number WP 004919755).

In certain example embodiments, the RNA-targeting effector protein is a Cas13c effector protein as disclosed in U.S. Provisional Patent Application No. 62/525,165 filed June 26, 2017, and International Patent Publication No. WO2018/035250 filed August 16, 2017. In certain example embodiments, the Cas13c protein may be from an organism of a genus such as Fusobacterium or Anaerosalibacter. Example wildtype orthologue sequences of Cas13c are: EH019081, WP_094899336, WP_040490876, WP_047396607, WP_035935671, WP_035906563, WP_042678931, WP_062627846, WP_005959231, WP_027128616, WP_062624740, WP_096402050.

In certain example embodiments, the Cas13 protein may be selected from any of the following: Cas13a: Leptotrichia shahii, Leptotrichia wadei (Lw2), Listeria seeligeri, Lachnospiraceae bacterium MA2020, Lachnospiraceae bacterium NK4A179, [Clostridium] aminophilum DSM 10710, Carnobacterium gallinarum DSM 4847 , Carnobacterium gallinarum DSM 4847, Paludibacter propionicigenes WB4, Listeria weihenstephanensis FSL R9-0317, Listeriaceae bacterium FSL M6-0635, Leptotrichia wadei F0279, Rhodobacter capsulatus SB 1003, Rhodobacter capsulatus R121, Rhodobacter capsulatus DE442, Leptotrichia buccalis C-1013-b, Herbinix hemicellulosilytica, [Eubacterium] rectale, Eubacteriaceae bacterium CHKCI004, Blautia sp. Marseille-P2398, Leptotrichia sp. oral taxon 879 str. F0557; Cas 13b: Bergeyella zoohelcum, Prevotella intermedia, Prevotella buccae, Alistipes sp. ZOR0009, Prevotella sp. MA2016, Riemerella anatipestifer, Prevotella aurantiaca, Prevotella saccharolytica, Prevotella intermedia, Capnocytophaga canimorsus, Porphyromonas gulae, Prevotella sp. P5-125, Flavobacterium branchiophilum, Porphyromonas gingivalis, Prevotella intermedia; Cas13c: Fusobacterium necrophorum subsp. funduliforme ATCC 51357 contig00003, Fusobacterium necrophorum DJ-2 contig0065, whole genome shotgun sequence, Fusobacterium necrophorum BFTR-1 contig0068, Fusobacterium necrophorum subsp. funduliforme 1_1_36S cont1.14, Fusobacterium perfoetens ATCC 29250 T364DRAFT_scaffold00009.9_C, Fusobacterium ulcerans ATCC 49185 cont2.38, Anaerosalibacter sp. ND1 genome assembly Anaerosalibacter massiliensis ND1.Cas13s non-specific RNase activity can be leveraged to cleave reporters upon target recognition, allowing for the design of sensitive and specific diagnostics using Cas13, including single nucleotide variants, detection based on rRNA sequences, screening for drug resistance, monitoring microbe outbreaks, genetic perturbations, and screening of environmental samples, as described, for example, in PCT/US18/054472 filed October 22, 2018 at [0183] - [0327]. Reference is made to WO 2017/219027, WO2018/107129, US20180298445, US 2018-0274017, US 2018-0305773, WO 2018/170340, U.S. Application 15/922,837, filed March 15, 2018 entitled "Devices for CRISPR Effector System Based Diagnostics", PCT/US18/50091, filed September 7, 2018 "Multi-Effector CRISPR Based Diagnostic Systems", PCT/US18/66940 filed December 20, 2018 entitled "CRISPR Effector System Based Multiplex Diagnostics", PCT/US18/054472 filed October 4, 2018 entitled "CRISPR Effector System Based Diagnostic", U.S. Provisional 62/740,728 filed October 3, 2018 entitled "CRISPR Effector System Based Diagnostics for Hemorrhagic Fever Detection", U.S. Provisional 62/690,278 filed June 26, 2018 and U.S. Provisional 62/767,059 filed November 14, 2018 both entitled "CRISPR Double Nickase Based Amplification, Compositions, Systems and Methods", U.S. Provisional 62/690,160 filed June 26, 2018 and 62,767,077 filed November 14, 2018, both entitled "CRISPR/CAS and Transposase Based Amplification Compositions, Systems, And Methods", U.S. Provisional 62/690,257 filed June 26, 2018 and 62/767,052 filed November 14, 2018 both entitled "CRISPR Effector System Based Amplification Methods, Systems, And Diagnostics", US Provisional 62/767,076 filed November 14, 2018 entitled "Multiplexing Highly Evolving Viral Variants With SHERLOCK" and 62/767,070 filed November 14, 2018 entitled "Droplet SHERLOCK." Reference is further made to WO2017/127807, WO2017/184786, WO 2017/184768, WO 2017/189308, WO 2018/035388, WO 2018/170333, WO 2018/191388, WO 2018/213708, WO 2019/005866, PCT/US18/67328 filed December 21, 2018 entitled "Novel CRISPR Enzymes and Systems", PCT/US18/67225 filed December 21, 2018 entitled "Novel CRISPR Enzymes and Systems"and PCT/US18/67307 filed December 21, 2018 entitled "Novel CRISPR Enzymes and Systems", US 62/712,809 filed July 31, 2018 entitled "Novel CRISPR Enzymes and Systems", U.S. 62/744,080 filed October 10, 2018 entitled "Novel Cas12b Enzymes and Systems" and U.S. 62/751,196 filed October 26 2018 entitled "Novel Cas12b Enzymes and Systems", U.S. 715,640 filed August 7, 2-18 entitled "Novel CRISPR Enzymes and Systems", WO 2016/205711, U.S. 9,790,490, WO 2016/205749, WO 2016/205764, WO 2017/070605, WO 2017/106657, and WO 2016/149661, WO2018/035387, WO2018/194963, Cox DBT, et al., RNA editing with CRISPR-Cas13, Science. 2017 Nov 24;358(6366):1019-1027; Gootenberg JS, et al., Multiplexed and portable nucleic acid detection platform with Cas13, Cas12a, and Csm6., Science. 2018 Apr 27;360(6387):439-444; Gootenberg JS, et al., Nucleic acid detection with CRISPR-Cas13a/C2c2., Science. 2017 Apr 28;356(6336):438-442; Abudayyeh OO, et al., RNA targeting with CRISPR-Cas13, Nature. 2017 Oct 12;550(7675):280-284; Smargon AA, et al., Cas13b Is a Type VI-B CRISPR-Associated RNA-Guided RNase Differentially Regulated by Accessory Proteins Csx27 and Csx28. Mol Cell. 2017 Feb 16;65(4):618-630.e7; Abudayyeh OO, et al., C2c2 is a single-component programmable RNA-guided RNA-targeting CRISPR effector, Science. 2016 Aug 5;353(6299):aaf5573; Yang L, et al., Engineering and optimising deaminase fusions for genome editing. Nat Commun. 2016 Nov 2;7:13330, Myrvhold et al., Field deployable viral diagnostics using CRISPR-Cas13, Science 2018 360, 444-448, Shmakov et al. "Diversity and evolution of class 2 CRISPR-Cas systems," Nat Rev Microbiol. 2017 15(3):169-182.

### DNA-Targeting Effector Proteins

In certain example embodiments, the assays may comprise a DNA-targeting effector protein. In certain example embodiments, the assays may comprise multiple DNA-targeting effectors or one or more orthologs in combination with one or more RNA-targeting effectors. In certain example embodiments, the DNA targeting are Type V Cas proteins, such as Cas12 proteins. In certain other example embodiments, the Cas12 proteins are Cas12a, Cas12b, Cas12c, or a combination thereof.

### Cas 12a Orthologs

The present invention encompasses the use of a Cpf1 effector protein, derived from a Cpfl locus denoted as subtype V-A. Herein such effector proteins are also referred to as "Cpf1p", e.g., a Cpfl protein (and such effector protein or Cpf1 protein or protein derived from a Cpfl locus is also called "CRISPR enzyme"). Presently, the subtype V-A loci encompasses cas1, cas2, a distinct gene denoted cpf1 and a CRISPR array. Cpf1(CRISPR-associated protein Cpf1, subtype PREFRAN) is a large protein (about 1300 amino acids) that contains a RuvC-like nuclease domain homologous to the corresponding domain of Cas9 along with a counterpart to the characteristic arginine-rich cluster of Cas9. However, Cpf1 lacks the HNH nuclease domain that is present in all Cas9 proteins, and the RuvC-like domain is contiguous in the Cpf1 sequence, in contrast to Cas9 where it contains long inserts including the HNH domain. Accordingly, in particular embodiments, the CRISPR-Cas enzyme comprises only a RuvC-like nuclease domain.

The programmability, specificity, and collateral activity of the RNA-guided Cpfl also make it an ideal switchable nuclease for non-specific cleavage of nucleic acids. In one embodiment, a Cpfl system is engineered to provide and take advantage of collateral non-specific cleavage of RNA. In another embodiment, a Cpf1 system is engineered to provide and take advantage of collateral non-specific cleavage of ssDNA. Accordingly, engineered Cpfl systems provide platforms for nucleic acid detection and transcriptome manipulation. Cpf1 is developed for use as a mammalian transcript knockdown and binding tool. Cpf1 is capable of robust collateral cleavage of RNA and ssDNA when activated by sequence-specific targeted DNA binding.

Homologs and orthologs may be identified by homology modelling (see, e.g., Greer, Science vol. 228 (1985) 1055, and Blundell et al. Eur J Biochem vol 172 (1988), 513) or "structural BLAST" (Dey F, Cliff Zhang Q, Petrey D, Honig B. Toward a "structural BLAST": using structural relationships to infer function. Protein Sci. 2013 Apr;22(4):359-66. doi: 10.1002/pro.2225.). See also Shmakov et al. (2015) for application in the field of CRISPR-Cas loci. Homologous proteins may but need not be structurally related, or are only partially structurally related. The Cpfl gene is found in several diverse bacterial genomes, typically in the same locus with cas1, cas2, and cas4 genes and a CRISPR cassette (for example, FNFX1_1431-FNFX1_1428 of Francisella cf . novicida Fx1). In particular embodiments, the effector protein is a Cpfl effector protein from an organism from a genus comprising Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium or Acidaminococcus.

In further particular embodiments, the Cpfl effector protein is from an organism selected from S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii.

The effector protein may comprise a chimeric effector protein comprising a first fragment from a first effector protein (e.g., a Cpfl) ortholog and a second fragment from a second effector (e.g., a Cpf1) protein ortholog, and wherein the first and second effector protein orthologs are different. At least one of the first and second effector protein (e.g., a Cpfl) orthologs may comprise an effector protein (e.g., a Cpfl) from an organism comprising Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium or Acidaminococcus; e.g., a chimeric effector protein comprising a first fragment and a second fragment wherein each of the first and second fragments is selected from a Cpfl of an organism comprising Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium or Acidaminococcus wherein the first and second fragments are not from the same bacteria; for instance a chimeric effector protein comprising a first fragment and a second fragment wherein each of the first and second fragments is selected from a Cpfl of S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii; Francisella tularensis 1, Prevotella albensis, Lachnospiraceae bacterium MC2017 1, Butyrivibrio proteoclasticus, Peregrinibacteria bacterium GW2011_GWA2_33_10, Parcubacteria bacterium GW2011_GWC2_44_17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens and Porphyromonas macacae, wherein the first and second fragments are not from the same bacteria. In a more preferred embodiment, the Cpf1p is derived from a bacterial species selected from Francisella tularensis 1, Prevotella albensis, Lachnospiraceae bacterium MC2017 1, Butyrivibrio proteoclasticus, Peregrinibacteria bacterium GW2011_GWA2_33_10, Parcubacteria bacterium GW2011_GWC2_44_17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens and Porphyromonas macacae. In certain embodiments, the Cpf1p is derived from a bacterial species selected from Acidaminococcus sp. BV3L6, Lachnospiraceae bacterium MA2020. In certain embodiments, the effector protein is derived from a subspecies of Francisella tularensis 1, including but not limited to Francisella tularensis subsp. Novicida.

In some embodiments, the Cpf1p is derived from an organism from the genus of Eubacterium. In some embodiments, the CRISPR effector protein is a Cpf1 protein derived from an organism from the bacterial species of Eubacterium rectale. In some embodiments, the amino acid sequence of the Cpfl effector protein corresponds to NCBI Reference Sequence WP_055225123.1, NCBI Reference Sequence WP_055237260.1, NCBI Reference Sequence WP_055272206.1, or GenBank ID OLA16049.1. In some embodiments, the Cpfl effector protein has a sequence homology or sequence identity of at least 60%, more particularly at least 70, such as at least 80%, more preferably at least 85%, even more preferably at least 90%, such as for instance at least 95%, with NCBI Reference Sequence WP_055225123.1, NCBI Reference Sequence WP_055237260.1, NCBI Reference Sequence WP_055272206.1, or GenBank ID OLA16049.1. The skilled person will understand that this includes truncated forms of the Cpfl protein whereby the sequence identity is determined over the length of the truncated form. In some embodiments, the Cpfl effector recognizes the PAM sequence of TTTN or CTTN.

In particular embodiments, the homologue or orthologue of Cpfl as referred to herein has a sequence homology or identity of at least 80%, more preferably at least 85%, even more preferably at least 90%, such as for instance at least 95% with Cpfl. In further embodiments, the homologue or orthologue of Cpfl as referred to herein has a sequence identity of at least 80%, more preferably at least 85%, even more preferably at least 90%, such as for instance at least 95% with the wild type Cpfl. Where the Cpf1 has one or more mutations (mutated), the homologue or orthologue of said Cpfl as referred to herein has a sequence identity of at least 80%, more preferably at least 85%, even more preferably at least 90%, such as for instance at least 95% with the mutated Cpf1.

In an embodiment, the Cpfl protein may be an ortholog of an organism of a genus which includes, but is not limited to Acidaminococcus sp, Lachnospiraceae bacterium or Moraxella bovoculi; in particular embodiments, the type V Cas protein may be an ortholog of an organism of a species which includes, but is not limited to Acidaminococcus sp. BV3L6; Lachnospiraceae bacterium ND2006 (LbCpf1) or Moraxella bovoculi 237.In particular embodiments, the homologue or orthologue of Cpfl as referred to herein has a sequence homology or identity of at least 80%, more preferably at least 85%, even more preferably at least 90%, such as for instance at least 95% with one or more of the Cpf1 sequences disclosed herein. In further embodiments, the homologue or orthologue of Cpf as referred to herein has a sequence identity of at least 80%, more preferably at least 85%, even more preferably at least 90%, such as for instance at least 95% with the wild type FnCpf1, AsCpf1 or LbCpf1. The skilled person will understand that this includes truncated forms of the Cpfl protein whereby the sequence identity is determined over the length of the truncated form. In certain of the following, Cpfl amino acids are followed by nuclear localization signals (NLS) (italics), a glycine-serine (GS) linker, and 3x HA tag. Further Cpfl orthologs include NCBI WP_055225123.1, NCBI WP_055237260.1, NCBI WP_055272206.1, and GenBank OLA16049.1.

### Cas 12b Orthologs

The present invention encompasses the use of a Cas 12b (C2c1) effector proteins, derived from a C2c1 locus denoted as subtype V-B. Herein such effector proteins are also referred to as "C2c1p", e.g., a C2c1 protein (and such effector protein or C2c1 protein or protein derived from a C2c1 locus is also called "CRISPR enzyme"). Presently, the subtype V-B loci encompasses cas1-Cas4 fusion, cas2, a distinct gene denoted C2c1 and a CRISPR array. C2c1 (CRISPR-associated protein C2c1) is a large protein (about 1100 - 1300 amino acids) that contains a RuvC-like nuclease domain homologous to the corresponding domain of Cas9 along with a counterpart to the characteristic arginine-rich cluster of Cas9. However, C2c1 lacks the HNH nuclease domain that is present in all Cas9 proteins, and the RuvC-like domain is contiguous in the C2c1 sequence, in contrast to Cas9 where it contains long inserts including the HNH domain. Accordingly, in particular embodiments, the CRISPR-Cas enzyme comprises only a RuvC-like nuclease domain.

The programmability, specificity, and collateral activity of the RNA-guided C2c1 also make it an ideal switchable nuclease for non-specific cleavage of nucleic acids. In one embodiment, a C2c1 system is engineered to provide and take advantage of collateral non-specific cleavage of RNA. In another embodiment, a C2c1 system is engineered to provide and take advantage of collateral non-specific cleavage of ssDNA. Accordingly, engineered C2c1 systems provide platforms for nucleic acid detection and transcriptome manipulation, and inducing cell death. C2c1 is developed for use as a mammalian transcript knockdown and binding tool. C2c1 is capable of robust collateral cleavage of RNA and ssDNA when activated by sequence-specific targeted DNA binding.

In certain embodiments, C2c1 is provided or expressed in an in vitro system or in a cell, transiently or stably, and targeted or triggered to non-specifically cleave cellular nucleic acids. In one embodiment, C2c1 is engineered to knock down ssDNA, for example viral ssDNA. In another embodiment, C2c1 is engineered to knock down RNA. The system can be devised such that the knockdown is dependent on a target DNA present in the cell or in vitro system, or triggered by the addition of a target nucleic acid to the system or cell.

C2c1 (also known as Cas12b) proteins are RNA guided nucleases. In certain embodiments, the Cas protein may comprise at least 80% sequence identity to a polypeptide as described in International Patent Publication WO 2016/205749 at Fig. 17-21, Fig. 41A-41M, 44A-44E. Its cleavage relies on a tracr RNA to recruit a guide RNA comprising a guide sequence and a direct repeat, where the guide sequence hybridizes with the target nucleotide sequence to form a DNA/RNA heteroduplex. Based on current studies, C2c1 nuclease activity also requires relies on recognition of PAM sequence. C2c1 PAM sequences are T-rich sequences. In some embodiments, the PAM sequence is 5' TTN 3' or 5' ATTN 3', wherein N is any nucleotide. In a particular embodiment, the PAM sequence is 5' TTC 3'. In a particular embodiment, the PAM is in the sequence of Plasmodium falciparum.

In particular embodiments, the effector protein is a C2c1 effector protein from an organism from a genus comprising Alicyclobacillus, Desulfovibrio, Desulfonatronum, Opitutaceae, Tuberibacillus, Bacillus, Brevibacillus, Candidatus, Desulfatirhabdium, Citrobacter, Elusimicrobia, Methylobacterium, Omnitrophica, Phycisphaerae, Planctomycetes, Spirochaetes, and Verrucomicrobiaceae.

In further particular embodiments, the C2c1 effector protein is from a species selected from Alicyclobacillus acidoterrestris (e.g., ATCC 49025), Alicyclobacillus contaminans (e.g., DSM 17975), Alicyclobacillus macrosporangiidus (e.g. DSM 17980), Bacillus hisashii strain C4, Candidatus Lindowbacteria bacterium RIFCSPLOWO2, Desulfovibrio inopinatus (e.g., DSM 10711), Desulfonatronum thiodismutans (e.g., strain MLF-1), Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWB1_27_13, Verrucomicrobiaceae bacterium UBA2429, Tuberibacillus calidus (e.g., DSM 17572), Bacillus thermoamylovorans (e.g., strain B4166), Brevibacillus sp. CF112, Bacillus sp. NSP2.1, Desulfatirhabdium butyrativorans (e.g., DSM 18734), Alicyclobacillus herbarius (e.g., DSM 13609), Citrobacter freundii (e.g., ATCC 8090), Brevibacillus agri (e.g., BAB-2500), Methylobacterium nodulans (e.g., ORS 2060).

The effector protein may comprise a chimeric effector protein comprising a first fragment from a first effector protein (e.g., a C2c1) ortholog and a second fragment from a second effector (e.g., a C2c1) protein ortholog, and wherein the first and second effector protein orthologs are different. At least one of the first and second effector protein (e.g., a C2c1) orthologs may comprise an effector protein (e.g., a C2c1) from an organism comprising Alicyclobacillus, Desulfovibrio, Desulfonatronum, Opitutaceae, Tuberibacillus, Bacillus, Brevibacillus, Candidatus, Desulfatirhabdium, Elusimicrobia, Citrobacter, Methylobacterium, Omnitrophicai, Phycisphaerae, Planctomycetes, Spirochaetes, and Verrucomicrobiaceae ; e.g., a chimeric effector protein comprising a first fragment and a second fragment wherein each of the first and second fragments is selected from a C2c1 of an organism comprising Alicyclobacillus, Desulfovibrio, Desulfonatronum, Opitutaceae, Tuberibacillus, Bacillus, Brevibacillus, Candidatus, Desulfatirhabdium, Elusimicrobia, Citrobacter, Methylobacterium, Omnitrophicai, Phycisphaerae, Planctomycetes, Spirochaetes, and Verrucomicrobiaceae wherein the first and second fragments are not from the same bacteria; for instance a chimeric effector protein comprising a first fragment and a second fragment wherein each of the first and second fragments is selected from a C2c1 of Alicyclobacillus acidoterrestris (e.g., ATCC 49025), Alicyclobacillus contaminans (e.g., DSM 17975), Alicyclobacillus macrosporangiidus (e.g. DSM 17980), Bacillus hisashii strain C4, Candidatus Lindowbacteria bacterium RIFCSPLOWO2, Desulfovibrio inopinatus (e.g., DSM 10711), Desulfonatronum thiodismutans (e.g., strain MLF-1), Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWB1_27_13, Verrucomicrobiaceae bacterium UBA2429, Tuberibacillus calidus (e.g., DSM 17572), Bacillus thermoamylovorans (e.g., strain B4166), Brevibacillus sp. CF112, Bacillus sp. NSP2.1, Desulfatirhabdium butyrativorans (e.g., DSM 18734), Alicyclobacillus herbarius (e.g., DSM 13609), Citrobacter freundii (e.g., ATCC 8090), Brevibacillus agri (e.g., BAB-2500), Methylobacterium nodulans (e.g., ORS 2060) , wherein the first and second fragments are not from the same bacteria.

In a more preferred embodiment, the C2c1p is derived from a bacterial species selected from Alicyclobacillus acidoterrestris (e.g., ATCC 49025), Alicyclobacillus contaminans (e.g., DSM 17975), Alicyclobacillus macrosporangiidus (e.g. DSM 17980), Bacillus hisashii strain C4, Candidatus Lindowbacteria bacterium RIFCSPLOWO2, Desulfovibrio inopinatus (e.g., DSM 10711), Desulfonatronum thiodismutans (e.g., strain MLF-1), Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWB1_27_13, Verrucomicrobiaceae bacterium UBA2429, Tuberibacillus calidus (e.g., DSM 17572), Bacillus thermoamylovorans (e.g., strain B4166), Brevibacillus sp. CF112, Bacillus sp. NSP2.1, Desulfatirhabdium butyrativorans (e.g., DSM 18734), Alicyclobacillus herbarius (e.g., DSM 13609), Citrobacter freundii (e.g., ATCC 8090), Brevibacillus agri (e.g., BAB-2500), Methylobacterium nodulans (e.g., ORS 2060). In certain embodiments, the C2c1p is derived from a bacterial species selected from Alicyclobacillus acidoterrestris (e.g., ATCC 49025), Alicyclobacillus contaminans (e.g., DSM 17975).

In particular embodiments, the homologue or orthologue of C2c1 as referred to herein has a sequence homology or identity of at least 80%, more preferably at least 85%, even more preferably at least 90%, such as for instance at least 95% with C2c1. In further embodiments, the homologue or orthologue of C2c1 as referred to herein has a sequence identity of at least 80%, more preferably at least 85%, even more preferably at least 90%, such as for instance at least 95% with the wild type C2c1. Where the C2c1 has one or more mutations (mutated), the homologue or orthologue of said C2c1 as referred to herein has a sequence identity of at least 80%, more preferably at least 85%, even more preferably at least 90%, such as for instance at least 95% with the mutated C2c1.

In an embodiment, the C2c1 protein may be an ortholog of an organism of a genus which includes, but is not limited to *Alicyclobacillus, Desulfovibrio, Desulfonatronum, Opitutaceae, Tuberibacillus, Bacillus, Brevibacillus, Candidatus, Desulfatirhabdium, Elusimicrobia, Citrobacter, Methylobacterium, Omnitrophicai, Phycisphaerae, Planctomycetes, Spirochaetes,* and *Verrucomicrobiaceae*; in particular embodiments, the type V Cas protein may be an ortholog of an organism of a species which includes, but is not limited to Alicyclobacillus acidoterrestris (e.g., ATCC 49025), Alicyclobacillus contaminans (e.g., DSM 17975), Alicyclobacillus macrosporangiidus (e.g. DSM 17980), Bacillus hisashii strain C4, Candidatus Lindowbacteria bacterium RIFCSPLOWO2, Desulfovibrio inopinatus (e.g., DSM 10711), Desulfonatronum thiodismutans (e.g., strain MLF-1), Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWB1_27_13, Verrucomicrobiaceae bacterium UBA2429, Tuberibacillus calidus (e.g., DSM 17572), Bacillus thermoamylovorans (e.g., strain B4166), Brevibacillus sp. CF112, Bacillus sp. NSP2.1, Desulfatirhabdium butyrativorans (e.g., DSM 18734), Alicyclobacillus herbarius (e.g., DSM 13609), Citrobacter freundii (e.g., ATCC 8090), Brevibacillus agri (e.g., BAB-2500), Methylobacterium nodulans (e.g., ORS 2060).In particular embodiments, the homologue or orthologue of C2c1 as referred to herein has a sequence homology or identity of at least 80%, more preferably at least 85%, even more preferably at least 90%, such as for instance at least 95% with one or more of the C2c1 sequences disclosed herein. In further embodiments, the homologue or orthologue of C2c1 as referred to herein has a sequence identity of at least 80%, more preferably at least 85%, even more preferably at least 90%, such as for instance at least 95% with the wild type AacC2c1 or BthC2c1.

In particular embodiments, the C2c1 protein of the invention has a sequence homology or identity of at least 60%, more particularly at least 70, such as at least 80%, more preferably at least 85%, even more preferably at least 90%, such as for instance at least 95% with AacC2c1 or BthC2c1. In further embodiments, the C2c1 protein as referred to herein has a sequence identity of at least 60%, such as at least 70%, more particularly at least 80%, more preferably at least 85%, even more preferably at least 90%, such as for instance at least 95% with the wild type AacC2c1. In particular embodiments, the C2c1 protein of the present invention has less than 60% sequence identity with AacC2c1. The skilled person will understand that this includes truncated forms of the C2c1 protein whereby the sequence identity is determined over the length of the truncated form.

In certain methods according to the present invention, the CRISPR-Cas protein is preferably mutated with respect to a corresponding wild-type enzyme such that the mutated CRISPR-Cas protein lacks the ability to cleave one or both DNA strands of a target locus containing a target sequence. In particular embodiments, one or more catalytic domains of the C2c1 protein are mutated to produce a mutated Cas protein which cleaves only one DNA strand of a target sequence.

In particular embodiments, the CRISPR-Cas protein may be mutated with respect to a corresponding wild-type enzyme such that the mutated CRISPR-Cas protein lacks substantially all DNA cleavage activity. In some embodiments, a CRISPR-Cas protein may be considered to substantially lack all DNA and/or RNA cleavage activity when the cleavage activity of the mutated enzyme is about no more than 25%, 10%, 5%, 1%, 0.1%, 0.01%, or less of the nucleic acid cleavage activity of the non-mutated form of the enzyme; an example can be when the nucleic acid cleavage activity of the mutated form is nil or negligible as compared with the non-mutated form.

In certain embodiments of the methods provided herein the CRISPR-Cas protein is a mutated CRISPR-Cas protein which cleaves only one DNA strand, i.e. a nickase. More particularly, in the context of the present invention, the nickase ensures cleavage within the non-target sequence, i.e. the sequence which is on the opposite DNA strand of the target sequence and which is 3' of the PAM sequence. By means of further guidance, and without limitation, an arginine-to-alanine substitution (R911A) in the Nuc domain of C2c1 from Alicyclobacillus acidoterrestris converts C2c1 from a nuclease that cleaves both strands to a nickase (cleaves a single strand). It will be understood by the skilled person that where the enzyme is not AacC2c1, a mutation may be made at a residue in a corresponding position.

### Cas 12c orthologs

In certain embodiments, the effector protein, particularly a Type V loci effector protein, more particularly a Type V-C loci effector protein, a Cas12c protein, even more particularly a C2c3p, may originate, may be isolated or may be derived from a bacterial metagenome selected from the group consisting of the bacterial metagenomes listed in the Table in Fig. 43A-43B of PCT/US2016/038238, which presents analysis of the Type-V-C Cas12c loci.

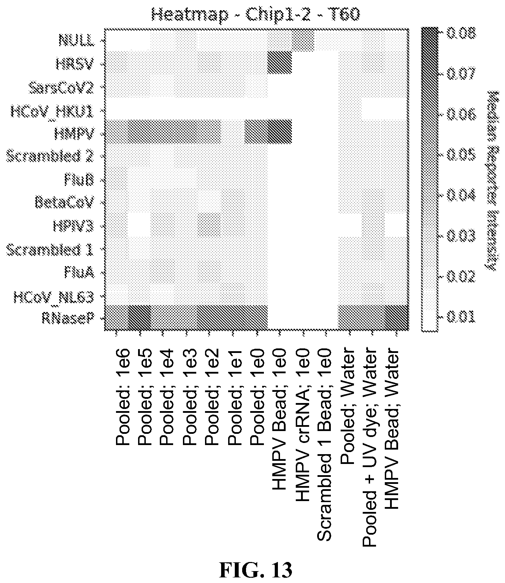

In certain embodiments, the effector protein, particularly a Type V loci effector protein, more particularly a Type V-C loci effector protein, even more particularly a C2c3p, may comprise, consist essentially of or consist of an amino acid sequence selected from the group consisting of amino acid sequences shown in the multiple sequence alignment in FIG. 13I of PCT/US2016/038238.

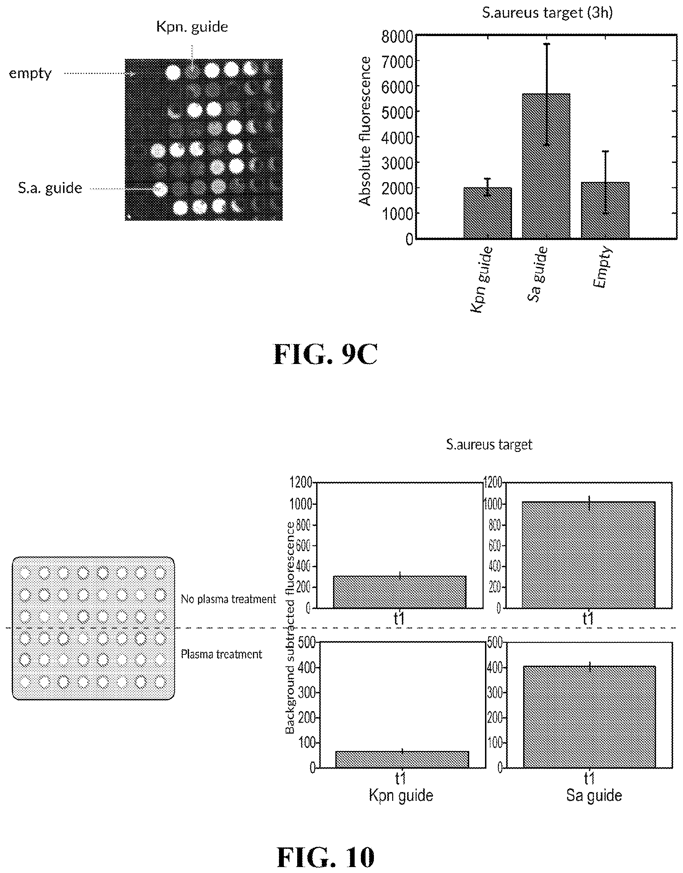

In certain embodiments, a Type V-C locus as intended herein may encode Cas1 and the C2c3p effector protein. See FIG. 14 of PCT/US2016/038238, depicting the genomic architecture of the Cas12c CRISPR-Cas loci. In certain embodiments, a Cas1 protein encoded by a Type V-C locus as intended herein may cluster with Type I-B system. See FIG. 10A and 10B and FIG. 10C-V of PCT/US2016/038238, illustrating a Cas1 tree including Cas1 encoded by representative Type V-C loci.

In certain embodiments, the effector protein, particularly a Type V loci effector protein, more particularly a Type V-C loci effector protein, even more particularly a C2c3p, such as a native C2c3p, may be about 1100 to about 1500 amino acids long, e.g., about 1100 to about 1200 amino acids long, or about 1200 to about 1300 amino acids long, or about 1300 to about 1400 amino acids long, or about 1400 to about 1500 amino acids long, e.g., about 1100, about 1200, about 1300, about 1400 or about 1500 amino acids long, or at least about 1100, at least about 1200, at least about 1300, at least about 1400 or at least about 1500 amino acids long.

In certain embodiments, the effector protein, particularly a Type V loci effector protein, more particularly a Type V-C loci effector protein, even more particularly a C2c3p, and preferably the C-terminal portion of said effector protein, comprises the three catalytic motifs of the RuvC-like nuclease (i.e., RuvCI, RuvCII and RuvCIII). In certain embodiments, said effector protein, and preferably the C-terminal portion of said effector protein, may further comprise a region corresponding to the bridge helix (also known as arginine-rich cluster) that in Cas9 protein is involved in crRNA-binding. In certain embodiments, said effector protein, and preferably the C-terminal portion of said effector protein, may further comprise a Zn finger region. Preferably, the Zn-binding cysteine residue(s) may be conserved in C2c3p. In certain embodiments, said effector protein, and preferably the C-terminal portion of said effector protein, may comprise the three catalytic motifs of the RuvC-like nuclease (i.e., RuvCI, RuvCII and RuvCIII), the region corresponding to the bridge helix, and the Zn finger region, preferably in the following order, from N to C terminus: RuvCI-bridge helix-RuvCII-Zinc finger-RuvCIII. See FIG. 13A and 13C of PCT/US2016/038238, for illustration of representative Type V-C effector proteins domain architecture.

In certain embodiments, Type V-C loci as intended herein may comprise CRISPR repeats between 20 and 30 bp long, more typically between 22 and 27 bp long, yet more typically 25 bp long, e.g., 20, 21, 22, 23, 24, 25, 26, 27, 28, 29, or 30 bp long.

Orthologous proteins may but need not be structurally related, or are only partially structurally related. In particular embodiments, the homologue or orthologue of a Type V protein such as Cas12c as referred to herein has a sequence homology or identity of at least 80%, more preferably at least 85%, even more preferably at least 90%, such as for instance at least 95% with a Cas12c. In further embodiments, the homologue or orthologue of a Type V Cas12c as referred to herein has a sequence identity of at least 80%, more preferably at least 85%, even more preferably at least 90%, such as for instance at least 95% with the wild type Cas12c.

In an embodiment, the Type V RNA-targeting Cas protein may be a Cas12c ortholog of an organism of a genus which includes but is not limited to *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter.*

In an embodiment, the Cas12c or an ortholog or homolog thereof, may comprise one or more mutations (and hence nucleic acid molecule(s) coding for same may have mutation(s). The mutations may be artificially introduced mutations and may include but are not limited to one or more mutations in a catalytic domain. Examples of catalytic domains with reference to a Cas enzyme may include but are not limited to RuvC I, RuvC II, RuvC III, HNH domains, and HEPN domains, as described herein. In an embodiment, the Cas12c or an ortholog or homolog thereof, may comprise one or more mutations. The mutations may be artificially introduced mutations and may include but are not limited to one or more mutations in a catalytic domain.

### Amplification

In certain example embodiments, target RNAs and/or DNAs may be amplified prior to activating the CRISPR effector protein. In some instances, amplification is performed in the sample prior to exposing or distributing to the guide molecules. Other embodiments permit amplification to be performed subsequent to distribution of the sample to an individual discrete volume, and, accordingly, may include nucleic acid amplification reagents in the individual discrete volume comprising the target molecule. Any suitable RNA or DNA amplification technique may be used. In certain example embodiments, the RNA or DNA amplification is an isothermal amplification. In certain example embodiments, the isothermal amplification may be nucleic-acid sequenced-based amplification (NASBA), recombinase polymerase amplification (RPA), loop-mediated isothermal amplification (LAMP), strand displacement amplification (SDA), helicase-dependent amplification (HDA), or nicking enzyme amplification reaction (NEAR). In certain example embodiments, non-isothermal amplification methods may be used which include, but are not limited to, PCR, multiple displacement amplification (MDA), rolling circle amplification (RCA), ligase chain reaction (LCR), or ramification amplification method (RAM). In some preferred embodiments, the RNA or DNA amplification is RPA or PCR.

In certain example embodiments, the RNA or DNA amplification is NASBA, which is initiated with reverse transcription of target RNA by a sequence-specific reverse primer to create a RNA/DNA duplex. RNase H is then used to degrade the RNA template, allowing a forward primer containing a promoter, such as the T7 promoter, to bind and initiate elongation of the complementary strand, generating a double-stranded DNA product. The RNA polymerase promoter-mediated transcription of the DNA template then creates copies of the target RNA sequence. Importantly, each of the new target RNAs can be detected by the guide RNAs thus further enhancing the sensitivity of the assay. Binding of the target RNAs by the guide RNAs then leads to activation of the CRISPR effector protein and the methods proceed as outlined above. The NASBA reaction has the additional advantage of being able to proceed under moderate isothermal conditions, for example at approximately 41°C, making it suitable for systems and devices deployed for early and direct detection in the field and far from clinical laboratories.

In certain other example embodiments, a recombinase polymerase amplification (RPA) reaction may be used to amplify the target nucleic acids. RPA reactions employ recombinases which are capable of pairing sequence-specific primers with homologous sequence in duplex DNA. If target DNA is present, DNA amplification is initiated and no other sample manipulation such as thermal cycling or chemical melting is required. The entire RPA amplification system is stable as a dried formulation and can be transported safely without refrigeration. RPA reactions may also be carried out at isothermal temperatures with an optimum reaction temperature of 37-42° C. The sequence specific primers are designed to amplify a sequence comprising the target nucleic acid sequence to be detected. In certain example embodiments, a RNA polymerase promoter, such as a T7 promoter, is added to one of the primers. This results in an amplified double-stranded DNA product comprising the target sequence and a RNA polymerase promoter. After, or during, the RPA reaction, a RNA polymerase is added that will produce RNA from the double-stranded DNA templates. The amplified target RNA can then in turn be detected by the CRISPR effector system. In this way target DNA can be detected using the embodiments disclosed herein. RPA reactions can also be used to amplify target RNA. The target RNA is first converted to cDNA using a reverse transcriptase, followed by second strand DNA synthesis, at which point the RPA reaction proceeds as outlined above.

### Transposase Based Amplification

Embodiments disclosed herein provide systems and methods for isothermal amplification of target nucleic acid sequences by contacting oligonucleotides containing the target nucleic acid sequence with a transposon complex. The oligonucleotides may be single stranded or double stranded RNA, DNA, or RNA/DNA hybrid oligonucleotides. The transposon complex comprises a transposase and a transposon sequence comprising one or more RNA polymerase promoters. The transposase facilitates insertion of the one or more RNA polymerase promoters into the oligonucleotide. A RNA polymerase promoter can then transcribe the target nucleic acid sequence from the inserted one or more RNA polymerase promoters. One advantage of this system is that there is no need to heat or melt double-stranded DNA templates, since RNA polymerase polymerases require a double-stranded template. Such isothermal amplification is fast and simple, obviating the need for complicated and expensive instrumentation for denaturation and cooling. In certain example embodiment the RNA polymerase promoter is a native of modified T7 RNA promoter.

The term "transposon", as used herein, refers to a nucleic acid segment, which is recognized by a transposase or an integrase enzyme and which is an essential component of a functional nucleic acid-protein complex (i.e. a transposome) capable of transposition. The term "transposase" as used herein refers to an enzyme, which is a component of a functional nucleic acid-protein complex capable of transposition and which is mediating transposition. The term "transposase" also refers to integrases from retrotransposons or of retroviral origin. Transposon complexes form between a transposase enzyme and a fragment of double stranded DNA that contains a specific binding sequence for the enzyme, termed "transposon end". The sequence of the transposon binding site can be modified with other bases, at certain positions, without affecting the ability for transposon complex to form a stable structure that can efficiently transpose into target DNA.

In embodiments provided herein, the transposon complex may comprise a transposase and a transposon sequence comprising one or more RNA polymerase promoters. The term "promoter" refers to a region of DNA involved in binding the RNA polymerase to initiate transcription. In specific embodiments, the RNA polymerase promoter may be a T7 RNA polymerase promoter. The T7 RNA promoter may be inserted into the double-stranded polynucleotide using the transposase. In some embodiments, insertion of the T7 RNA polymerase promoter into the oligonucleotide may be random.

The frequency of transposition is very low for most transposons, which use complex mechanisms to limit activity. Tn5 transposase, for example, utilizes a DNA binding sequence that is suboptimal and the C-terminus of the transposase interferes with DNA binding. Mechanisms involved in Tn5 transposition have been carefully characterized by Reznikoff and colleagues. Tn5 transposes by a cut-and-paste mechanism. The transposon has two pairs of 19 bp elements that are utilized by the transposase: outside elements (OE) and inside elements (IE). One transposase monomer binds to each of the two elements that are utilized. After a monomer is bound to each end of the transposon, the two monomers dimerize, forming a synapse. Vectors with donor backbones of at least 200 bp, but less than 1000 bp, are most functional for transposition in bacteria. Transposon cleavage occurs by trans catalysis and only when monomers bound to each DNA end are in a synaptic complex. Tn5 transposes with a relaxed target site selection and can therefore insert into target DNA with little to no target sequence specificity.

The natural downregulation of Tn5 transposition can be overcome by selection of a hyperactive transposase and by optimizing the transposase-binding elements [Yorket al. 1998]. A mosaic element (ME), made by modification of three bases of the wild type OE, led to a 50-fold increase in transposition events in bacteria as well as cell-free systems. The combined effect of the optimized ME and hyperactive mutant transposase is estimated to result in a 100-fold increase in transposition activity. Goryshin et al showed that preformed Tn5 transposition complexes could be functionally introduced into bacterial or yeast by electroporation [Goryshin et al. 2000]. Linearization of the DNA, to have inverted repeats precisely positioned at both ends of the transposon, allowed Goryshin and coworkers to bypass the cutting step of transposition thus enhancing transposition efficiency.

In some embodiments, the transposase may be used to tagment the oligonucleotide sequence comprising the target sequence. The term "tagmentation" refers to a step in the Assay for Transposase Accessible Chromatin using sequencing (ATAC-seq) as described. (See, Buenrostro, J. D., Giresi, P. G., Zaba, L. C., Chang, H. Y., Greenleaf, W. J., Transposition of native chromatin for fast and sensitive epigenomic profiling of open chromatin, DNA-binding proteins and nucleosome position. Nature methods 2013; 10 (12): 1213-1218). Specifically, a hyperactive Tn5 transposase loaded in vitro with adapters for high-throughput DNA sequencing, can simultaneously fragment and tag a genome with sequencing adapters. In one embodiment the adapters are compatible with the methods described herein.

In some embodiments, the transposase may be a Tn5 transposase. In some embodiments, the transposase may be a variant of a Tn5 transposase, or an engineered transposase. Transposases may be engineered using any method known in the art. The engineered transposase may be optimized to function at a temperature ranging from 30°C to 45°C, 35°C to 40°C or any temperature in between. The engineered transposase may be optimized to release from the oligonucleotide at a faster rate compared to a wild type transposase.

In some embodiments, the transposase may be a Tn5 transposase, a Mu transposase, or a Tn7 transposase. Transposition efficiency *in vitro* may vary depending on the transposon system used. Generally, Tn5 and Mu transposases effect higher levels of transposition efficiency. In some embodiments, insertion may be random. In some embodiments, insertion may occur in GC rich regions of the target sequence.

In some embodiments, the transposon sequence may comprise two 19 base pair Mosaic End (ME) Tn5 transposase recognition sequences. Tn5 transposases will generally transpose any DNA sequence contained between such short 19 base pair ME Tn5 transposase recognition sequences.

In some embodiments, use of a transposase allows for separation of a double-stranded polynucleotide in the absence of heat or melting. Embodiments can be as described in PCT/US2019/039195, entitled CRISPR/Cas and Transposase Based Amplification Compositions, Systems and Methods.

### Nickase Dependent Amplification

In an embodiment of the invention may comprise nickase-based amplification. The nicking enzyme may be a CRISPR protein. Accordingly, the introduction of nicks into dsDNA can be programmable and sequence-specific. In an embodiment of the invention, two guides can be designed to target opposite strands of a dsDNA target. According to the invention, the nickase can be Cpfl, C2c1, Cas9 or any ortholog or CRISPR protein that cleaves or is engineered to cleave a single strand of a DNA duplex. The nicked strands may then be extended by a polymerase. In an embodiment, the locations of the nicks are selected such that extension of the strands by a polymerase is towards the central portion of the target duplex DNA between the nick sites. In certain embodiments, primers are included in the reaction capable of hybridizing to the extended strands followed by further polymerase extension of the primers to regenerate two dsDNA pieces: a first dsDNA that includes the first strand Cpfl guide site or both the first and second strand Cpf1 guide sites, and a second dsDNA that includes the second strand Cpf1 guide site or both the first and second strand Cprf guide sites. These pieces continue to be nicked and extended in a cyclic reaction that exponentially amplifies the region of the target between nicking sites.

The amplification can be isothermal and selected for temperature. In one embodiment, the amplification proceeds rapidly at 37 degrees. In other embodiments, the temperature of the isothermal amplification may be chosen by selecting a polymerase (e.g. Bsu, Bst, Phi29, klenow fragment etc.).operable at a different temperature.

Thus, whereas nicking isothermal amplification techniques use nicking enyzmes with fixed sequence preference (e.g. in nicking enzyme amplification reaction or NEAR), which requires denaturing of the original dsDNA target to allow annealing and extension of primers that add the nicking substrate to the ends of the target, use of a CRISPR nickase wherein the nicking sites can be programed via guide RNAs means that no denaturing step is necessary, enabling the entire reaction to be truly isothermal. This also simplifies the reaction because these primers that add the nicking substrate are different than the primers that are used later in the reaction, meaning that NEAR requires two primer sets (i.e. 4 primers) while Cpfl nicking amplification only requires one primer set (i.e. two primers). This makes nicking Cpf1 amplification much simpler and easier to operate without complicated instrumentation to perform the denaturation and then cooling to the isothermal temperature.

In an aspect, the isothermal amplification reagents may be utilized with a thermostable CRISPR-Cas protein. The combination of thermostable protein and isothermal amplification reagents may be utilized to further improve reaction times for detection and diagnostics.

Accordingly, in certain example embodiments the systems disclosed herein may include amplification reagents. Different components or reagents useful for amplification of nucleic acids are described herein. For example, an amplification reagent as described herein may include a buffer, such as a Tris buffer. A Tris buffer may be used at any concentration appropriate for the desired application or use, for example including, but not limited to, a concentration of 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 25 mM, 50 mM, 75 mM, 1 M, or the like. One of skill in the art will be able to determine an appropriate concentration of a buffer such as Tris for use with the present invention.

A salt, such as magnesium chloride (MgCl2), potassium chloride (KCl), or sodium chloride (NaCl), may be included in an amplification reaction, such as PCR, in order to improve the amplification of nucleic acid fragments. Although the salt concentration will depend on the particular reaction and application, in some embodiments, nucleic acid fragments of a particular size may produce optimum results at particular salt concentrations. Larger products may require altered salt concentrations, typically lower salt, in order to produce desired results, while amplification of smaller products may produce better results at higher salt concentrations. One of skill in the art will understand that the presence and/or concentration of a salt, along with alteration of salt concentrations, may alter the stringency of a biological or chemical reaction, and therefore any salt may be used that provides the appropriate conditions for a reaction of the present invention and as described herein.

Other components of a biological or chemical reaction may include a cell lysis component in order to break open or lyse a cell for analysis of the materials therein. A cell lysis component may include, but is not limited to, a detergent, a salt as described above, such as NaCl, KCl, ammonium sulfate [(NH4)2SO4], or others. Detergents that may be appropriate for the invention may include Triton X-100, sodium dodecyl sulfate (SDS), CHAPS (3-[(3-cholamidopropyl)dimethylammonio]-1-propanesulfonate), ethyl trimethyl ammonium bromide, nonyl phenoxypolyethoxylethanol (NP-40). Concentrations of detergents may depend on the particular application, and may be specific to the reaction in some cases. Amplification reactions may include dNTPs and nucleic acid primers used at any concentration appropriate for the invention, such as including, but not limited to, a concentration of 100 nM, 150 nM, 200 nM, 250 nM, 300 nM, 350 nM, 400 nM, 450 nM, 500 nM, 550 nM, 600 nM, 650 nM, 700 nM, 750 nM, 800 nM, 850 nM, 900 nM, 950 nM, 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM, 100 mM, 150 mM, 200 mM, 250 mM, 300 mM, 350 mM, 400 mM, 450 mM, 500 mM, or the like. Likewise, a polymerase useful in accordance with the invention may be any specific or general polymerase known in the art and useful or the invention, including Taq polymerase, Q5 polymerase, or the like.

In some embodiments, amplification reagents as described herein may be appropriate for use in hot-start amplification. Hot start amplification may be beneficial in some embodiments to reduce or eliminate dimerization of adaptor molecules or oligos, or to otherwise prevent unwanted amplification products or artifacts and obtain optimum amplification of the desired product. Many components described herein for use in amplification may also be used in hot-start amplification. In some embodiments, reagents or components appropriate for use with hot-start amplification may be used in place of one or more of the composition components as appropriate. For example, a polymerase or other reagent may be used that exhibits a desired activity at a particular temperature or other reaction condition. In some embodiments, reagents may be used that are designed or optimized for use in hot-start amplification, for example, a polymerase may be activated after transposition or after reaching a particular temperature. Such polymerases may be antibody-based or aptamer-based. Polymerases as described herein are known in the art. Examples of such reagents may include, but are not limited to, hot-start polymerases, hot-start dNTPs, and photo-caged dNTPs. Such reagents are known and available in the art. One of skill in the art will be able to determine the optimum temperatures as appropriate for individual reagents.

Amplification of nucleic acids may be performed using specific thermal cycle machinery or equipment, and may be performed in single reactions or in bulk, such that any desired number of reactions may be performed simultaneously. In some embodiments, amplification may be performed using microfluidic or robotic devices, or may be performed using manual alteration in temperatures to achieve the desired amplification. In some embodiments, optimization may be performed to obtain the optimum reactions conditions for the particular application or materials. One of skill in the art will understand and be able to optimize reaction conditions to obtain sufficient amplification.

In certain embodiments, detection of DNA with the methods or systems of the invention requires transcription of the (amplified) DNA into RNA prior to detection.

It will be evident that detection methods of the invention can involve nucleic acid amplification and detection procedures in various combinations. The nucleic acid to be detected can be any naturally occurring or synthetic nucleic acid, including but not limited to DNA and RNA, which may be amplified by any suitable method to provide an intermediate product that can be detected. Detection of the intermediate product can be by any suitable method including but not limited to binding and activation of a CRISPR protein which produces a detectable signal moiety by direct or collateral activity.

### Helicase-Dependent Amplification

In helicase-dependent amplification, a helicase enzyme is used to unwind a double stranded nucleic acid to generate templates for primer hybridization and subsequent primer-extension. This process utilizes two oligonucleotide primers, each hybridizing to the 3'-end of either the sense strand containing the target sequence or the anti-sense strand containing the reverse-complementary target sequence. The HDA reaction is a general method for helicase-dependent nucleic acid amplification.

In combining this method with a CRISPR-SHERLOCK system, the target nucleic acid may be amplified by opening R-loops of the target nucleic acid using first and second CRISPR/Cas complexes. The first and second strand of the target nucleic acid may thus be unwound using a helicase, allowing primers and polymerase to bind and extend the DNA under isothermal conditions.

The term "helicase" refers here to any enzyme capable of unwinding a double stranded nucleic acid enzymatically. For example, helicases are enzymes that are found in all organisms and in all processes that involve nucleic acid such as replication, recombination, repair, transcription, translation and RNA splicing. (Kornberg and Baker, DNA Replication, W. H. Freeman and Company (2nd ed. (1992)), especially chapter 11). Any helicase that translocates along DNA or RNA in a 5' to 3' direction or in the opposite 3' to 5' direction may be used in present embodiments of the invention. This includes helicases obtained from prokaryotes, viruses, archaea, and eukaryotes or recombinant forms of naturally occurring enzymes as well as analogues or derivatives having the specified activity. Examples of naturally occurring DNA helicases, described by Kornberg and Baker in chapter 11 of their book, DNA Replication, W. H. Freeman and Company (2nd ed. (1992)), include *E*. *coli* helicase I, II, III, & IV, Rep, DnaB, PriA, PcrA, T4 Gp41helicase, T4 Dda helicase, T7 Gp4 helicases, SV40 Large T antigen, yeast RAD. Additional helicases that may be useful in HDA include RecQ helicase (Harmon and Kowalczykowski, J. Biol. Chem. 276:232-243 (2001)), thermostable UvrD helicases from *T. tengcongensis* (disclosed in this invention, Example XII) and *T. thermophilus* (Collins and McCarthy, Extremophiles. 7:35-41. (2003)), thermostable DnaB helicase from *T. aquaticus* (Kaplan and Steitz, J. Biol. Chem. 274:6889-6897 (1999)), and MCM helicase from archaeal and eukaryotic organisms ((Grainge et al., Nucleic Acids Res. 31:4888-4898 (2003)).

A traditional definition of a helicase is an enzyme that catalyzes the reaction of separating/unzipping/unwinding the helical structure of nucleic acid duplexes (DNA, RNA or hybrids) into single-stranded components, using nucleoside triphosphate (NTP) hydrolysis as the energy source (such as ATP). However, it should be noted that not all helicases fit this definition anymore. A more general definition is that they are motor proteins that move along the single-stranded or double stranded nucleic acids (usually in a certain direction, 3' to 5' or 5 to 3, or both), i.e. translocases, that can or cannot unwind the duplexed nucleic acid encountered. In addition, some helicases simply bind and "melt" the duplexed nucleic acid structure without an apparent translocase activity.

Helicases exist in all living organisms and function in all aspects of nucleic acid metabolism. Helicases are classified based on the amino acid sequences, directionality, oligomerization state and nucleic-acid type and structure preferences. The most common classification method was developed based on the presence of certain amino acid sequences, called motifs. According to this classification helicases are divided into 6 super families: SF1, SF2, SF3, SF4, SF5 and SF6. SF1 and SF2 helicases do not form a ring structure around the nucleic acid, whereas SF3 to SF6 do. Superfamily classification is not dependent on the classical taxonomy.

DNA helicases are responsible for catalyzing the unwinding of double-stranded DNA (dsDNA) molecules to their respective single-stranded nucleic acid (ssDNA) forms. Although structural and biochemical studies have shown how various helicases can translocate on ssDNA directionally, consuming one ATP per nucleotide, the mechanism of nucleic acid unwinding and how the unwinding activity is regulated remains unclear and controversial (T. M. Lohman, E. J. Tomko, C. G. Wu, "Non-hexameric DNA helicases and translocases: mechanisms and regulation," Nat Rev Mol Cell Biol 9:391-401 (2008)). Since helicases can potentially unwind all nucleic acids encountered, understanding how their unwinding activities are regulated can lead to harnessing helicase functions for biotechnology applications.

The term "HDA" refers to Helicase Dependent Amplification, which is an *in vitro* method for amplifying nucleic acids by using a helicase preparation for unwinding a double stranded nucleic acid to generate templates for primer hybridization and subsequent primer-extension. This process utilizes two oligonucleotide primers, each hybridizing to the 3'-end of either the sense strand containing the target sequence or the anti-sense strand containing the reverse-complementary target sequence. The HDA reaction is a general method for helicase-dependent nucleic acid amplification.

The invention comprises use of any suitable helicase known in the art. These include, but are not necessarily limited to, UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase.

In particularly preferred embodiments, the helicase comprises a super mutation. In particular embodiments, Although the E coli mutation has been described, the mutations were generated by sequence alignment (e.g. D409A/D410A for TteUvrd) and result in thermophilic enzymes working at lower temperatures like 37C, which is advantageous for amplification methods and systems described herein. In some embodiments, the super mutations is an aspartate to alanine mutation, with position based on sequence alignment. In some embodiments, the super mutant helicase is selected from WP_003870487.1 Thermoanaerobacter ethanolicus 403/404, WP_049660019.1 Bacillus sp. FJAT-27231 407/408, WP_034654680.1 Bacillus megaterium 415/416, WP_095390358.1 Bacillus simplex 407/408, and WP_055343022.1 Paeniclostridium sordellii 402/403.

Accordingly, in certain example embodiments the systems disclosed herein may include amplification reagents. Different components or reagents useful for amplification of nucleic acids are described herein. For example, an amplification reagent as described herein may include a buffer, such as a Tris buffer. A Tris buffer may be used at any concentration appropriate for the desired application or use, for example including, but not limited to, a concentration of 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 25 mM, 50 mM, 75 mM, 1 M, or the like. One of skill in the art will be able to determine an appropriate concentration of a buffer such as Tris for use with the present invention.

A salt, such as magnesium chloride (MgCl₂), potassium chloride (KCl), or sodium chloride (NaCl), may be included in an amplification reaction, such as PCR, in order to improve the amplification of nucleic acid fragments. Although the salt concentration will depend on the particular reaction and application, in some embodiments, nucleic acid fragments of a particular size may produce optimum results at particular salt concentrations. Larger products may require altered salt concentrations, typically lower salt, in order to produce desired results, while amplification of smaller products may produce better results at higher salt concentrations. One of skill in the art will understand that the presence and/or concentration of a salt, along with alteration of salt concentrations, may alter the stringency of a biological or chemical reaction, and therefore any salt may be used that provides the appropriate conditions for a reaction of the present invention and as described herein.

Other components of a biological or chemical reaction may include a cell lysis component in order to break open or lyse a cell for analysis of the materials therein. A cell lysis component may include, but is not limited to, a detergent, a salt as described above, such as NaCl, KCl, ammonium sulfate [(NH₄)₂SO₄], or others. Detergents that may be appropriate for the invention may include Triton X-100, sodium dodecyl sulfate (SDS), CHAPS (3-[(3-cholamidopropyl)dimethylammonio]-1-propanesulfonate), ethyl trimethyl ammonium bromide, nonyl phenoxypolyethoxylethanol (NP-40). Concentrations of detergents may depend on the particular application, and may be specific to the reaction in some cases. Amplification reactions may include dNTPs and nucleic acid primers used at any concentration appropriate for the invention, such as including, but not limited to, a concentration of 100 nM, 150 nM, 200 nM, 250 nM, 300 nM, 350 nM, 400 nM, 450 nM, 500 nM, 550 nM, 600 nM, 650 nM, 700 nM, 750 nM, 800 nM, 850 nM, 900 nM, 950 nM, 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM, 100 mM, 150 mM, 200 mM, 250 mM, 300 mM, 350 mM, 400 mM, 450 mM, 500 mM, or the like. Likewise, a polymerase useful in accordance with the invention may be any specific or general polymerase known in the art and useful or the invention, including Taq polymerase, Q5 polymerase, or the like.

In some embodiments, amplification reagents as described herein may be appropriate for use in hot-start amplification. Hot start amplification may be beneficial in some embodiments to reduce or eliminate dimerization of adaptor molecules or oligos, or to otherwise prevent unwanted amplification products or artifacts and obtain optimum amplification of the desired product. Many components described herein for use in amplification may also be used in hot-start amplification. In some embodiments, reagents or components appropriate for use with hot-start amplification may be used in place of one or more of the composition components as appropriate. For example, a polymerase or other reagent may be used that exhibits a desired activity at a particular temperature or other reaction condition. In some embodiments, reagents may be used that are designed or optimized for use in hot-start amplification, for example, a polymerase may be activated after transposition or after reaching a particular temperature. Such polymerases may be antibody-based or aptamer-based. Polymerases as described herein are known in the art. Examples of such reagents may include, but are not limited to, hot-start polymerases, hot-start dNTPs, and photo-caged dNTPs. Such reagents are known and available in the art. One of skill in the art will be able to determine the optimum temperatures as appropriate for individual reagents.

Amplification of nucleic acids may be performed using specific thermal cycle machinery or equipment, and may be performed in single reactions or in bulk, such that any desired number of reactions may be performed simultaneously. In some instances, amplification can be performed in droplets or prior to droplet formation. In some embodiments, amplification may be performed using microfluidic or robotic devices, or may be performed using manual alteration in temperatures to achieve the desired amplification. In some embodiments, optimization may be performed to obtain the optimum reactions conditions for the particular application or materials. One of skill in the art will understand and be able to optimize reaction conditions to obtain sufficient amplification.

In some instances, the nucleic acid amplification reagents comprise recombinase polymerase amplification (RPA) reagents, nucleic acid sequence-based amplification (NASBA) reagents, loop-mediated isothermal amplification (LAMP) reagents, strand displacement amplification (SDA) reagents, helicase-dependent amplification (HDA) reagents, nicking enzyme amplification reaction (NEAR) reagents, RT-PCR reagents, multiple displacement amplification (MDA) reagents, rolling circle amplification (RCA) reagents, ligase chain reaction (LCR) reagents, ramification amplification method (RAM) reagents, transposase based amplification reagents; or Programmable CRISPR Nicking Amplification (PCNA) reagents.

In certain embodiments, detection of DNA with the methods or systems of the invention requires transcription of the (amplified) DNA into RNA prior to detection.

It will be evident that detection methods of the invention can involve nucleic acid amplification and detection procedures in various combinations. The nucleic acid to be detected can be any naturally occurring or synthetic nucleic acid, including but not limited to DNA and RNA, which may be amplified by any suitable method to provide an intermediate product that can be detected. Detection of the intermediate product can be by any suitable method including but not limited to binding and activation of a Cas protein which produces a detectable signal moiety by direct or collateral activity.

### Amplification and/or enhancement of detectable positive signal

In certain example embodiments, further modification may be introduced that further amplify the detectable positive signal. For example, activated CRISPR effector protein collateral activation may be use to generate a secondary target or additional guide sequence, or both. In one example embodiment, the reaction solution would contain a secondary target that is spiked in at high concentration. The secondary target may be distinct from the primary target (i.e. the target for which the assay is designed to detect) and in certain instances may be common across all reaction volumes. A secondary guide sequence for the secondary target may be protected, e.g. by a secondary structural feature such as a hairpin with a RNA loop, and unable to bind the second target or the CRISPR effector protein. Cleavage of the protecting group by an activated CRISPR effector protein (i.e. after activation by formation of complex with the primary target(s) in solution) and formation of a complex with free CRISPR effector protein in solution and activation from the spiked in secondary target. In certain other example embodiments, a similar concept is used with a second guide sequence to a secondary target sequence. The secondary target sequence may be protected a structural feature or protecting group on the secondary target. Cleavage of a protecting group off the secondary target then allows additional CRISPR effector protein/second guide sequence/secondary target complex to form. In yet another example embodiment, activation of CRISPR effector protein by the primary target(s) may be used to cleave a protected or circularized primer, which is then released to perform an isothermal amplification reaction, such as those disclosed herein, on a template that encodes a secondary guide sequence, secondary target sequence, or both. Subsequent transcription of this amplified template would produce more secondary guide sequence and/or secondary target sequence, followed by additional CRISPR effector protein collateral activation.

### Reporter Molecules

Reporter molecules, as detailed herein, may be comprised on a detection bead, a reporter bead, or both. In one embodiment (described but not specifically claimed herein), the reporter molecule is comprised on a detection bead and no reporter bead is provided in the systems and methods described herein. In one embodiment, the reporter molecule is provided on a separate reporter bead. In one aspect, the reporter molecule may comprise a first molecule, such as for instance, FITC or 5(6)-carboxyfluorescein (FAM) attached to the 5' end of a nucleic acid molecule and a second molecule (such as for instance biotin) attached to the 3' end of the nucleic acid molecule (or vice versa). Such a construct may comprise a nucleic acid tailored for the detection system utilized. In certain embodiments, the reporter molecule is an RNA-based molecule. In one embodiment, the reporter molecule is a DNA-based molecule. The reporter molecule may comprise a masking construct, which may be comprised on a bead, for instance a detection bead or reporter bead, or in another individual discrete volume, for instance a microwell or droplet.

### Nucleic Acid-based masking construct

As used herein, a "masking construct" refers to a molecule that can be cleaved or otherwise deactivated by an activated CRISPR system effector protein described herein. The term "masking construct" may also be referred to in the alternative as a "detection construct." In certain example embodiments, the masking construct is a nucleic acid based masking construct. The masking construct can be a DNA-based or RNA-based masking construct. For ease of reference, the following example will reference an RNA based masking construct, but the same principles can apply to a DNA-based masking construct as well.

In one embodiment, the masking construct is an RNA-based masking construct. The RNA-based masking construct comprises a RNA element that is cleavable by a CRISPR effector protein. Cleavage of the RNA element releases agents or produces conformational changes that allow a detectable signal to be produced. Example constructs demonstrating how the RNA element may be used to prevent or mask generation of detectable signal are described below and embodiments of the invention comprise variants of the same. Prior to cleavage, or when the masking construct is in an 'active' state, the masking construct blocks the generation or detection of a positive detectable signal. It will be understood that in certain example embodiments a minimal background signal may be produced in the presence of an active RNA masking construct. A positive detectable signal may be any signal that can be detected using optical, fluorescent, chemiluminescent, electrochemical or other detection methods known in the art. The term "positive detectable signal" is used to differentiate from other detectable signals that may be detectable in the presence of the masking construct. For example, in certain embodiments a first signal may be detected when the masking agent is present (i.e. a negative detectable signal), which then converts to a second signal (e.g. the positive detectable signal) upon detection of the target molecules and cleavage or deactivation of the masking agent by the activated CRISPR effector protein.

Accordingly, in certain embodiments of the invention, the RNA-based masking construct suppresses generation of a detectable positive signal or the RNA-based masking construct suppresses generation of a detectable positive signal by masking the detectable positive signal, or generating a detectable negative signal instead, or the RNA-based masking construct comprises a silencing RNA that suppresses generation of a gene product encoded by a reporting construct, wherein the gene product generates the detectable positive signal when expressed.

In further embodiments, the RNA-based masking construct is a ribozyme that generates the negative detectable signal, and wherein the positive detectable signal is generated when the ribozyme is deactivated, or the ribozyme converts a substrate to a first color and wherein the substrate converts to a second color when the ribozyme is deactivated.

In other embodiments, the RNA-based masking agent is an RNA aptamer, or the aptamer sequesters an enzyme, wherein the enzyme generates a detectable signal upon release from the aptamer by acting upon a substrate, or the aptamer sequesters a pair of agents that when released from the aptamers combine to generate a detectable signal.

In another embodiment, the RNA-based masking construct comprises an RNA oligonucleotide to which a detectable ligand and a masking component are attached. In another embodiment, the detectable ligand is a fluorophore and the masking component is a quencher molecule, or the reagents to amplify target RNA molecules such as, but not limited to, NASBA or RPA reagents.

In certain example embodiments, the masking construct may suppress generation of a gene product. The gene product may be encoded by a reporter construct that is added to the sample. The masking construct may be an interfering RNA involved in a RNA interference pathway, such as a short hairpin RNA (shRNA) or small interfering RNA (siRNA). The masking construct may also comprise microRNA (miRNA). While present, the masking construct suppresses expression of the gene product. The gene product may be a fluorescent protein or other RNA transcript or proteins that would otherwise be detectable by a labeled probe, aptamer, or antibody but for the presence of the masking construct. Upon activation of the effector protein the masking construct is cleaved or otherwise silenced allowing for expression and detection of the gene product as the positive detectable signal.

In certain example embodiments, the masking construct may sequester one or more reagents needed to generate a detectable positive signal such that release of the one or more reagents from the masking construct results in generation of the detectable positive signal. The one or more reagents may combine to produce a colorimetric signal, a chemiluminescent signal, a fluorescent signal, or any other detectable signal and may comprise any reagents known to be suitable for such purposes. In certain example embodiments, the one or more reagents are sequestered by RNA aptamers that bind the one or more reagents. The one or more reagents are released when the effector protein is activated upon detection of a target molecule and the RNA aptamers are degraded.

In certain example embodiments, the masking construct may be immobilized on a solid substrate in an individual discrete volume (defined further below) and sequesters a single reagent. For example, the reagent may be a bead comprising a dye. When sequestered by the immobilized reagent, the individual beads are too diffuse to generate a detectable signal, but upon release from the masking construct are able to generate a detectable signal, for example by aggregation or simple increase in solution concentration. In certain example embodiments, the immobilized masking agent is a RNA-based aptamer that can be cleaved by the activated effector protein upon detection of a target molecule.

In certain other example embodiments, the masking construct binds to an immobilized reagent in solution thereby blocking the ability of the reagent to bind to a separate labeled binding partner that is free in solution. Thus, upon application of a washing step to a sample, the labeled binding partner can be washed out of the sample in the absence of a target molecule. However, if the effector protein is activated, the masking construct is cleaved to a degree sufficient to interfere with the ability of the masking construct to bind the reagent thereby allowing the labeled binding partner to bind to the immobilized reagent. Thus, the labeled binding partner remains after the wash step indicating the presence of the target molecule in the sample. In certain aspects, the masking construct that binds the immobilized reagent is an RNA aptamer. The immobilized reagent may be a protein and the labeled minding partner may be a labeled antibody. Alternatively, the immobilized reagent may be streptavidin and the labeled binding partner may be labeled biotin. The label on the binding partner used in the above embodiments may be any detectable label known in the art. In addition, other known binding partners may be used in accordance with the overall design described herein.

In certain example embodiments, the masking construct may comprise a ribozyme. Ribozymes are RNA molecules having catalytic properties. Ribozymes, both naturally and engineered, comprise or consist of RNA that may be targeted by the effector proteins disclosed herein. The ribozyme may be selected or engineered to catalyze a reaction that either generates a negative detectable signal or prevents generation of a positive control signal. Upon deactivation of the ribozyme by the activated effector protein the reaction generating a negative control signal, or preventing generation of a positive detectable signal, is removed thereby allowing a positive detectable signal to be generated. In one example embodiment, the ribozyme may catalyze a colorimetric reaction causing a solution to appear as a first color. When the ribozyme is deactivated the solution then turns to a second color, the second color being the detectable positive signal. An example of how ribozymes can be used to catalyze a colorimetric reaction are described in Zhao et al. "Signal amplification of glucosamine-6-phosphate based on ribozyme glmS," Biosens Bioelectron. 2014; 16:337-42, and provide an example of how such a system could be modified to work in the context of the embodiments disclosed herein. Alternatively, ribozymes, when present can generate cleavage products of, for example, RNA transcripts. Thus, detection of a positive detectable signal may comprise detection of non-cleaved RNA transcripts that are only generated in the absence of the ribozyme.

In certain example embodiments, the one or more reagents is a protein, such as an enzyme, capable of facilitating generation of a detectable signal, such as a colorimetric, chemiluminescent, or fluorescent signal, that is inhibited or sequestered such that the protein cannot generate the detectable signal by the binding of one or more RNA aptamers to the protein. Upon activation of the effector proteins disclosed herein, the RNA aptamers are cleaved or degraded to an extent that they no longer inhibit the protein's ability to generate the detectable signal. In certain example embodiments, the aptamer is a thrombin inhibitor aptamer. In certain example embodiments the thrombin inhibitor aptamer has a sequence of GGGAACAAAGCUGAAGUACUUACCC (SEQ ID NO: 14). When this aptamer is cleaved, thrombin will become active and will cleave a peptide colorimetric or fluorescent substrate. In certain example embodiments, the colorimetric substrate is para-nitroanilide (pNA) covalently linked to the peptide substrate for thrombin. Upon cleavage by thrombin, pNA is released and becomes yellow in color and easily visible to the eye. In certain example embodiments, the fluorescent substrate is 7-amino-4-methylcoumarin a blue fluorophore that can be detected using a fluorescence detector. Inhibitory aptamers may also be used for horseradish peroxidase (HRP), beta-galactosidase, or calf alkaline phosphatase (CAP) and within the general principals laid out above.

In certain embodiments, RNase activity is detected colorimetrically via cleavage of enzyme-inhibiting aptamers. One potential mode of converting RNase activity into a colorimetric signal is to couple the cleavage of an RNA aptamer with the re-activation of an enzyme that is capable of producing a colorimetric output. In the absence of RNA cleavage, the intact aptamer will bind to the enzyme target and inhibit its activity. The advantage of this readout system is that the enzyme provides an additional amplification step: once liberated from an aptamer via collateral activity (e.g. Cas13a collateral activity), the colorimetric enzyme will continue to produce colorimetric product, leading to a multiplication of signal.

In certain embodiments, an existing aptamer that inhibits an enzyme with a colorimetric readout is used. Several aptamer/enzyme pairs with colorimetric readouts exist, such as thrombin, protein C, neutrophil elastase, and subtilisin. These proteases have colorimetric substrates based upon pNA and are commercially available. In certain embodiments, a novel aptamer targeting a common colorimetric enzyme is used. Common and robust enzymes, such as beta-galactosidase, horseradish peroxidase, or calf intestinal alkaline phosphatase, could be targeted by engineered aptamers designed by selection strategies such as SELEX. Such strategies allow for quick selection of aptamers with nanomolar binding efficiencies and could be used for the development of additional enzyme/aptamer pairs for colorimetric readout.

In certain embodiments, RNase activity is detected colorimetrically via cleavage of RNA-tethered inhibitors. Many common colorimetric enzymes have competitive, reversible inhibitors: for example, beta-galactosidase can be inhibited by galactose. Many of these inhibitors are weak, but their effect can be increased by increases in local concentration. By linking local concentration of inhibitors to RNase activity, colorimetric enzyme and inhibitor pairs can be engineered into RNase sensors. The colorimetric RNase sensor based upon small-molecule inhibitors involves three components: the colorimetric enzyme, the inhibitor, and a bridging RNA that is covalently linked to both the inhibitor and enzyme, tethering the inhibitor to the enzyme. In the uncleaved configuration, the enzyme is inhibited by the increased local concentration of the small molecule; when the RNA is cleaved (e.g. by Cas13a collateral cleavage), the inhibitor will be released and the colorimetric enzyme will be activated.

In certain embodiments, RNase activity is detected colorimetrically via formation and/or activation of G-quadruplexes. G quadraplexes in DNA can complex with heme (iron (III)-protoporphyrin IX) to form a DNAzyme with peroxidase activity. When supplied with a peroxidase substrate (e.g. ABTS: (2,2'-Azinobis [3-ethylbenzothiazoline-6-sulfonic acid]-diammonium salt)), the G-quadraplex-heme complex in the presence of hydrogen peroxide causes oxidation of the substrate, which then forms a green color in solution. An example G-quadraplex forming DNA sequence is: GGGTAGGGCGGGTTGGGA (SEQ ID NO: 15). By hybridizing an RNA sequence to this DNA aptamer, formation of the G-quadraplex structure will be limited. Upon RNase collateral activation (e.g. C2c2-complex collateral activation), the RNA staple will be cleaved allowing the G quadraplex to form and heme to bind. This strategy is particularly appealing because color formation is enzymatic, meaning there is additional amplification beyond RNase activation.

In certain example embodiments, the masking construct may be immobilized on a solid substrate in an individual discrete volume (defined further below) and sequesters a single reagent. For example, the reagent may be a bead comprising a dye. When sequestered by the immobilized reagent, the individual beads are too diffuse to generate a detectable signal, but upon release from the masking construct are able to generate a detectable signal, for example by aggregation or simple increase in solution concentration. In certain example embodiments, the immobilized masking agent is a RNA-based aptamer that can be cleaved by the activated effector protein upon detection of a target molecule.

In one example embodiment, the masking construct comprises a detection agent that changes color depending on whether the detection agent is aggregated or dispersed in solution. For example, certain nanoparticles, such as colloidal gold, undergo a visible purple to red color shift as they move from aggregates to dispersed particles. Accordingly, in certain example embodiments, such detection agents may be held in aggregate by one or more bridge molecules. At least a portion of the bridge molecule comprises RNA. Upon activation of the effector proteins disclosed herein, the RNA portion of the bridge molecule is cleaved allowing the detection agent to disperse and resulting in the corresponding change in color. In certain example embodiments the, bridge molecule is a RNA molecule. In certain example embodiments, the detection agent is a colloidal metal. The colloidal metal material may include water-insoluble metal particles or metallic compounds dispersed in a liquid, a hydrosol, or a metal sol. The colloidal metal may be selected from the metals in groups IA, IB, IIB and IIIB of the periodic table, as well as the transition metals, especially those of group VIII. Preferred metals include gold, silver, aluminum, ruthenium, zinc, iron, nickel and calcium. Other suitable metals also include the following in all of their various oxidation states: lithium, sodium, magnesium, potassium, scandium, titanium, vanadium, chromium, manganese, cobalt, copper, gallium, strontium, niobium, molybdenum, palladium, indium, tin, tungsten, rhenium, platinum, and gadolinium. The metals are preferably provided in ionic form, derived from an appropriate metal compound, for example the A1³⁺, Ru³⁺, Zn²⁺, Fe³⁺, Ni²⁺ and Ca²⁺ ions.

When the RNA bridge is cut by the activated CRISPR effector, the beforementioned color shift is observed. In certain example embodiments the particles are colloidal metals. In certain other example embodiments, the colloidal metal is a colloidal gold. In certain example embodiments, the colloidal nanoparticles are 15 nm gold nanoparticles (AuNPs). Due to the unique surface properties of colloidal gold nanoparticles, maximal absorbance is observed at 520 nm when fully dispersed in solution and appear red in color to the naked eye. Upon aggregation of AuNPs, they exhibit a red-shift in maximal absorbance and appear darker in color, eventually precipitating from solution as a dark purple aggregate.

In certain example embodiments the nanoparticles are modified to include DNA linkers extending from the surface of the nanoparticle. Individual particles are linked together by single-stranded RNA (ssRNA) bridges that hybridize on each end of the RNA to at least a portion of the DNA linkers. Thus, the nanoparticles will form a web of linked particles and aggregate, appearing as a dark precipitate. Upon activation of the CRISPR effectors disclosed herein, the ssRNA bridge will be cleaved, releasing the AU NPS from the linked mesh and producing a visible red color. Example DNA linkers and RNA bridge sequences are listed below. Thiol linkers on the end of the DNA linkers may be used for surface conjugation to the AuNPS. Other forms of conjugation may be used. In certain example embodiments, two populations of AuNPs may be generated, one for each DNA linker. This will help facilitate proper binding of the ssRNA bridge with proper orientation. In certain example embodiments, a first DNA linker is conjugated by the 3' end while a second DNA linker is conjugated by the 5' end.

| | |
|---|---|
| C2c2 colorimetric DNA1 | TTATAACTATTCCTAAAAAAAAAAA/3ThioMC3-D/ (SEQ. I.D. No: 16) |
| C2c2 colorimetric DNA2 | /5ThioMC6-D/AAAAAAAAAACTCCCCTAATAACAAT (SEQ. I.D. No. 17) |
| C2c2 colorimetric bridge | GGGUAGGAAUAGUUAUAAUUUCCCUUUCCCAU UGUUAUUAGGGAG (SEQ. I.D. No. 18) |

In certain other example embodiments, the masking construct may comprise an RNA oligonucleotide to which are attached a detectable label and a masking agent of that detectable label. An example of such a detectable label/masking agent pair is a fluorophore and a quencher of the fluorophore. Quenching of the fluorophore can occur as a result of the formation of a non-fluorescent complex between the fluorophore and another fluorophore or non-fluorescent molecule. This mechanism is known as ground-state complex formation, static quenching, or contact quenching. Accordingly, the RNA oligonucleotide may be designed so that the fluorophore and quencher are in sufficient proximity for contact quenching to occur. Fluorophores and their cognate quenchers are known in the art and can be selected for this purpose by one having ordinary skill in the art. The particular fluorophore/quencher pair is not critical in the context of this invention, only that selection of the fluorophore/quencher pairs ensures masking of the fluorophore. Upon activation of the effector proteins disclosed herein, the RNA oligonucleotide is cleaved thereby severing the proximity between the fluorophore and quencher needed to maintain the contact quenching effect. Accordingly, detection of the fluorophore may be used to determine the presence of a target molecule in a sample.

In certain other example embodiments, the masking construct may comprise one or more RNA oligonucleotides to which are attached one or more metal nanoparticles, such as gold nanoparticles. In some embodiments, the masking construct comprises a plurality of metal nanoparticles crosslinked by a plurality of RNA oligonucleotides forming a closed loop. In one embodiment, the masking construct comprises three gold nanoparticles crosslinked by three RNA oligonucleotides forming a closed loop. In some embodiments, the cleavage of the RNA oligonucleotides by the CRISPR effector protein leads to a detectable signal produced by the metal nanoparticles.

In certain other example embodiments, the masking construct may comprise one or more RNA oligonucleotides to which are attached one or more quantum dots. In some embodiments, the cleavage of the RNA oligonucleotides by the CRISPR effector protein leads to a detectable signal produced by the quantum dots.

In one example embodiment, the masking construct may comprise a quantum dot. The quantum dot may have multiple linker molecules attached to the surface. At least a portion of the linker molecule comprises RNA. The linker molecule is attached to the quantum dot at one end and to one or more quenchers along the length or at terminal ends of the linker such that the quenchers are maintained in sufficient proximity for quenching of the quantum dot to occur. The linker may be branched. As above, the quantum dot/quencher pair is not critical, only that selection of the quantum dot/quencher pair ensures masking of the fluorophore. Quantum dots and their cognate quenchers are known in the art and can be selected for this purpose by one having ordinary skill in the art Upon activation of the effector proteins disclosed herein, the RNA portion of the linker molecule is cleaved thereby eliminating the proximity between the quantum dot and one or more quenchers needed to maintain the quenching effect. In certain example embodiments the quantum dot is streptavidin conjugated. RNA are attached via biotin linkers and recruit quenching molecules with the sequences /5Biosg/UCUCGUACGUUC/3IAbRQSp/ (SEQ ID NO: 19) or /5Biosg/UCUCGUACGUUCUCUCGUACGUUC/3IAbRQSp/ (SEQ ID NO:20), where /5Biosg/ is a biotin tag and /31AbRQSp/ is an Iowa black quencher. Upon cleavage, by the activated effectors disclosed herein the quantum dot will fluoresce visibly.

In a similar fashion, fluorescence energy transfer (FRET) may be used to generate a detectable positive signal. FRET is a non-radiative process by which a photon from an energetically excited fluorophore (i.e. "donor fluorophore") raises the energy state of an electron in another molecule (i.e. "the acceptor") to higher vibrational levels of the excited singlet state. The donor fluorophore returns to the ground state without emitting a fluoresce characteristic of that fluorophore. The acceptor can be another fluorophore or non-fluorescent molecule. If the acceptor is a fluorophore, the transferred energy is emitted as fluorescence characteristic of that fluorophore. If the acceptor is a non-fluorescent molecule the absorbed energy is loss as heat. Thus, in the context of the embodiments disclosed herein, the fluorophore/quencher pair is replaced with a donor fluorophore/acceptor pair attached to the oligonucleotide molecule. When intact, the masking construct generates a first signal (negative detectable signal) as detected by the fluorescence or heat emitted from the acceptor. Upon activation of the effector proteins disclosed herein the RNA oligonucleotide is cleaved and FRET is disrupted such that fluorescence of the donor fluorophore is now detected (positive detectable signal).

In certain example embodiments, the masking construct comprises the use of intercalating dyes which change their absorbance in response to cleavage of long RNAs to short nucleotides. Several such dyes exist. For example, pyronine-Y will complex with RNA and form a complex that has an absorbance at 572 nm. Cleavage of the RNA results in loss of absorbance and a color change. Methylene blue may be used in a similar fashion, with changes in absorbance at 688 nm upon RNA cleavage. Accordingly, in certain example embodiments the masking construct comprises an RNA and intercalating dye complex that changes absorbance upon the cleavage of RNA by the effector proteins disclosed herein.

In certain example embodiments, the masking construct may comprise an initiator for an HCR reaction. *See e.g.* Dirks and Pierce. PNAS 101, 15275-15728 (2004). HCR reactions utilize the potential energy in two hairpin species. When a single-stranded initiator having a portion of complementary to a corresponding region on one of the hairpins is released into the previously stable mixture, it opens a hairpin of one speces. This process, in turn, exposes a single-stranded region that opens a hairpin of the other species. This process, in turn, exposes a single stranded region identical to the original initiator. The resulting chain reaction may lead to the formation of a nicked double helix that grows until the hairpin supply is exhausted. Detection of the resulting products may be done on a gel or colorimetrically. Example colorimetric detection methods include, for example, those disclosed in Lu et al. "Ultra-sensitive colorimetric assay system based on the hybridization chain reaction-triggered enzyme cascade amplification ACS Appl Mater Interfaces, 2017, 9(1):167-175, Wang et al. "An enzyme-free colorimetric assay using hybridization chain reaction amplification and split aptamers" Analyst 2015, 150, 7657-7662, and Song et al. "Non covalent fluorescent labeling of hairpin DNA probe coupled with hybridization chain reaction for sensitive DNA detection." Applied Spectroscopy, 70(4): 686-694 (2016).

In certain example embodiments, the masking construct may comprise a HCR initiator sequence and a cleavable structural element, such as a loop or hairpin, that prevents the initiator from initiating the HCR reaction. Upon cleavage of the structure element by an activated CRISPR effector protein, the initiator is then released to trigger the HCR reaction, detection thereof indicating the presence of one or more targets in the sample. In certain example embodiments, the masking construct comprises a hairpin with a RNA loop. When an activated CRISRP effector protein cuts the RNA loop, the initiator can be released to trigger the HCR reaction.

### Optical barcodes, barcodes, and unique molecular identifier (UMI)

Systems as disclosed herein may comprise optical barcodes for one or more target molecules and an optical barcode associated with the detection CRISPR system. For example, barcodes for one or more target molecules and a sample of interest comprising the target molecule can be merged with CRISPR detection system-containing droplets containing optical barcodes. Optical barcodes can be associated with the guide molecule, the sample, the individual discrete volume, or a combination thereof, and may be comprised in the detection reagents.

The term "barcode" as used herein refers to a short sequence of nucleotides (for example, DNA or RNA) that is used as an identifier for an associated molecule, such as a target molecule and/or target nucleic acid, or as an identifier of the source of an associated molecule, such as a cell-of-origin. A barcode may also refer to any unique, non-naturally occurring, nucleic acid sequence that may be used to identify the originating source of a nucleic acid fragment. Although it is not necessary to understand the mechanism of an invention, it is believed that the barcode sequence provides a high-quality individual read of a barcode associated with a single cell, a viral vector, labeling ligand (e.g., an aptamer), protein, shRNA, sgRNA or cDNA such that multiple species can be sequenced together.

Barcoding may be performed based on any of the compositions or methods disclosed in patent publication WO 2014047561 A1, Compositions and methods for labeling of agents, incorporated herein in its entirety. In certain embodiments barcoding uses an error correcting scheme (T. K. Moon, Error Correction Coding: Mathematical Methods and Algorithms (Wiley, New York, ed. 1, 2005)). Not being bound by a theory, amplified sequences from single cells can be sequenced together and resolved based on the barcode associated with each cell.

Encoded particles may be delivered to the discrete volumes randomly resulting in a random combination of encoded particles in each well, or a unique combination of encoded particles may be specifically assigned to each discrete volume. The observable combination of encoded particles may then be used to identify each discrete volume. Optical assessments, such as phenotype, may be made and recorded for each discrete volume. Detectable signals can be used for encoding particles, including absorbance, luminescence, polarization, lifetime, or other linear or nonlinear optical property. In some instances, the barcode may be an optically detectable barcode that can be visualized with light or fluorescence microscopy. In certain example embodiments, the optical barcode comprises a sub-set of fluorophores or quantum dots of distinguishable colors from a set of defined colors. In some instances, optically encoded particles may be delivered to the discrete volumes randomly resulting in a random combination of optically encoded particles in each well, or a unique combination of optically encoded particles may be specifically assigned to each discrete volume.

In an exemplary embodiment, 3 fluorescent dyes, e.g. Alexa Fluor 555, 594, 647, at different levels, 105 barcodes can be generated. The addition of a fourth dye can be used and can be extended to scale to hundreds of unique barcodes; similarly, five colors can increase the number of unique barcodes that may be achieved by varying the ratios of the colors. By labeling with distinct ratios of dyes, dye ratios can be chosen so that after normalization the dyes are evenly spaced in logarithmic coordinates.

In one embodiment, the assigned or random subset(s) of fluorophores received in each discrete volume dictates the observable pattern of discrete encoded particles in each discrete volume thereby allowing each discrete volume to be independently identified. Each discrete volume is imaged with the appropriate imaging technique to detect the encoded particles. For example, if the encoded particles are optically encoded fluorescently labeled particles, each discrete volume is imaged using a fluorescent microscope. In another example, if the optically encoded particles are colorimetrically labeled each discrete volume is imaged using a microscope having one or more filters that match the wave length or absorption spectrum or emission spectrum inherent to each color label. Other detection methods are contemplated that match the optical system used, e.g., those known in the art for detecting quantum dots, dyes, etc. The pattern of observed discrete optically encoded particles for each discrete volume may be recorded for later use. In a preferred embodiment, the encoded particles are delivered to individual discrete volumes, for example arrayed microwells, and decoded for identification of the discrete volumes for later use. In particular embodiments, the encoded particles are dried, or lyophilized, in the wells of the microarray and decoded such that upon application of a sample to the microarray, identification of the array wells and contents are achievable by detection methods describd herein.

Optical barcodes can optionally include a unique oligonucleotide sequence, method for generating can be as described in, for example, International Patent Application Publication No. WO/2014/047561 at [050] - [0115]. In one example embodiment, a primer particle identifier is incorporated in the target molecules. Next generation sequencing (NGS) techniques known in the art can be used for sequencing, with clustering by sequence similarity of the one or more target sequences. Alignment by sequence variation will allow for identification of optically encoded particles delivered to a discrete volume based on the particle identifiers incorporated in the aligned sequence information. In one embodiment, the particle identifier of each primer incorporated in the aligned sequence information indicates the pattern of optically encoded particles that is observable in the corresponding discrete volume from which the amplicons are generated. In this way the nucleic acid sequence variation can be correlated back to the originating discrete volume and further matched to the optical assessments, such as phenotype, made of the nucleic acid containing specimens in that discrete volume.

In preferred embodiments, sequencing is performed using unique molecular identifiers (UMI). The term "unique molecular identifiers" (UMI) as used herein refers to a sequencing linker or a subtype of nucleic acid barcode used in a method that uses molecular tags to detect and quantify unique amplified products. A UMI is used to distinguish effects through a single clone from multiple clones. The term "clone" as used herein may refer to a single mRNA or target nucleic acid to be sequenced. The UMI may also be used to determine the number of transcripts that gave rise to an amplified product, or in the case of target barcodes as described herein, the number of binding events. In embodiments, the amplification is by PCR or by isothermal amplification, as described elsewhere herein.

In certain embodiments, an UMI with a random sequence of between 4 and 20 base pairs is added to a template, which is amplified and sequenced. In preferred embodiments, the UMI is added to the 5' end of the template. Sequencing allows for high resolution reads, enabling accurate detection of true variants. As used herein, a "true variant" will be present in every amplified product originating from the original clone as identified by aligning all products with a UMI. Each clone amplified will have a different random UMI that will indicate that the amplified product originated from that clone. Background caused by the fidelity of the amplification process can be eliminated because true variants will be present in all amplified products and background representing random error will only be present in single amplification products (See e.g., Islam S. et al., 2014. Nature Methods No:11, 163-166). Not being bound by a theory, the UMI's are designed such that assignment to the original can take place despite up to 4-7 errors during amplification or sequencing. Not being bound by a theory, an UMI may be used to discriminate between true barcode sequences.

Unique molecular identifiers can be used, for example, to normalize samples for variable amplification efficiency. For example, in various embodiments, featuring a solid or semisolid support (for example a hydrogel bead), to which nucleic acid barcodes (for example a plurality of barcodes sharing the same sequence) are attached, each of the barcodes may be further coupled to a unique molecular identifier, such that every barcode on the particular solid or semisolid support receives a distinct unique molecule identifier. A unique molecular identifier can then be, for example, transferred to a target molecule with the associated barcode, such that the target molecule receives not only a nucleic acid barcode, but also an identifier unique among the identifiers originating from that solid or semisolid support.

A nucleic acid barcode or UMI can have a length of at least, for example, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, or 100 nucleotides, and can be in single- or double-stranded form. Target molecule and/or target nucleic acids can be labeled with multiple nucleic acid barcodes in combinatorial fashion, such as a nucleic acid barcode concatemer. Typically, a nucleic acid barcode is used to identify a target molecule and/or target nucleic acid as being from a particular discrete volume, having a particular physical property (for example, affinity, length, sequence, etc.), or having been subject to certain treatment conditions. Target molecule and/or target nucleic acid can be associated with multiple nucleic acid barcodes to provide information about all of these features (and more). Each member of a given population of UMIs, on the other hand, is typically associated with (for example, covalently bound to or a component of the same molecule as) individual members of a particular set of identical, specific (for example, discreet volume-, physical property-, or treatment condition-specific) nucleic acid barcodes. Thus, for example, each member of a set of origin-specific nucleic acid barcodes, or other nucleic acid identifier or connector oligonucleotide, having identical or matched barcode sequences, may be associated with (for example, covalently bound to or a component of the same molecule as) a distinct or different UMI.

As disclosed herein, unique nucleic acid identifiers are used to label the target molecules and/or target nucleic acids, for example origin-specific barcodes and the like. The nucleic acid identifiers, nucleic acid barcodes, can include a short sequence of nucleotides that can be used as an identifier for an associated molecule, location, or condition. In certain embodiments, the nucleic acid identifier further includes one or more unique molecular identifiers and/or barcode receiving adapters. A nucleic acid identifier can have a length of about, for example, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, or 100 base pairs (bp) or nucleotides (nt). In certain embodiments, a nucleic acid identifier can be constructed in combinatorial fashion by combining randomly selected indices (for example, about 1, 2, 3, 4, 5, 6, 7, 8, 9, or 10 indexes). Each such index is a short sequence of nucleotides (for example, DNA, RNA, or a combination thereof) having a distinct sequence. An index can have a length of about, for example, 4, 5, 6, 7, 8, 9, 10, 11, 12, 13, 14, 15, 16, 17, 18, 19, 20, 21, 22, 23, 24, or 25 bp or nt. Nucleic acid identifiers can be generated, for example, by split-pool synthesis methods, such as those described, for example, in International Patent Publication Nos. WO 2014/047556 and WO 2014/143158.

One or more nucleic acid identifiers (for example a nucleic acid barcode) can be attached, or "tagged," to a target molecule. This attachment can be direct (for example, covalent or noncovalent binding of the nucleic acid identifier to the target molecule) or indirect (for example, via an additional molecule). Such indirect attachments may, for example, include a barcode bound to a specific-binding agent that recognizes a target molecule. In certain embodiments, a barcode is attached to protein G and the target molecule is an antibody or antibody fragment. Attachment of a barcode to target molecules (for example, proteins and other biomolecules) can be performed using standard methods well known in the art. For example, barcodes can be linked via cysteine residues (for example, C-terminal cysteine residues). In other examples, barcodes can be chemically introduced into polypeptides (for example, antibodies) via a variety of functional groups on the polypeptide using appropriate group-specific reagents (see for example www.drmr.com/abcon). In certain embodiments, barcode tagging can occur via a barcode receiving adapter associate with (for example, attached to) a target molecule, as described herein.

Target molecules can be optionally labeled with multiple barcodes in combinatorial fashion (for example, using multiple barcodes bound to one or more specific binding agents that specifically recognizing the target molecule), thus greatly expanding the number of unique identifiers possible within a particular barcode pool. In certain embodiments, barcodes are added to a growing barcode concatemer attached to a target molecule, for example, one at a time. In other embodiments, multiple barcodes are assembled prior to attachment to a target molecule. Compositions and methods for concatemerization of multiple barcodes are described, for example, in International Patent Publication No. WO 2014/047561.

In some embodiments, a nucleic acid identifier (for example, a nucleic acid barcode) may be attached to sequences that allow for amplification and sequencing (for example, SBS3 and P5 elements for Illumina sequencing). In certain embodiments, a nucleic acid barcode can further include a hybridization site for a primer (for example, a single-stranded DNA primer) attached to the end of the barcode. For example, an origin-specific barcode may be a nucleic acid including a barcode and a hybridization site for a specific primer. In particular embodiments, a set of origin-specific barcodes includes a unique primer specific barcode made, for example, using a randomized oligo type NNNNNNNNNNNN (SEQ ID NO:21).

A nucleic acid identifier can further include a unique molecular identifier and/or additional barcodes specific to, for example, a common support to which one or more of the nucleic acid identifiers are attached. Thus, a pool of target molecules can be added, for example, to a discrete volume containing multiple solid or semisolid supports (for example, beads) representing distinct treatment conditions (and/or, for example, one or more additional solid or semisolid support can be added to the discreet volume sequentially after introduction of the target molecule pool), such that the precise combination of conditions to which a given target molecule was exposed can be subsequently determined by sequencing the unique molecular identifiers associated with it. The unique molecular identifiers can be associated with the guide molecule, the sample, the individual discrete volume, or a combination thereof.

Labeled target molecules and/or target nucleic acids associated origin-specific nucleic acid barcodes (optionally in combination with other nucleic acid barcodes as described herein) can be amplified by methods known in the art, such as polymerase chain reaction (PCR). For example, the nucleic acid barcode can contain universal primer recognition sequences that can be bound by a PCR primer for PCR amplification and subsequent high-throughput sequencing. In certain embodiments, the nucleic acid barcode includes or is linked to sequencing adapters (for example, universal primer recognition sequences) such that the barcode and sequencing adapter elements are both coupled to the target molecule. In particular examples, the sequence of the origin specific barcode is amplified, for example using PCR. In some embodiments, an origin-specific barcode further comprises a sequencing adaptor. In some embodiments, an origin-specific barcode further comprises universal priming sites. A nucleic acid barcode (or a concatemer thereof), a target nucleic acid molecule (for example, a DNA or RNA molecule), a nucleic acid encoding a target peptide or polypeptide, and/or a nucleic acid encoding a specific binding agent may be optionally sequenced by any method known in the art, for example, methods of high-throughput sequencing, also known as next generation sequencing or deep sequencing. A nucleic acid target molecule labeled with a barcode (for example, an origin-specific barcode) can be sequenced with the barcode to produce a single read and/or contig containing the sequence, or portions thereof, of both the target molecule and the barcode. Exemplary next generation sequencing technologies include, for example, Illumina sequencing, Ion Torrent sequencing, 454 sequencing, SOLiD sequencing, and nanopore sequencing amongst others. In some embodiments, the sequence of labeled target molecules is determined by non-sequencing based methods. For example, variable length probes or primers can be used to distinguish barcodes (for example, origin-specific barcodes) labeling distinct target molecules by, for example, the length of the barcodes, the length of target nucleic acids, or the length of nucleic acids encoding target polypeptides. In other instances, barcodes can include sequences identifying, for example, the type of molecule for a particular target molecule (for example, polypeptide, nucleic acid, small molecule, or lipid). For example, in a pool of labeled target molecules containing multiple types of target molecules, polypeptide target molecules can receive one identifying sequence, while target nucleic acid molecules can receive a different identifying sequence. Such identifying sequences can be used to selectively amplify barcodes labeling particular types of target molecules, for example, by using PCR primers specific to identifying sequences specific to particular types of target molecules. For example, barcodes labeling polypeptide target molecules can be selectively amplified from a pool, thereby retrieving only the barcodes from the polypeptide subset of the target molecule pool.

A nucleic acid barcode can be sequenced, for example, after cleavage, to determine the presence, quantity, or other feature of the target molecule. In certain embodiments, a nucleic acid barcode can be further attached to a further nucleic acid barcode. For example, a nucleic acid barcode can be cleaved from a specific-binding agent after the specific-binding agent binds to a target molecule or a tag (for example, an encoded polypeptide identifier element cleaved from a target molecule), and then the nucleic acid barcode can be ligated to an origin-specific barcode. The resultant nucleic acid barcode concatemer can be pooled with other such concatemers and sequenced. The sequencing reads can be used to identify which target molecules were originally present in which discrete volumes.

### Barcodes reversibly coupled to solid substrate

In some embodiments, the origin-specific barcodes are reversibly coupled to a solid or semisolid substrate. In some embodiments, the origin-specific barcodes further comprise a nucleic acid capture sequence that specifically binds to the target nucleic acids and/or a specific binding agent that specifically binds to the target molecules. In specific embodiments, the origin-specific barcodes include two or more populations of origin-specific barcodes, wherein a first population comprises the nucleic acid capture sequence and a second population comprises the specific binding agent that specifically binds to the target molecules. In some examples, the first population of origin-specific barcodes further comprises a target nucleic acid barcode, wherein the target nucleic acid barcode identifies the population as one that labels nucleic acids. In some examples, the second population of origin-specific barcodes further comprises a target molecule barcode, wherein the target molecule barcode identifies the population as one that labels target molecules.

### Barcode with cleavage sites

A nucleic acid barcode may be cleavable from a specific binding agent, for example, after the specific binding agent has bound to a target molecule. In some embodiments, the origin-specific barcode further comprises one or more cleavage sites. In some examples, at least one cleavage site is oriented such that cleavage at that site releases the origin-specific barcode from a substrate, such as a bead, for example a hydrogel bead, to which it is coupled. In some examples, at least one cleavage site is oriented such that the cleavage at the site releases the origin-specific barcode from the target molecule specific binding agent. In some examples, a cleavage site is an enzymatic cleavage site, such an endonuclease site present in a specific nucleic acid sequence. In other embodiments, a cleavage site is a peptide cleavage site, such that a particular enzyme can cleave the amino acid sequence. In still other embodiments, a cleavage site is a site of chemical cleavage.

### Barcode Adapters

In some embodiments, the target molecule is attached to an origin-specific barcode receiving adapter, such as a nucleic acid. In some examples, the origin-specific barcode receiving adapter comprises an overhang and the origin-specific barcode comprises a sequence capable of hybridizing to the overhang. A barcode receiving adapter is a molecule configured to accept or receive a nucleic acid barcode, such as an origin-specific nucleic acid barcode. For example, a barcode receiving adapter can include a single-stranded nucleic acid sequence (for example, an overhang) capable of hybridizing to a given barcode (for example, an origin-specific barcode), for example, via a sequence complementary to a portion or the entirety of the nucleic acid barcode. In certain embodiments, this portion of the barcode is a standard sequence held constant between individual barcodes. The hybridization couples the barcode receiving adapter to the barcode. In some embodiments, the barcode receiving adapter may be associated with (for example, attached to) a target molecule. As such, the barcode receiving adapter may serve as the means through which an origin-specific barcode is attached to a target molecule. A barcode receiving adapter can be attached to a target molecule according to methods known in the art. For example, a barcode receiving adapter can be attached to a polypeptide target molecule at a cysteine residue (for example, a C-terminal cysteine residue). A barcode receiving adapter can be used to identify a particular condition related to one or more target molecules, such as a cell of origin or a discreet volume of origin. For example, a target molecule can be a cell surface protein expressed by a cell, which receives a cell-specific barcode receiving adapter. The barcode receiving adapter can be conjugated to one or more barcodes as the cell is exposed to one or more conditions, such that the original cell of origin for the target molecule, as well as each condition to which the cell was exposed, can be subsequently determined by identifying the sequence of the barcode receiving adapter/ barcode concatemer.

### Barcode with Capture Moiety

In some embodiments, an origin-specific barcode further includes a capture moiety, covalently or non-covalently linked. Thus, in some embodiments the origin-specific barcode, and anything bound or attached thereto, that include a capture moiety are captured with a specific binding agent that specifically binds the capture moiety. In some embodiments, the capture moiety is adsorbed or otherwise captured on a surface. In specific embodiments, a targeting probe is labeled with biotin, for instance by incorporation of biotin-16-UTP during *in vitro* transcription, allowing later capture by streptavidin. Other means for labeling, capturing, and detecting an origin-specific barcode include: incorporation of aminoallyl-labeled nucleotides, incorporation of sulfhydryl-labeled nucleotides, incorporation of allyl- or azide-containing nucleotides, and many other methods described in Bioconjugate Techniques (2nd Ed), Greg T. Hermanson, Elsevier (2008). In some embodiments, the targeting probes are covalently coupled to a solid support or other capture device prior to contacting the sample, using methods such as incorporation of aminoallyl-labeled nucleotides followed by 1-Ethyl-3-(3-dimethylaminopropyl)carbodiimide (EDC) coupling to a carboxy-activated solid support, or other methods described in Bioconjugate Techniques. In some embodiments, the specific binding agent has been immobilized for example on a solid support, thereby isolating the origin-specific barcode.

### Other Barcoding Embodiments

DNA barcoding is also a taxonomic method that uses a short genetic marker in an organism's DNA to identify it as belonging to a particular species. It differs from molecular phylogeny in that the main goal is not to determine classification but to identify an unknown sample in terms of a known classification. Kress et al., "Use of DNA barcodes to identify flowering plants" Proc. Natl. Acad. Sci. U.S.A. 102(23):8369-8374 (2005). Barcodes are sometimes used in an effort to identify unknown species or assess whether species should be combined or separated. Koch H., "Combining morphology and DNA barcoding resolves the taxonomy of Western Malagasy Liotrigona Moure, 1961" African Invertebrates 51(2): 413-421 (2010); and Seberg et al., "How many loci does it take to DNA barcode a crocus?" PLoS One 4(2):e4598 (2009). Barcoding has been used, for example, for identifying plant leaves even when flowers or fruit are not available, identifying the diet of an animal based on stomach contents or feces, and/or identifying products in commerce (for example, herbal supplements or wood). Soininen et al., "Analysing diet of small herbivores: the efficiency of DNA barcoding coupled with high-throughput pyrosequencing for deciphering the composition of complex plant mixtures" Frontiers in Zoology 6:16 (2009).

It has been suggested that a desirable locus for DNA barcoding should be standardized so that large databases of sequences for that locus can be developed. Most of the taxa of interest have loci that are sequencable without species-specific PCR primers. CBOL Plant Working Group, "A DNA barcode for land plants" PNAS 106(31):12794-12797 (2009). Further, these putative barcode loci are believed short enough to be easily sequenced with current technology. Kress et al., "DNA barcodes: Genes, genomics, and bioinformatics" PNAS 105(8):2761-2762 (2008). Consequently, these loci would provide a large variation between species in combination with a relatively small amount of variation within a species. Lahaye et al., "DNA barcoding the floras of biodiversity hotspots" Proc Natl Acad Sci USA 105(8):2923-2928 (2008).

DNA barcoding is based on a relatively simple concept. For example, most eukaryote cells contain mitochondria, and mitochondrial DNA (mtDNA) has a relatively fast mutation rate, which results in significant variation in mtDNA sequences between species and, in principle, a comparatively small variance within species. A 648-bp region of the mitochondrial cytochrome c oxidase subunit 1 (CO1) gene was proposed as a potential 'barcode'. As of 2009, databases of CO1 sequences included at least 620,000 specimens from over 58,000 species of animals, larger than databases available for any other gene. Ausubel, J., "A botanical macroscope" Proceedings of the National Academy of Sciences 106(31):12569 (2009).

Software for DNA barcoding requires integration of a field information management system (FIMS), laboratory information management system (LIMS), sequence analysis tools, workflow tracking to connect field data and laboratory data, database submission tools and pipeline automation for scaling up to eco-system scale projects. Geneious Pro can be used for the sequence analysis components, and the two plugins made freely available through the Moorea Biocode Project, the Biocode LIMS and Genbank Submission plugins handle integration with the FIMS, the LIMS, workflow tracking and database submission.

Additionally, other barcoding designs and tools have been described (see e.g., Birrell et al., (2001) Proc. Natl Acad. Sci. USA 98, 12608-12613; Giaever, et al., (2002) Nature 418, 387-391; Winzeler et al., (1999) Science 285, 901-906; and Xu et al., (2009) Proc Natl Acad Sci U S A. Feb 17;106(7):2289-94).

Target molecules, as described herein can include any target nucleic acid sequence, that, in embodiments, the one or more guide RNAs are designed to bind to one or more target molecules that are diagnostic for a disease state. In further embodiments, the disease state is an infection, an organ disease, a blood disease, an immune system disease, a cancer, a brain and nervous system disease, an endocrine disease, a pregnancy or childbirth-related disease, an inherited disease, or an environmentally-acquired disease. In still further embodiments, the disease state is an infection, including a microbial infection.

In further embodiments, the infection is caused by a virus, a bacterium, or a fungus, or the infection is a viral infection. In specific embodiments, the viral infection is caused by a double-stranded RNA virus, a positive sense RNA virus, a negative sense RNA virus, a retrovirus, or a combination thereof. In certain embodiments, the application can achieve multiplexed strain discrimination. In some embodiments, pathogen subtyping can be detected, in one embodiment, influenza subtyping, Staph or strep subtyping, and bacterial superinfection subtype detection can be performed. In one preferred embodiment, multiplexed detection and identification of all H and N subtypes of Influenza A virus can be performed. In one aspect, pooled (or arrayed) crRNAs are used to capture variation within subtypes. In certain instances, the infection is HIV. In an embodiment, drug resistant mutations in HIV Reverse Transcriptase can be performed via SNP detection. In some embodiments, the mutation can be K65R, K103N, V106M, Y181C, M184V, G190A. Similarly, SNP detection in other infections can be performed, such as in tuberculosis. In some embodiments, the mutation may be *katG*, 315ACC: Isoniazid resistance, *rpoB*, 531TTG: Rifampin resistance, *gyrA*, 94GGC: Fluoroquinolone resistance, *rrs*, 1401G: Aminoglycoside resistance. Additionally, HIV/TB co-infections can be detected. Massive multiplexing to detect pan-viral, viral zone pan-viral, pan-bacterial or pan-pathogen detection can be achieved. *See,* Ackerman, C.M., Myhrvold, C., Thakku, S.G. et al. Massively multiplexed nucleic acid detection with Cas13. Nature 582, 277-282 (2020). DOI:10.1038/s41586-020-2279-8.

As described herein, a sample containing target molecules for use with the invention may be a biological or environmental sample, such as a food sample (fresh fruits or vegetables, meats), a beverage sample, a paper surface, a fabric surface, a metal surface, a wood surface, a plastic surface, a soil sample, a freshwater sample, a wastewater sample, a saline water sample, exposure to atmospheric air or other gas sample, or a combination thereof. For example, household/commercial/industrial surfaces made of any materials including, but not limited to, metal, wood, plastic, rubber, or the like, may be swabbed and tested for contaminants. Soil samples may be tested for the presence of pathogenic bacteria or parasites, or other microbes, both for environmental purposes and/or for human, animal, or plant disease testing. Water samples such as freshwater samples, wastewater samples, or saline water samples can be evaluated for cleanliness and safety, and/or potability, to detect the presence of, for example, *Cryptosporidium parvum*, *Giardia lamblia*, or other microbial contamination. In further embodiments, a biological sample may be obtained from a source including, but not limited to, a tissue sample, saliva, blood, plasma, sera, stool, urine, sputum, mucous, lymph, synovial fluid, cerebrospinal fluid, ascites, pleural effusion, seroma, pus, or swab of skin or a mucosal membrane surface. In some particular embodiments, an environmental sample or biological samples may be crude samples and/or the one or more target molecules may not be purified or amplified from the sample prior to application of the method. Identification of microbes may be useful and/or needed for any number of applications, and thus any type of sample from any source deemed appropriate by one of skill in the art may be used in accordance with the invention.

The biological sample may be further processed prior to further evaluation, including, for example by enriching or isolating cells of interest. In one aspect, cells in a biological sample have been first enriched or sorted prior to further processing and/or library preparation. In embodiments, the cells are sorted by fluorescence-activated cell sorting (FACS) or magnetic-activated cell sorting (MACS). In an example embodiment, cells are first sorted using, for example, antibody coated (para)magnetic beads to sort antigen-specific T cells. Both tube-based and column-based methods for MACS can be used to isolate rare cell populations, or to further enrich a cell (sub)population of interest. Multiple rounds of MACS can further enrich cells, with successive rounds enriching with the same epitope tag or with different epitope tags. See, e.g. Lee et al., J. Biomol. Tech. 2012 Jull 23(2): 69-77. Cells can be eluted removing the magnetic bead where necessary, and further processed, including further enrichment. In one embodiment, T cells can be isolated from peripheral blood lymphocytes by lysing the red blood cells and depleting the monocytes, for example, by centrifugation through a PERCOLL^{™} gradient. A specific subpopulation of T cells, such as CD28+, T cells, can be further isolated by positive or negative selection techniques. For example, in one preferred embodiment, T cells are isolated by incubation with anti-CD3/anti-CD28 (i.e., 3×28)-conjugated beads, such as DYNABEADS^{®} M-450 CD3/CD28 T, or XCYTE DYNABEADS^{™} for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another preferred embodiment, the time period is 10 to 24 hours. In one preferred embodiment, the incubation time period is 24 hours. Once cells of interest are sorted, enriched, and/or isolated, the samples can be further processed, for example, by extraction of nucleic acids, appending of barcodes, droplet formation and analysis.

In some embodiments, the biological sample may include, but is not necessarily limited to, blood, plasma, serum, urine, stool, sputum, mucous, lymph fluid, synovial fluid, bile, ascites, pleural effusion, seroma, saliva, cerebrospinal fluid, aqueous or vitreous humor, or any bodily secretion, a transudate, an exudate (for example, fluid obtained from an abscess or any other site of infection or inflammation), or fluid obtained from a joint(for example, a normal joint or a joint affected by disease, such as rheumatoid arthritis, osteoarthritis, gout or septic arthritis), or a swab of skin or mucosal membrane surface. In specific embodiments, the sample may be blood, plasma or serum obtained from a human patient.

In some embodiments, the sample may be a plant sample. In some embodiments, the sample may be a crude sample. In some embodiments, the sample may be a purified sample.

### Methods

### Generating Sets of Guide molecules

Sets of guide molecules are generated, with each set of guide molecules comprising molecules capable of binding one or more target sequences of the same target molecules and designed to form a complex with a Cas protein. Design of guide molecules can be as described elsewhere herein. In particular embodiments, the guide molecule is designed to be longer than needed for binding of a target molecule, with an additional length of the molecule designed to be complementary to a length of a DNA sequence so that the guide molecule can hybridize to the DNA sequence, which may be appended to a bead at different locations of a bead. Once the guide molecules are prepared with the additional length complementary to DNA sequences, the distribution of the guide molecules to the beads can allow for the hybridization of the bead which can comprise the complementary DNA molecule to the guide molecule. In an aspect, the sets of guides are provided in a single solution and the hybridization sequence allows for the guides to sort and hybridize to appropriate beads based on their hybridization sequence.

The sets of the guide molecules may further comprise unique molecule identifiers, a binding partner for a binding -partner pair which may be provided on the 5' or 3' end of the molecule, optical barcodes, or a combination thereof.

### Distributing Guide Molecules

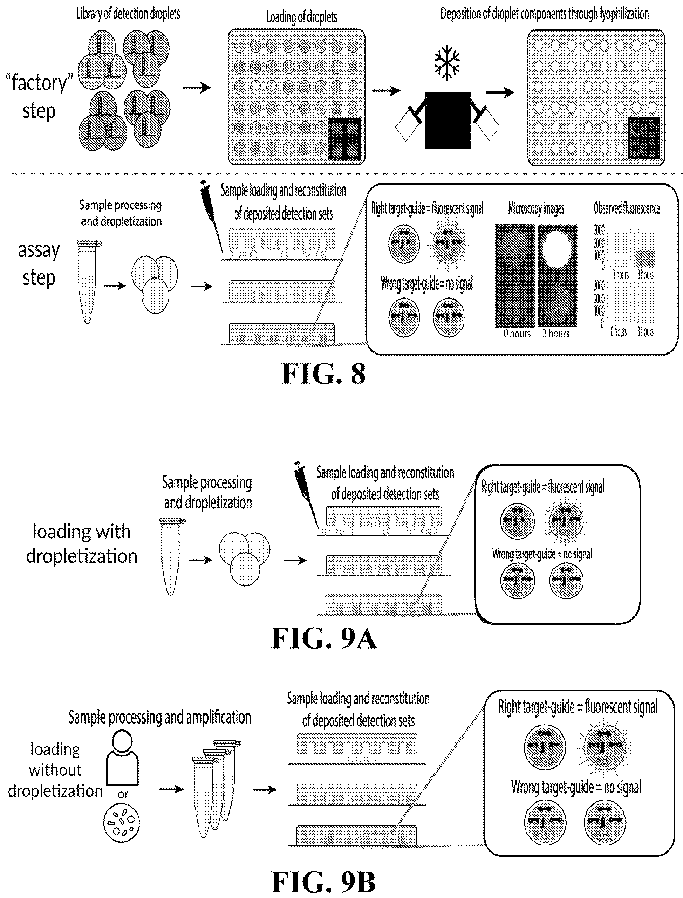

Distributing the guide molecules can include distributing individual guide molecules to individual discrete volumes. Distributing to individual discrete volumes can facilitate the spatial segregation of sets of guide molecules. Spatial segregation as described herein allows the distribution of sets of guide molecules in space such that identification and detection of signals generated can be identified and/or detected. Distribution may include distributing the guide molecules with reporter molecules and one or more other items or reagents to the individual discrete volumes. Such distribution can be automated or random. The distribution can occur all at one time, separately in a series of sequential steps, or one or more of the items to be distributed can be distributed together at discrete time frames. Without being bound by theory, the spatial segregation allows for reduction in background noise and stronger signal read for the detection of target molecules. In certain embodiments, distribution can be provided in droplets, optionally with subsequent lyophilization, as described in Figures 8-9C.

In one embodiment, distributing to each set of guide molecules a sample solution, detection reagents, and a reporter construct comprising a non-target sequence, and a Cas protein may comprise all of the components at once. In one embodiment, the sample solution, detection reagents, and a reporter construct comprising a non-target sequence, and a Cas protein can be present on the microfluidic device or flow cell onto which the guide molecules can be distributed. In one aspect, one or more of the Cas protein, detection reagents, guide molecules and/or reporter construct are lyophilized and provided on the substrate with distribution of the sample solution and any further components to the lyophilized reagents.

In embodiments, the individual discrete volumes are one or more beads. Distributing the guide molecules to the beads can comprise the attachments or association of the guide molecules to the bead, which is referred to herein as a detection bead. In an aspect, the guide molecules are chemically linked at their 5' or 3' end to the beads, allowing for cleavable or non-cleavable linkage of the guide molecule to the bead. The guide molecules can be coupled to the bead in a density that allows for the Cas protein to associate with the guide molecule while on the bead. When utilizing beads as the individual discrete volume, the beads may further be located in wells or encapsulated in droplets.

In an aspect, the guide molecule comprises on a 5' or 3' end a first binding partner of a binding-partner pair. The individual discrete volume may comprise a second binding partner of the binding-partner pair. Upon distribution of the guide molecule to the individual discrete volume, the guide molecule can bind to the individual discrete volume. In an aspect, the first binding partner is biotin and the second binding partier is streptavidin, or can be as described elsewhere herein.

In an aspect, the sets of guide molecules are pooled, and beads comprising a DNA sequence complementary to a designed portion of a guide molecule (e.g. extended spacer sequence) are pooled. The sets of guide molecules and the beads comprising the DNA complementary sequence can be mixed, and the guide molecules will sort to the beads and hybridize to the complementary DNA sequence on the bead.

In an aspect, the guides are distributed to a device such as a microwell array. The microwell array can comprise encoded reagent sets, which may be deposited prior to or with additional reagents, including the sets of guide molecules and reagents that comprise one or more detectable signals that can be encoded in the microwell array. Detectable signals may comprise a level of absorbance, fluorescence, luminescence, polarization, lifetime or other linear or nonlinear optical property. Detectable signals may be utilized for readout of presence of target molecule, barcodes encoded in each well or for particular guide molecule in sets of guide molecules, or other identifier associated with the guide molecule, sample, individual discrete volume, or a combination thereof.

In an aspect, the beads comprising one or more guides comprise a unique optical label. The beads functionalized with guide RNAs each associated with a unique optical label can be pooled together and distributed by, for example, encapsulating the beads in droplets and flowing the beads across a flow cell. In one embodiment, the bead can be distributed across a flow cell and guide molecules allowed to bind with a binding partner present on the flow cell.

### Distributing Reagents to the Guide Molecules

Distribution of reagents may occur all at one time, separately in a series of sequential steps, or one or more of the items to be distributed can be distributed together at discrete time frames. For example, the Cas protein may be mixed with the sets of guide molecules prior to distribution of the guide molecules to individual discrete molecules, ensuring complexing of the Cas molecule to the guide molecules. The guide molecules, and optionally one or more of the Cas protein, detection reagents and/or reporter construct can be lyophilized after distribution to individual discrete volumes. In an aspect, the guide molecules are lyophilized in a well, or after attachment to a bead. Optionally, the Cas protein is lyophilized with the guide molecules subsequent to distribution. Other reagents, including detection constructs, optical barcodes, unique molecular identifiers, amplification reagents, can be distributed together with guide molecules, and can optionally be lyophilized. In an embodiment, when the guide molecules are being distributed to a microwell array, the array wells may be encoded, which allows distribution of sample to pre-decoded well, particularly advantageous when supplying the microwell device as part of a kit.

### Initiating a detection reaction

Initiation of the detection reaction allows for the Cas protein to cleave the non-target sequence of the reporter constructs once activated by the presence of target sequences. Initiation may occur upon contact of a sample comprising a target sequence with the Cas protein, guide molecules and reporter construct. The reaction may be initiated upon incubation, which may occur at room temperature or at 37C, or comprise incubation at one or more temperatures.

In some cases, the microfluidic device is incubated for a period of time prior to imaging/assessment and imaged or assessed at multiple time points to track changes in the measured amount of reporter over time. In an aspect, samples can be incubated at 37 °C.

### Measuring the signal generated

Methods comprise measuring the signal generated from the reporter construct associated with each set of guide molecules from cleavage of the non-target sequence of the reporter constructs. In addition, optical assessments can be made at varying time frames, for example upon initiating a detection reaction and at several time frames. initiation of the CRISPR detection system with the target molecules. Once the target molecule is detected by a guide molecule, the CRISPR effector protein is activated, deactivating the masking construct, for example, by cleaving the masking construct such that a detectable positive signal is unmasked, released, or generated. Detection and measuring a detectable signal of each merged droplet at one or more time periods can be performed, indicating the presence of target molecules when, for example the positive detectable signal is present. In one embodiment imaging is end-point imaging only. As described elsewhere herein, the methods and approaches can allow for a fully automated image analysis approach, allowing for multiplexing and sample throughput with lower per sample cost. Imaging may be for example, fluorescent, visible light or other imaging modality that allows for the visualization/detection of the detectable signal. In one aspect,the imaging can be conducted in more than one channel and may comprise capturing one or more images in one or more channels corresponding to the optically encoded particles. Exemplary measurements of signals generated has been shown, for example, as described in C.M. Ackerman and C. Myrhvold et al., Massively multiplexed nucleic acid detection with Cas13, Nature 582, 277-282 (2020)/

### Kits

Kits comprising the system and for performing the high throughput methods disclosed herein are also provided. In an aspect (described but not specifically claimed herein), the kit comprises a device comprising a set of arrayed capture wells, the capture wells comprising lyophilized guide molecules, and optionally a Cas protein, detection reagents, reporter molecule, or a combination thereof. The kit may further comprise a pre-decoded microwell array.

A kit comprising a device comprising a set of arrayed capture wells; a Cas protein; one or more sets of detection beads, wherein each bead in a set comprises a plurality of guide sequences configured to detect a particular target sequence and each set of beads is configured to detect a different target sequence; and a reporter construct comprising a non-target sequence are also provided.

A kit comprising a flow cell; a Cas protein; one or more sets of detection beads, wherein each bead in a set comprises a plurality of guide sequences configured to detect a particular target sequence and each set of beads is configured to detect a different target sequence; and a reporter construct comprising a non-target sequence are also provided.

The kits may further comprise a means for generating droplets, the droplets comprising at least a sample, the Cas protein, and the detection bead. Droplet generators are known in the art, for example, QX200, BioRad. Droplets can be formed as sample fluid flows from droplet generator which contains lysis reagent and barcodes through microfluidic outlet channel which contains oil, towards junction. Defined volumes of loaded reagent emulsion, corresponding to defined numbers of droplets, are dispensed on-demand into the flow stream of carrier fluid. Equipment requirements for droplet generation can be substantially reduced by using a custom-fabricated pressure manifold or other droplet generation hardware.

In an aspect, the device may further comprise one or more spacer lanes between the wells.

Mechanisms may be provided for inducing fusion of droplets in the capture wells. Merger or fusion of the droplets can be accomplished by, for example, passing the tip of a corona treater over the device. Other mechanisms for merger are known in the art, with an aspect applying a high voltage AC electric field to induce droplet merging.

The kit may be provided with one or more reporter constructs, and the reporter construct can optionally be attached to a reporter bead. In an aspect, the detection bead and reporter bead are of different sizes and the capture wells of the device can be sized to hold one of each, a detection bead and a reporter bead.

The guide molecule may comprise, on a 5' or 3' end, a first binding partner of a binding-partner pair, and the bead comprises a second binding partner of the binding-partner pair. In an aspect, the first binding partner is biotin and the second binding partner is streptavidin.

The Cas protein provided with the kits described herein can be an RNA-targeting Cas or a DNA targeting Cas. The Cas protein can be as described elsewhere herein, for example, a Class 1 or Class 2 Cas protein. The Cas protein can be a Class 2, Type II Cas, Type V Cas, or Type VI Cas. Detection reagents as described herein may further comprise amplification reagents in the kit. The amplification reagents are isothermal amplification reagents. In an aspect, the one or more of the detection reagents, detection bead, and reporter construct are lyophilized inside the capture wells of the device.

### Microfluidic devices comprising an array of microwells

Microfluidic devices comprising an array of microwells can be utilized in the systems, methods and kits of the present invention. In certain example embodiments, the device is a microfluidic capable of generating and/or merging different droplets, which may comprise a detection bead (i.e. individual discrete volumes). For example, a first set of droplets may be formed containing samples to be screened and a second set of droplets formed containing the elements of the systems, including described herein. The first and second set of droplets can be merged and then diagnostic methods as described herein are carried out on the merged droplet set. The microfluidic devices can be provided with one or more elements of the systems lyophilized, in a preferred embodiment, the sets of guide molecules are lyophilized. Microfluidic devices may comprise wells that are pre-decoded, allowing use of the device and identity of the wells and/or their contents prior to sample loading.

Microfluidic devices disclosed herein may be silicone-based chips and may be fabricated using a variety of techniques, including, but not limited to, hot embossing, molding of elastomers, injection molding, LIGA, soft lithography, silicon fabrication and related thin film processing techniques. Suitable materials for fabricating the microfluidic devices include, but are not limited to, cyclic olefin copolymer (COC), polycarbonate, poly(dimethylsiloxane) (PDMS), and poly(methylacrylate) (PMMA). In one embodiment, soft lithography in PDMS may be used to prepare the microfluidic devices. For example, a mold may be made using photolithography which defines the location of flow channels, valves, and filters within a substrate. The substrate material is poured into a mold and allowed to set to create a stamp. The stamp is then sealed to a solid support, such as but not limited to, glass. Due to the hydrophobic nature of some polymers, such as PDMS, which absorbs some proteins and may inhibit certain biological processes, a passivating agent may be necessary (Schoffner et al. Nucleic Acids Research, 1996, 24:375-379). Suitable passivating agents are known in the art and include, but are not limited to, silanes, parylene, n-Dodecyl-b-D-matoside (DDM), pluronic, Tween-20, other similar surfactants, polyethylene glycol (PEG), albumin, collagen, and other similar proteins and peptides.

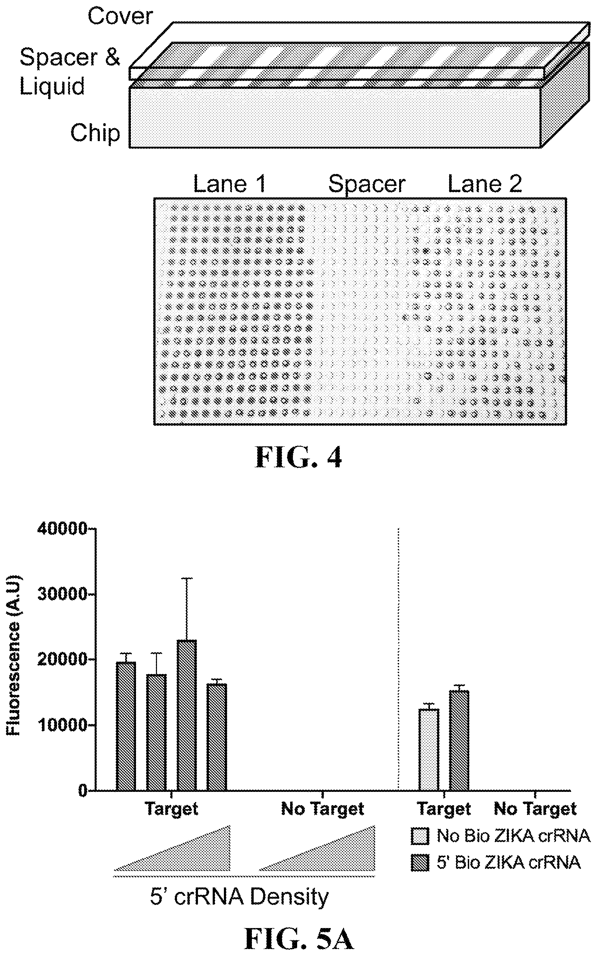

Exemplary microfluidic devices that may be used in the context of the invention is described in Kulesa, et al. PNAS, 115, 6685-6690, as shown in FIG. 4. In an exemplary design, the lane dimensions are allocated to align with a 384W plate at 3.5 mm wide and accommodate 25 µL of fluid with a 1 mm spacer x 1 cm x 0.5 mm. The number of wells per lane are flexible, and while an exemplary lane length was designed to accommodate 1000 wells/lane of 100 µm diameter wells, other numbers of wells are possible, and may vary depending on well size and, if beads are used, bead size and whether a separate detection bead and reporter bead are utilized.

In certain example embodiments, the device may comprise individual wells, such as microplate wells. The size of the microplate wells may be the size of standard 6, 24, 96, 384, 1536, 3456, or 9600 sized wells. In certain embodiments, the microwells can number at more than 40,0000 or more than 190,000. In certain example embodiments, the elements of the systems described herein may be freeze dried and applied to the surface of the well prior to distribution and use.

Microwell chips can be designed as disclosed in US Patent Application No. 15/559, 381. In one embodiment, the microwell chip can be designed in a format measuring around 6.2 x 7.2 cm, containing 49200 microwells, or a larger format, measuring 7.4 x 10 cm, containing 97, 194 microwells. The array of microwells can be shaped, for example, as two circles of a diameter of about 50 - 300 µm, in particular embodiments at 150 µm diameter set at 10% overlap. The array of microwells can be arranged in a hexagonal lattice at 50 µm inter-well spacing. In some instances, the microwells can be arranged in other shapes, spacing and sizes in order to hold a varying number of droplets. The microwell chips are advantageously, in some embodiments, sized for use with standard laboratory equipment, including imaging equipment such as microscopes.

In an exemplary method, compounds can be mixed with a unique ratio of fluorescent dyes (e.g. Alexa Fluor 555, 594, 647). Each mixture of target molecule with a dye mixture can be emulsified into droplets. Similarly, each detection CRISPR system with optical barcode can be emulsified into droplets. In some embodiments, the droplets are approximately 1 nL each. The CRISPR detection system droplets and target molecule droplets can then be combined and applied to the microwell chip. The droplets can be combined by simple mixing or other methods of combination. Exemplary embodiments of use of the chips in combination with droplets can be as described in PCT.US2019/061577 at [0510]-[0514].

The devices disclosed may further comprise inlet and outlet ports, or openings, which in turn may be connected to valves, tubes, channels, chambers, and syringes and/or pumps for the introduction and extraction of fluids into and from the device. The devices may be connected to fluid flow actuators that allow directional movement of fluids within the microfluidic device. Example actuators include, but are not limited to, syringe pumps, mechanically actuated recirculating pumps, electroosmotic pumps, bulbs, bellows, diaphragms, or bubbles intended to force movement of fluids. In certain example embodiments, the devices are connected to controllers with programmable valves that work together to move fluids through the device. In certain example embodiments, the devices are connected to the controllers discussed in further detail below. The devices may be connected to flow actuators, controllers, and sample loading devices by tubing that terminates in metal pins for insertion into inlet ports on the device.

The present invention may be used with a wireless lab-on-chip (LOC) diagnostic sensor system (see e.g., US patent number 9,470,699 "Diagnostic radio frequency identification sensors and applications thereof"). In certain embodiments, the present invention is performed in a LOC controlled by a wireless device (e.g., a cell phone, a personal digital assistant (PDA), a tablet) and results are reported to said device.

Radio frequency identification (RFID) tag systems include an RFID tag that transmits data for reception by an RFID reader (also referred to as an interrogator). In a typical RFID system, individual objects (e.g., store merchandise) are equipped with a relatively small tag that contains a transponder. The transponder has a memory chip that is given a unique electronic product code. The RFID reader emits a signal activating the transponder within the tag through the use of a communication protocol. Accordingly, the RFID reader is capable of reading and writing data to the tag. Additionally, the RFID tag reader processes the data according to the RFID tag system application. Currently, there are passive and active type RFID tags. The passive type RFID tag does not contain an internal power source, but is powered by radio frequency signals received from the RFID reader. Alternatively, the active type RFID tag contains an internal power source that enables the active type RFID tag to possess greater transmission ranges and memory capacity. The use of a passive versus an active tag is dependent upon the particular application.

Lab-on-the chip technology is well described in the scientific literature and consists of multiple microfluidic channels, input or chemical wells. Reactions in wells can be measured using radio frequency identification (RFID) tag technology since conductive leads from RFID electronic chip can be linked directly to each of the test wells. An antenna can be printed or mounted in another layer of the electronic chip or directly on the back of the device. Furthermore, the leads, the antenna and the electronic chip can be embedded into the LOC chip, thereby preventing shorting of the electrodes or electronics. Since LOC allows complex sample separation and analyses, this technology allows LOC tests to be done independently of a complex or expensive reader. Rather a simple wireless device such as a cell phone or a PDA can be used. In one embodiment, the wireless device also controls the separation and control of the microfluidics channels for more complex LOC analyses. In one embodiment, a LED and other electronic measuring or sensing devices are included in the LOC-RFID chip. Not being bound by a theory, this technology is disposable and allows complex tests that require separation and mixing to be performed outside of a laboratory.

In preferred embodiments, the LOC may be a microfluidic device. The LOC may be a passive chip, wherein the chip is powered and controlled through a wireless device. In certain embodiments, the LOC includes a microfluidic channel for holding reagents and a channel for introducing a sample. In certain embodiments, a signal from the wireless device delivers power to the LOC and activates mixing of the sample and assay reagents. Specifically, in the case of the present invention, the system may include a masking agent, CRISPR effector protein, and guide RNAs specific for a target molecule. Upon activation of the LOC, the microfluidic device may mix the sample and assay reagents. Upon mixing, a sensor detects a signal and transmits the results to the wireless device. In certain embodiments, the unmasking agent is a conductive RNA molecule. The conductive RNA molecule may be attached to the conductive material. Conductive molecules can be conductive nanoparticles, conductive proteins, metal particles that are attached to the protein or latex or other beads that are conductive. In certain embodiments, if DNA or RNA is used then the conductive molecules can be attached directly to the matching DNA or RNA strands. The release of the conductive molecules may be detected across a sensor. The assay may be a one step process.

Since the electrical conductivity of the surface area can be measured precisely quantitative results are possible on the disposable wireless RFID electro-assays. Furthermore, the test area can be very small allowing for more tests to be done in a given area and therefore resulting in cost savings. In certain embodiments, separate sensors each associated with a different CRISPR effector protein and guide RNA immobilized to a sensor are used to detect multiple target molecules. Not being bound by a theory, activation of different sensors may be distinguished by the wireless device.

In addition to the conductive methods described herein, other methods may be used that rely on RFID or Bluetooth as the basic low-cost communication and power platform for a disposable RFID assay. For example, optical means may be used to assess the presence and level of a given target molecule. In certain embodiments, an optical sensor detects unmasking of a fluorescent masking agent.

In certain embodiments, the device of the present invention may include handheld portable devices for diagnostic reading of an assay (see e.g., Vashist et al., Commercial Smartphone-Based Devices and Smart Applications for Personalized Healthcare Monitoring and Management, Diagnostics 2014, 4(3), 104-128; mReader from Mobile Assay; and Holomic Rapid Diagnostic Test Reader).

As noted herein, certain embodiments allow detection via colorimetric change which has certain attendant benefits when embodiments are utilized in POC situations and or in resource poor environments where access to more complex detection equipment to readout the signal may be limited. However, portable embodiments disclosed herein may also be coupled with hand-held spectrophotometers that enable detection of signals outside the visible range. An example of a hand-held spectrophotometer device that may be used in combination with the present invention is described in Das et al. "Ultra-portable, wireless smartphone spectrophotometer for rapid, non-destructive testing of fruit ripeness." Nature Scientific Reports. 2016, 6:32504, DOI: 10.1038/srep32504. Finally, in certain embodiments utilizing quantum dot-based masking constructs, use of a hand-held UV light, or other suitable device, may be successfully used to detect a signal owing to the near complete quantum yield provided by quantum dots.

The invention is further described in the following examples, which do not limit the scope of the invention described in the claims.

### EXAMPLES

### Example 1 - Spatial Segregation and Detection

An exemplary design for spatial segregation of CRISPR guide RNAs is provided in Figure 1. The reporter may be in solution or on a bead. If both reporter and crRNA are on beads, the beads can be different sizes, with wells sized to hold one crRNA detection bead and one reporter bead per well. Exemplary attachment strategies include direct modification of biotin at the 5' end of crRNA, the 3' end of crRNA (Figure 2, upper panel). A short complementary DNA to a part of the spacer sequence, or an extended sequence of the guide RNA is shown in an example embodiment of Figure 2 (middle panel). A FAM-biotin reporter may be appended to a detection bead, the reporter leaving the bead upon cleaving. (Figure 2, lower panel).

More than one size of bead can be utilized in methods, an exemplary embodiment shows a use of a small diameter bead and a large diameter bead. (FIG. 3). The small beads Dynabeads M280, with a diameter of about 2.8 um, can be purified with a magnet with enough surface area to use in bulk reactions and evaluate attachment chemistries with about 1e5 oligonucleotides attached per bead. Exemplary large beads with a diameter of about 100 µm are Spherotech polystyrene. The beads can be centrifuged to obtain a pellet; the beads can be evaluated in microwells, using a density of about 1e8 oligos per bead. An increased surface area may be necessary for use with bulk reactions.

Figure 4 depicts an exemplary chip design with dimensions allocated to align with a 384 well plate. In this exemplary design, the lane dimensions are allocated to align with a 384W plate at 3.5 mm wide and accommodate 25 µL of fluid with a 1 mm spacer x 1 cm x 0.5 mm.

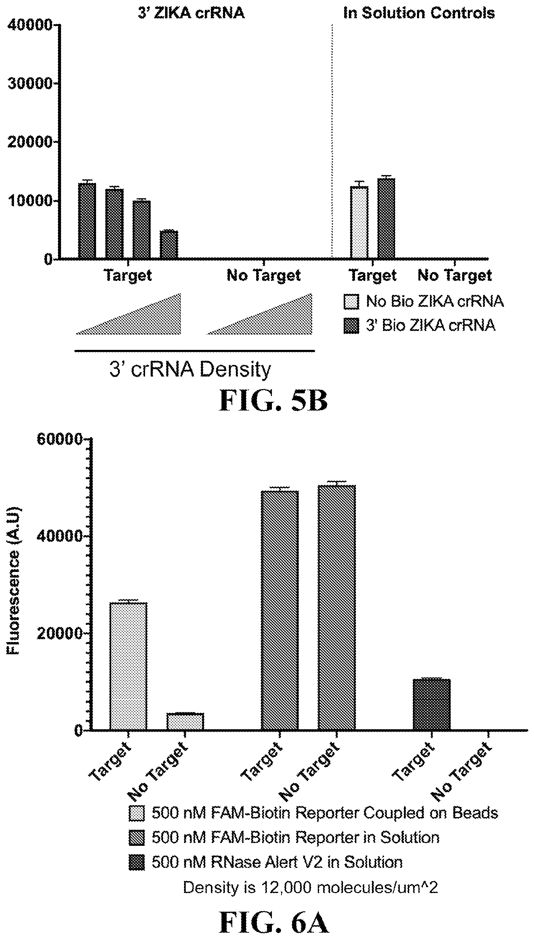

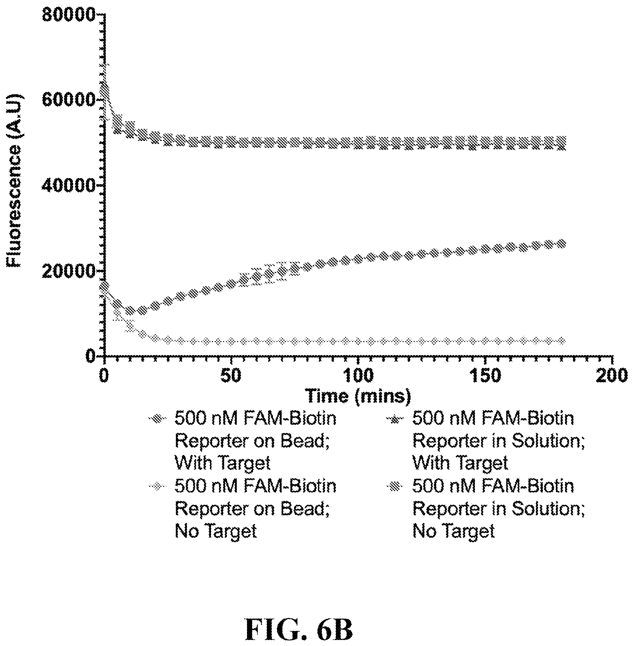

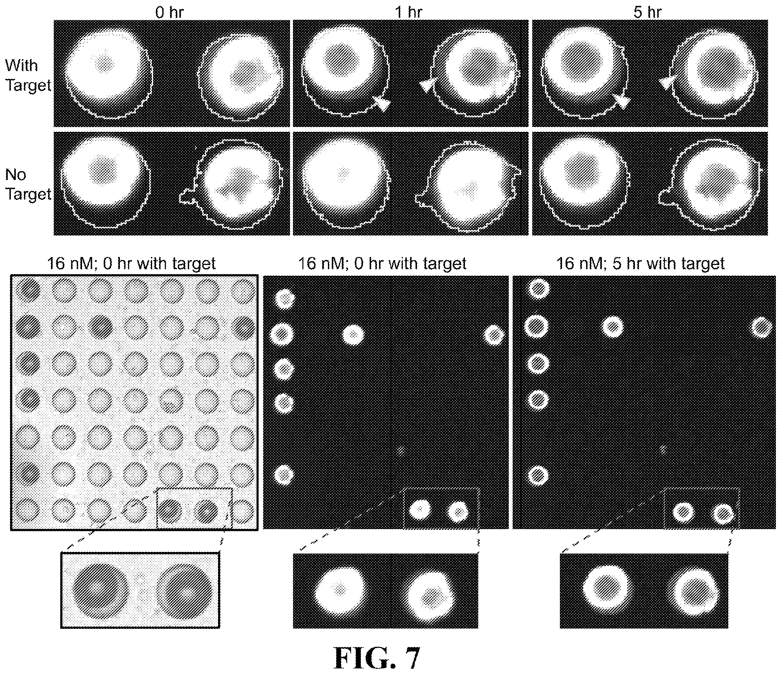

In an example evaluation, biotinylated crRNAs can be coupled to streptavidin beads are evaluated. Zika crRNAs biotinylated at the 5' end (FIG. 5A) or at the 3' end (FIG. 5B) and coupled at different concentrations. Lowest crRNA density is 6,000 molecules/µm², 2-fold steps in concentration. Figures 6A and 6B explores the FAM-Biotin reporter that is quenched when coupled to streptavidin beads. Fluorescence is tracked when the reporter is coupled on beads or in solution (FIG. 6A); fluorescence kinetics of the FAM-Biotin reporter in solution and on bead, with and without target. Figure 7 includes imaging of an exemplary FAM-Biotin reporter couple to 100 um beads used in microwell format, shown with and without target and at several time points.

Exemplary lyophilization workflows are included in both Figures 8 and 9A-9C. In Figure 8, example steps include the step of preparing a library of detection droplets, loading of droplets in wells, deposition of droplet components through lyophilization and assay step for sample loading. Figure 9B includes sample processing and amplification, including sample loading and reconstitution of deposited detection sets, with generation of a fluorescent signal upon detection of a target in the sample. Figure 9C includes detection results with ability to use workflow loading without dropletization, for example as depicted in Figure 9B.

Figure 10 depicts differences of microfluidic device loading with and without plasma treatment, with a measurement of signal to background indicating plasma treatment increases the signal:background ratio.

### Example 2 - Simplified Workflow

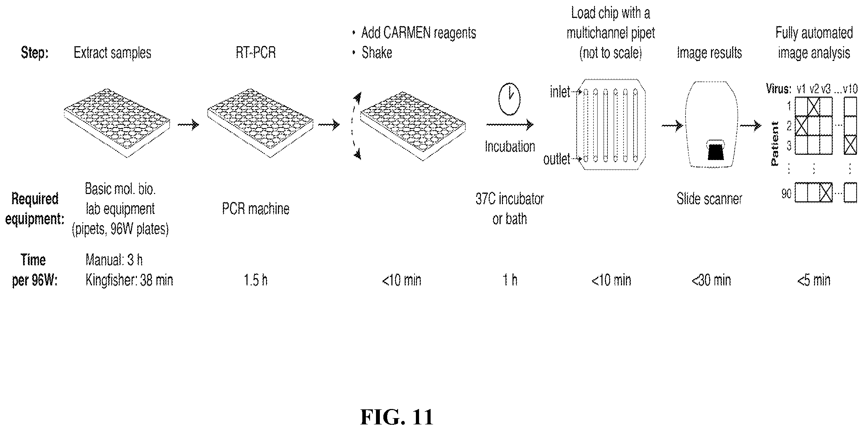

The current example provides a workflow process utilizing low barrier to entry capital equipment while remaining sensitive, simple and enabling differential diagnosis of multiple pathogens. An exemplary user workflow is shown in Figure 11. The equipment required in the workflow is basic lab equipment, including 96W plates, pipettes, a PCR machine, incubator or bath, and a slide scanner. As depicted in the example workflow of Figure 11, total workflow time is less than about 4 hours and allows for high sensitivity, about 1-10 cp/ul, multiplexing (10-plex) and sample throughput (1,000 per PCR machine per day, or 500 per FTE per day with automated extraction), with low capital cost and per-sample cost (<$10). Manual extraction of samples increases total assay time, with alternative extraction methods being considered fopr circumvention in the workflow. Advantageously, the sample workflow and assay detailed will provide 10-plex differential diagnosis with the throughput and sensitivity of single-plex RT-qPCR, and with higher specificity.

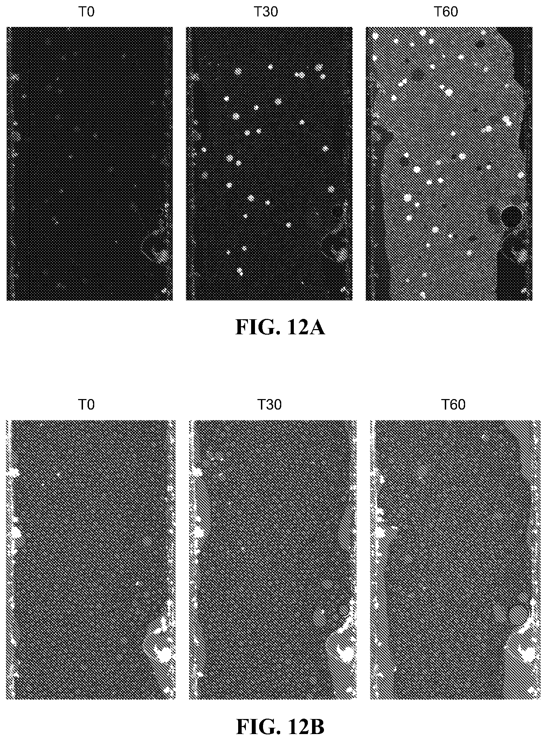

An exemplary experimental design utilizing Human metapneumovirus (HMPV) specific beads was conducted with limit of detection 1e6 → 1e0 copies/uL (cp/uL) with RNaseP in the master mix. Pooled beads were explored versus the targets. Experiments were evaluated on two chips, the first with measurements at room temperature for the first 10 minutes, the second chip performed at room temperature for the first 30 minutes with a 30-minute incubation at 37C for T60. Droplets generated were flowed onto flow cells and imaged at time intervals. Figures 12A and 12B provide exemplary images of beads comprising sequence specific for HMPV. Figure 12A provides fluorescence images in blue channel of HMPV bead with 1e0 target and 37°C incubation time where droplets were injected into a flow chamber; Figure 12B is the transmitted light image in the UV channel showing droplets containing the beads that test for virus that is present in the sample light up. Figure 13 shows heatmap of a chip incubated at 37°C, with pooled beads at difference concentrations, and measured reporter intensity. This simplified, deployable version of high=throughput multiplexed diagnostics platform can be used on commercially available instruments, with a simplified deployable version that allows for reduced capital equipment. Simplified nucleic acid extraction (<30 min per 96W plate with no robot required), crRNAs coupled to color-coded or other optically encoded beads that can be pooled ahead of time, simple, easy emulsification performed by shaking without a requirement for microfluidics or merging of droplets, end-point imaging only, and a fully automated image analysis pipeline are provided with the exemplary methods applicants have developed herein.

## Claims

1. A high throughput method for detecting target molecules comprising:
a. generating sets of guide molecules, each set of guide molecules comprising guide molecules capable of binding one or more target sequences of a target molecule and designed to form a complex with a Cas protein;
b. distributing a plurality of sets of guide molecules, thereby spatially segregating each set of guide molecules, comprising distributing the plurality of sets of guide molecules to individual discrete volumes;
c. distributing, to each set of guide molecules, a sample solution, detection reagents, and a reporter construct comprising a non-target sequence, and a Cas protein;
d. initiating a detection reaction, wherein the Cas protein cleaves the non-target sequence of the reporter constructs once activated by the one or more target sequences; and
e. measuring the signal generated from the reporter construct associated with each set of guide molecules from cleavage of the non-target sequence of the reporter constructs,
wherein each individual discrete volume comprises a set of one or more detection beads, wherein each detection bead comprises the set of guide molecules capable of binding the one or more target sequences of the target molecule, wherein the detection bead is encapsulated in a droplet,
wherein a reporter bead is encapsulated in the same droplet as the detection bead, or in a separate droplet that can be fused with the droplet comprising the detection bead, and
wherein the detection bead and the reporter bead are sized differently and such that each individual discrete volume can only hold one of each bead.

2. The method of claim 1, wherein the detection reagents are encoded prior to being delivered to the individual discrete volumes; and/or
the guide molecule comprises RNA, the RNA comprising a nucleic acid sequence hybridized to a DNA linker sequence disposed on the detection bead.

3. The method of claim 2, further comprising the step of mixing the detection bead with Cas protein, thereby coupling the Cas protein to the set of guide molecules disposed on the bead; or
wherein the guide molecule is a nucleic acid and is coupled to the detection bead at the 5' end or the 3' end of the guide molecule; or
wherein the method further comprises multiple sets of detection beads, each bead in a given set comprising guide molecules configured to detect a particular target molecule, and each different set of beads configured to detect a different target molecule such that detection of multiple target molecules is screened at once.

4. The method of claim 1, wherein the reporter construct is attached to a reporter bead.

5. The method of any one of claims 1, wherein the distributing comprises distributing to wells arrayed on a microfluidic device,
optionally further comprising:
lyophilizing the guide molecules, and optionally the Cas protein, detection reagents, reporter molecule, or a combination thereof; and
amplifying target molecules in the sample prior to distributing the sample.

6. The method of claim 3, wherein the beads are sized between 2 µm to 100 µm.

7. The method of claim 3, wherein the guide molecule comprises, on a 5' or 3' end, a first binding partner of a binding-partner pair, and the bead comprises a second binding partner of the binding-partner pair;
optionally wherein the first binding partner is biotin and the second binding partner is streptavidin.

8. The method of claim 1, wherein the guide molecule further comprises optical barcodes associated with the guide molecules, the sample, or both.

9. The method of claim 3, wherein the detection bead, reporter bead, or both are magnetic.

10. The method of claim 1, wherein the detection reagents further comprise amplification reagents,
optionally wherein the amplification reagents are isothermal amplification reagents.

11. The method of claim 1, wherein the Cas protein is an RNA-targeting protein, a DNA-targeting protein, or a combination thereof,
optionally wherein the Cas protein is a Class 1 or Class 2 Cas protein,
further optionally wherein the Cas protein is a Class 2, Type II, Type V, or Type VI protein.

12. The method of claim 1, wherein the detectable signal is a level of absorbance, fluorescence, luminescence, polarization, lifetime, or other linear or nonlinear optical property.

13. The method of claim 1, further comprising optical barcodes or unique molecular identifiers associated with the guide molecule, the sample, the individual discrete volume, or a combination thereof.

14. A kit comprising:
a. a device comprising a set of arrayed capture wells;
b. a Cas protein;
c. one or more sets of detection beads, wherein each bead in a set comprises a plurality of guide sequences configured to detect a particular target sequence and each set of beads is configured to detect a different target sequence;
d. a reporter construct comprising a non-target sequence;
wherein the reporter construct is attached to a reporter bead; and
wherein the detection bead and reporter bead are of different sizes and wherein the capture wells are sized to hold one of each.

## Patentansprüche

1. Ein Hochdurchsatzverfahren zum Nachweis von Zielmolekülen, umfassend:
a. Erzeugen von Sätzen von Leitmolekülen, wobei jeder Satz von Leitmolekülen Leitmoleküle umfasst, die eine oder mehrere Zielsequenzen eines Zielmoleküls binden können und so konzipiert sind, dass sie einen Komplex mit einem Cas-Protein bilden;
b. Verteilen einer Vielzahl von Sätzen von Leitmolekülen, wodurch jeder Satz von Leitmolekülen räumlich getrennt wird, umfassend das Verteilen der Vielzahl von Sätzen von Leitmolekülen auf einzelne diskrete Volumina;
c. Verteilen einer Probenlösung, von Nachweisreagenzien und eines Reporterkonstrukts, das eine Nicht-Zielsequenz und ein Cas-Protein umfasst, an jeden Satz von Leitmolekülen;
d. Auslösen einer Nachweisreaktion, wobei das Cas-Protein die Nicht-Zielsequenz der Reporter Konstrukte spaltet, sobald es durch die eine oder die mehreren Zielsequenzen aktiviert wurde; und
e. Messen des Signals, das von dem mit jeder Gruppe von Leitmolekülen assoziierten Reporterkonstrukt durch Spaltung der Nicht-Zielsequenz der Reporter Konstrukte erzeugt wird,
wobei jedes einzelne diskrete Volumen eine Gruppe von einem oder mehreren Detektionskügelchen umfasst, wobei jedes Detektionskügelchen die Gruppe von Leitmolekülen umfasst, die in der Lage sind, die eine oder mehreren Zielsequenzen des Zielmoleküls zu binden, wobei das Detektionskügelchen in einem Tröpfchen eingekapselt ist,
wobei ein Reporterkügelchen in demselben Tröpfchen wie das Detektionskügelchen oder in einem separaten Tröpfchen, das mit dem Tröpfchen, das das Detektionskügelchen umfasst, fusioniert werden kann, eingekapselt ist, und
wobei das Detektionskügelchen und das Reporterkügelchen unterschiedlich groß sind und so, dass jedes einzelne diskrete Volumen nur einen von jedem Kügelchen aufnehmen kann.

2. Verfahren nach Anspruch 1, wobei die Nachweisreagenzien vor ihrer Abgabe an die einzelnen diskreten Volumina kodiert werden; und/oder das Leitmolekül RNA umfasst, wobei die RNA eine Nukleinsäuresequenz umfasst, die an eine auf dem Nachweiskügelchen angeordnete DNA-Linkersequenz hybridisiert ist.

3. Verfahren nach Anspruch 2, das ferner den Schritt des Mischens des Nachweiskügelchen mit Cas-Protein umfasst, wodurch das Cas-Protein an den Satz von Leitmolekülen gekoppelt wird, die auf dem Kügelchen angeordnet sind; oder
wobei das Leitmolekül eine Nukleinsäure ist und an das 5'-Ende oder das 3'-Ende des Leitmoleküls an die Nachweisperle gekoppelt ist; oder
wobei das Verfahren ferner mehrere Sätze von Nachweiskügelchen umfasst, wobei jedes Kügelchen in einem gegebenen Satz Leitmoleküle umfasst, die zum Nachweis eines bestimmten Zielmoleküls konfiguriert sind, und jeder verschiedene Satz von Kügelchen zum Nachweis eines anderen Zielmoleküls konfiguriert ist, so dass der Nachweis mehrerer Zielmoleküle gleichzeitig untersucht wird.

4. Verfahren nach Anspruch 1, wobei das Reporterkonstrukt an einem Reporterkügelchen angebracht ist.

5. Verfahren nach wenigstens Anspruch 1, wobei das Verteilen als Verteilen auf einer gut aufgestellten Mikrofluidikvorrichtung angeordnet sind,
optional ferner umfassend:
Lyophilisieren der Leitmoleküle und optional des Cas-Proteins, der Detektionsreagenzien, des Reportermoleküls oder einer Kombination davon; und
Amplifizieren von Zielmolekülen in der Probe vor dem Verteilen der Probe.

6. Verfahren nach Anspruch 3, wobei die Kügelchen eine Größe zwischen 2 µm und 100 µm aufweisen.

7. Verfahren nach Anspruch 3, wobei das Leitmolekül an einem 5'- oder 3'-Ende einen ersten Bindungspartner eines Bindungspartnerpaares umfasst und die Kügelchen einen zweiten Bindungspartner des Bindungspartnerpaares umfassen;
optional wobei der erste Bindungspartner Biotin und der zweite Bindungspartner Streptavidin ist.

8. Verfahren nach Anspruch 1, wobei das Leitmolekül ferner optische Barcodes umfasst, die mit den Leitmolekülen, der Probe oder beiden assoziiert sind.

9. Verfahren nach Anspruch 3, wobei die Detektionskügelchen, die Reporterkügelchen oder beide magnetisch sind.

10. Verfahren nach Anspruch 1, wobei die Detektionsreagenzien ferner Amplifikationsreagenzien umfassen,
wobei die Amplifikationsreagenzien optional isotherme Amplifikationsreagenzien sind.

11. Verfahren nach Anspruch 1, wobei das Cas-Protein ein RNA-bindendes Protein, ein DNA-bindendes Protein oder eine Kombination davon ist,
wobei das Cas-Protein optional ein Cas-Protein der Klasse 1 oder Klasse 2 ist,
wobei das Cas-Protein optional ein Protein der Klasse 2, Typ II, Typ V oder Typ VI ist.

12. Verfahren nach Anspruch 1, wobei das nachweisbare Signal ein Absorptionsgrad, eine Fluoreszenz, eine Lumineszenz, eine Polarisation, eine Lebensdauer oder eine andere lineare oder nichtlineare optische Eigenschaft ist.

13. Verfahren nach Anspruch 1, das ferner optische Barcodes oder eindeutige molekulare Identifikatoren umfasst, die mit dem Leitmolekül, der Probe, dem einzelnen diskreten Volumen oder einer Kombination davon assoziiert sind.

14. Kit, das umfasst:
a. eine Vorrichtung, die einen Satz von angeordneten Auffangvertiefungen umfasst;
b. ein Cas-Protein;
c. einen oder mehrere Sätze von Detektionskügelchen, wobei jedes Kügelchen in einem Satz eine Vielzahl von Leitsequenzen umfasst, die zum Nachweis einer bestimmten Zielsequenz konfiguriert sind, und jeder Satz von Kügelchen zum Nachweis einer anderen Zielsequenz konfiguriert ist;
d. ein Reporterkonstrukt, das eine Nicht-Zielsequenz umfasst; wobei das Reporterkonstrukt an einem Reporterkügelchen angebracht ist; und
wobei das Detektionskügelchen und das Reporterkügelchen unterschiedliche Größen aufweisen und wobei die Auffangvertiefungen so dimensioniert sind, dass sie jeweils eines davon aufnehmen können.

## Revendications

1. Procédé à haut rendement pour la détection de molécules cibles comprenant :
a. la génération d'ensembles de molécules guides, chaque ensemble de molécules guides comprenant des molécules guides aptes à se lier à une ou plusieurs séquences cibles d'une molécule cible et étant conçues pour former un complexe avec une protéine Cas ;
b. la distribution d'une pluralité d'ensembles de molécules guides, séparant ainsi spatialement chaque ensemble de molécules guides, comprenant la distribution de la pluralité d'ensembles de molécules guides à des volumes distincts individuels ;
c. la distribution, à chaque ensemble de molécules guides, d'une solution échantillon, de réactifs de détection et d'une construction rapporteuse comprenant une séquence non-cible, et d'une protéine Cas ;
d. le déclenchement d'une réaction de détection, dans lequel la protéine Cas clive la séquence non-cible des constructions rapporteuses une fois activée par les une ou plusieurs séquences cibles ; et
e. la mesure du signal généré à partir de la construction rapporteuse associée à chaque ensemble de molécules guides à partir du clivage de la séquence non-cible des constructions rapporteuses,
dans lequel chaque volume distinct individuel comprend un ensemble d'une ou de plusieurs billes de détection, dans lequel chaque bille de détection comprend l'ensemble de molécules guides aptes à se lier aux une ou plusieurs séquences cibles de la molécule cible, dans lequel la bille de détection est encapsulée dans une gouttelette,
dans lequel une bille rapporteuse est encapsulée dans la même gouttelette que la bille de détection, ou dans une gouttelette distincte qui peut être fusionnée avec la gouttelette comprenant la bille de détection, et
dans lequel la bille de détection et la bille rapporteuse sont de taille différente et de sorte que chaque volume distinct individuel ne puisse contenir qu'une seule bille de chaque sorte.

2. Procédé selon la revendication 1, dans lequel les réactifs de détection sont codés avant d'être distribués aux volumes distincts individuels ; et/ou
la molécule guide comprend de l'ARN, l'ARN comprenant une séquence d'acide nucléique hybridée à une séquence de liaison d'ADN disposée sur la bille de détection.

3. Procédé selon la revendication 2, comprenant en outre l'étape de mélange de la bille de détection avec la protéine Cas, couplant ainsi la protéine Cas à l'ensemble de molécules guides disposées sur la bille ; ou
dans lequel la molécule guide est un acide nucléique et est couplée à la bille de détection à l'extrémité 5' ou à l'extrémité 3' de la molécule guide ; ou
dans lequel le procédé comprend en outre plusieurs ensembles de billes de détection, chaque bille d'un ensemble donné comprenant des molécules guides configurées pour détecter une molécule cible particulière, et chaque ensemble différent de billes configuré pour détecter une molécule cible différente, de sorte que la détection de plusieurs molécules cibles soit examinée simultanément.

4. Procédé selon la revendication 1, dans lequel la construction rapporteuse est fixée à une bille rapporteuse.

5. Procédé selon l'une quelconque des revendications 1, dans lequel la distribution comprend la distribution dans des puits disposés en réseau sur un dispositif microfluidique,
comprenant en outre éventuellement :
la lyophilisation des molécules guides, et éventuellement de la protéine Cas, des réactifs de détection, de la molécule rapporteuse, ou d'une combinaison de ceux-ci ; et
l'amplification des molécules cibles dans l'échantillon avant la distribution de l'échantillon.

6. Procédé selon la revendication 3, dans lequel les billes sont d'une taille comprise entre 2 µm et 100 µm.

7. Procédé selon la revendication 3, dans lequel la molécule guide comprend, sur une extrémité 5' ou 3', un premier partenaire de liaison d'une paire de partenaires de liaison, et la bille comprend un second partenaire de liaison de la paire de partenaires de liaison ;
éventuellement dans lequel le premier partenaire de liaison est la biotine et le second partenaire de liaison est la streptavidine.

8. Procédé selon la revendication 1, dans lequel la molécule guide comprend en outre des codes-barres optiques associés aux molécules guides, à l'échantillon, ou aux deux.

9. Procédé selon la revendication 3, dans lequel la bille de détection, la bille rapporteuse ou les deux sont magnétiques.

10. Procédé selon la revendication 1, dans lequel les réactifs de détection comprennent en outre des réactifs d'amplification,
éventuellement dans lequel les réactifs d'amplification sont des réactifs d'amplification isothermiques.

11. Procédé selon la revendication 1, dans lequel la protéine Cas est une protéine ciblant l'ARN, une protéine ciblant l'ADN, ou une combinaison de celles-ci,
éventuellement, dans lequel la protéine Cas est une protéine Cas de classe 1 ou de classe 2, en outre éventuellement dans lequel la protéine Cas est une protéine de classe 2, de type II, de type V ou de type VI.

12. Procédé selon la revendication 1, dans lequel le signal détectable est un niveau d'absorbance, de fluorescence, de luminescence, de polarisation, de durée de vie ou d'une autre propriété optique linéaire ou non linéaire.

13. Procédé selon la revendication 1, comprenant en outre des codes-barres optiques ou des identifiants moléculaires uniques associés à la molécule guide, à l'échantillon, au volume distinct individuel, ou à une combinaison de ceux-ci.

14. Kit comprenant :
a. un dispositif comprenant un ensemble de puits de capture disposés en réseau ;
b. une protéine Cas ;
c. un ou plusieurs ensembles de billes de détection, dans lequel chaque bille d'un ensemble comprend une pluralité de séquences de guidage configurées pour détecter une séquence cible particulière et chaque ensemble de billes est configuré pour détecter une séquence cible différente ;
d. une construction rapporteuse comprenant une séquence non-cible ; dans lequel la construction rapporteuse est fixée à une bille rapporteuse ; et
dans lequel la bille de détection et la bille rapporteuse sont de tailles différentes et dans lequel les puits de capture sont dimensionnés pour contenir une de chaque sorte.
